# EUROPEAN PATENT SPECIFICATION

(11) **EP 2 997 158 B1**
(45) Date of publication and mention of the grant of the patent: **26.07.2017**
(21) Application number: 14721386.2
(22) Date of filing: 05.05.2014
(51) Int. Cl.: C12Q 1/68

(54) **METHOD FOR DETERMINING THE RISK OF DEVELOPING RADIATION-INDUCED TOXICITY AFTER EXPOSURE TO RADIATION**
VERFAHREN ZUR BESTIMMUNG DES RISIKOS DER ENTWICKLUNG VON STRAHLUNGSINDUZIERTER TOXIZITÄT NACH EINER STRAHLENEXPOSITION
PROCÉDÉ POUR DÉTERMINER LE RISQUE DE DÉVELOPPER UNE TOXICITÉ INDUITE PAR UNE RADIATION APRÈS UNE EXPOSITION À UNE RADIATION

(30) Priority: 13.05.2013 EP 13167540
(43) Date of publication of application: 23.03.2016
(73) Proprietor: Universiteit Maastricht, 6211 LK Maastricht (NL); Academisch Ziekenhuis Maastricht, 6229 HX Maastricht (NL); STICHTING MAASTRICHT RADIATION ONCOLOGY "MAASTRO CLINIC", 6229 ET Maastricht (NL)
(72) Inventor: LAMBIN, Philippe, 1470 Genappe-Bousval (BE); NALBANTOV, Georgi Ilkov, 1619 Sofia (BG); SMEETS, Hubertus Julius Maria, 6211 NS Maastricht (NL); VOETS, An Mieke, 3740 Bilzen (BE)
(74) Representative: Habets, Winand
(86) International application number: PCT/EP2014/059089
(87) International publication number: WO 2014/184028

(56) References cited:
- US-A1- 2007 190 534
- CAI X W ET AL: "Plasma Proteomic Analysis May Identify New Markers for Radiation-Induced Lung Toxicity in Patients With Non-Small-Cell Lung Cancer", INTERNATIONAL JOURNAL OF RADIATION: ONCOLOGY BIOLOGY PHYSICS, PERGAMON PRESS, USA, vol. 77, no. 3, 1 July 2010 (2010-07-01), pages 867-876, XP027265627, ISSN: 0360-3016 [retrieved on 2010-05-26]
- QUAN WEN ET AL: "Mitochondrial DNA alterations of peripheral lymphocytes in acute lymphoblastic leukemia patients undergoing total body irradiation therapy", RADIATION ONCOLOGY, BIOMED CENTRAL LTD, LO, vol. 6, no. 1, 6 October 2011 (2011-10-06) , page 133, XP021111037, ISSN: 1748-717X, DOI: 10.1186/1748-717X-6-133
- BARNETT GILLIAN C ET AL: "Normal tissue reactions to radiotherapy: towards tailoring treatment dose by genotype.", NATURE REVIEWS. CANCER FEB 2009, vol. 9, no. 2, February 2009 (2009-02), pages 134-142, XP055069665, ISSN: 1474-1768
- ALSBEIH GHAZI A ET AL: "Involvement of mitochondrial DNA sequence variations and respiratory activity in late complications following radiotherapy.", CLINICAL CANCER RESEARCH : AN OFFICIAL JOURNAL OF THE AMERICAN ASSOCIATION FOR CANCER RESEARCH 1 DEC 2009, vol. 15, no. 23, 1 December 2009 (2009-12-01), pages 7352-7360, XP55029701, ISSN: 1078-0432

## Description

### Field of the invention

The invention is in the art of medical treatments, in particular the treatment of tumors with ionizing radiation. It provides means and methods for predicting whether a subject is likely to develop radiation-induced toxicity upon exposure to radiation. The invention allows the optimized treatment of a subject in need of a radiation treatment.

### Background of the invention

Radiation- and/ or chemical-induced toxicity in non-malignant tissues may result in debilitating side effects (e.g., intestinal radiation toxicity, pneumonitis, fibrosis, dyspnea, necrosis, telangiectasia, functional impairment, secondary cancer and mucositis). Therapeutic radiation exposure, for example, utilized in bone marrow transplant and more than half of all cancer patients, plays a critical role in approximately 25% of cancer cures. In spite of advances in the ability to deliver localized radiation for the treatment of cancer, radiation toxicity in non-malignant tissue remains the most important dose-limiting factor in clinical radiation toxicity. Moreover, patients suffering from radiation induced toxicity or long-term side effects of radiation, have a poor long term prognosis even if they are cured of the malignancy for which they received radiation treatment.

The term radiation induced toxicity is commonly used to include any one of the following conditions: Anemia, Febrile neutropenia, Hemolysis, Leukocytosis, Activated partial thromboplastin time prolonged, Electrocardiogram QT corrected interval prolonged, INR increased, Acidosis, Alkalosis, Hypercalcemia, Hyperglycemia, Hyperkalemia, Hypermagnesemia, Hypernatremia, Hypertriglyceridemia, Hyperuricemia, Hypoalbuminemia, Hypocalcemia, Hypoglycemia, Hypokalemia, Hypomagnesemia, Hyponatremia, Hypophosphatemia, Acute kidney injury, Chronic kidney disease, Hemoglobinuria, Alanine aminotransferase increased, Alkaline phosphatase increased, Aspartate aminotransferase increased, Blood bilirubin increased, Cardiac troponin I increased, Cardiac troponin T increased, CD4 lymphocytes decreased, Cholesterol high, CPK increased, Creatinine increased, Fibrinogen decreased, GGT increased, Haptoglobin decreased, Hemoglobin increased, Lipase increased, Lymphocyte count decreased, Lymphocyte count increased, Neutrophil count decreased, Platelet count decreased,

Serum amylase increased, White blood cell decreased and Proteinuria.

Common terminology criteria for adverse events (CTCAE) may be found online at http://evs.nci.nih.gov/ftp1/CTCAE/About.html.

Although higher radiation doses are associated with improved local control and overall survival, the current radiation practice is based on reducing the risk of developing secondary complication during or after radiation therapy to approximately 5-15%. However, in practice only a small number (15%) of patients are hypersensitive to radiation. Thus, identifying patients that are hypersensitive to radiation allows for the improvement of the radiotherapy plan, eventually selection for proton therapy or other radiation modalities and make it more optimal for the remainder of the patients.

The relevance of genetic predisposition for individual differences in radiotherapy (RT) toxicity or radiation-induced toxicity has been recognized for long (Barnett, G.C., et al. Normal tissue reactions to radiotherapy: towards tailoring treatment dose by genotype. Nat Rev Cancer 9, 134-142 (2009)). Studies using candidate gene approaches have searched for associations between single nucleotide polymorphisms (SNPs) in multiple genes and radiotoxicity, resulting in conflicting findings (Quarmby, S., et al. Association of transforming growth factor beta-1 single nucleotide polymorphisms with radiation-induced damage to normal tissues in breast cancer patients. Int J Radiat Biol 79, 137-143 (2003), Andreassen, C.N., Alsner, J., Overgaard, M. & Overgaard, J. Prediction of normal tissue radiosensitivity from polymorphisms in candidate genes. Radiother Oncol 69, 127-135 (2003), Azria, D., et al. Single nucleotide polymorphisms, apoptosis, and the development of severe late adverse effects after radiotherapy. Clin Cancer Res 14, 6284-6288 (2008), Barnett, G.C., et al. Independent validation of genes and polymorphisms reported to be associated with radiation toxicity: a prospective analysis study. Lancet Oncol 13, 65-77 (2012), Voets, A.M., et al. No association between TGF-beta1 polymorphisms and radiation-induced lung toxicity in a European cohort of lung cancer patients. Radiotherapy and oncology : Journal of the European Society for Therapeutic Radiology and Oncology 105, 296-298 (2012)).

Quan Wen et al., (2011) Radiation Oncology Biomed Central LTD, LO, 6: 133 discloses that mitochondrial DNA (mtDNA) and a deletion, referred to as "common deletion" thereof may be considered as predictive factor for radiation toxicity.

Xu-Wei Cai et al., (2010) Int. J. Radiation: Oncology Biology Physiscs, Pergamon Press USA; 77; 867-876 relates to markers of radiation sensitivity based on the analysis of proteins in plasma.

Alsbeih Ghazi et al., (2009) Clinical Cancer research: An official Journal of the American Association for Cancer Research: 15: 7352-7360 discloses non-synonymous variants of mitochondrial genes as an indicator of radiation sensitivity to radiotherapy

There is still a need in the art for more reliable markers that predict the risk of developing radiation-induced toxicity or radiation damage, in particular in the treatment of radiation-curable diseases such as thoracic cancers, such as for instance lung cancer and breast cancer. Such markers would be useful in guiding the physician towards a prediction of the radiation dose that may be applied to a certain subject without inducing damage to the body.

### Summary of the invention

A set of 321 lung cancer patients were studied herein, as well as a group of 21 patients with breast cancer. Both groups received radiation therapy. Radiation induced toxicity was determined in all patients. In the lung cancer group we scored the occurrence of dyspnea, or shortness of breath, according to the CTCAEv3.0 criteria whereas in the breast cancer group, we scored fibrosis according to the LENT-SOMA criteria (Int J Radiat Oncol Biol Phys. 1995 Mar 30;31(5):1049-91).

We found that an increased number of non-synonymous variations within mitochondrial genes encoding proteins which occurred at positions that were less than 90% conserved, correlated with an increased risk for radiation-induced toxicity.

We conclude that mitochondrial DNA variation can successfully predict whether a subject will develop radiation-induced toxicity after ionizing radiotherapy. In particular the non-synonymous variations (nucleotide variations resulting in a change of an amino acid in a protein encoded by a mitochondrial gene) appeared to have a predicting power for the development of radiation-induced toxicity after radiation therapy, in particular when these mutations occurred at a position that was less than 90% conserved.

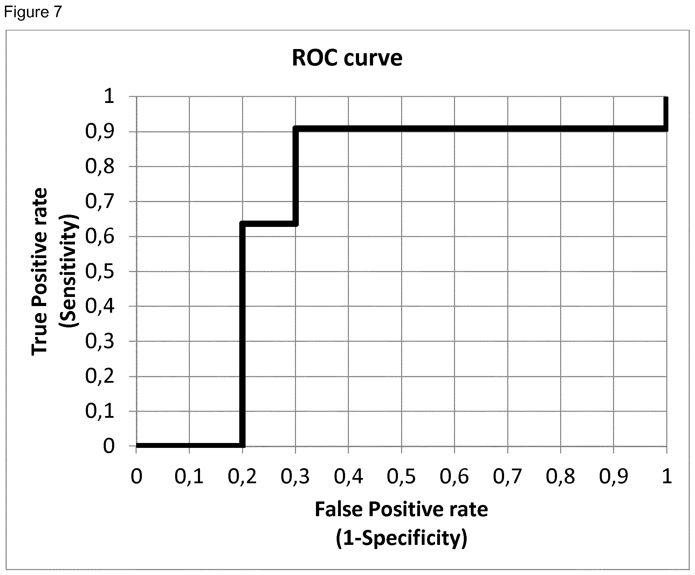

Hence, the invention relates to a method for predicting the risk of developing radiation induced toxicity comprising the steps of: obtaining mitochondrial DNA from a sample of a subject and determining the number of non-synonymous variations present in at least one gene encoding a mitochondrial protein, wherein said non-synonymous variations occur at positions that are less than 90% conserved, wherein a higher number of non-synonymous variations corresponds to a higher risk of developing radiation induced toxicity, as defined in the claims. When the data obtained in our population of lung cancer patients were expressed in a ROC curve, this resulted in an AUC of 0.770 for one set and 0.725 for the other set. When the data obtained in our population of breast cancer patients were expressed in a ROC curve, this resulted in an AUC of 0.714 (figure 7).

This shows that the method as disclosed herein provides a valuable and reliable tool for the prediction of radiation induced toxicity. The method according to the invention has a significantly better predictive power than the current gold standard for radiation therapy patients (mean lung dose, AUC 0.457 in our dataset). Incorporation of mtDNA information in personalized radiation therapy planning might eventually allow for escalating doses in patients predicted to be at low risk for radiation induced toxicity, improving their chance of disease free survival.

### Detailed description of the invention

The invention will herein be first described for a group of 321 lung cancer patients that were treated with radiation therapy in two different hospitals.

Lung cancer patients undergoing ionizing radiation therapy have an increased chance of developing radiation induced toxicity in their lungs, so-called radiation induced lung toxicity (RILT). The term RILT is herein defined as the development of dyspnea after irradiation. In the examples as provided herein the term RILT is used to indicate dyspnea grade ≥2 (CTCAEv3.0) within 6 months after radiation treatment.

As used herein, the phrases "radiation toxicity" or "radiation-induced toxicity" refer to radiation-induced injury to a cell or tissue arising from exposure to radiation. Radiation induces mainly DNA damage and oxidative damage, direct or indirect. The inflammation, scarring (collagen deposition) etc are mainly due to secondary repair mechanisms (removing damaged cells, closing gaps etc. Phenotypically, radiation-induced injury includes one or more of the following: structural radiation injury to a cell or tissue, increased neutrophil infiltration, increased collagen type III deposition, and increased smooth muscle cell proliferation relative to that seen in a cell or tissue not exposed to radiation. More in particular, the term radiation induced toxicity is commonly used to include any one of the following conditions: Anemia, Febrile neutropenia, Hemolysis, Leukocytosis, Activated partial thromboplastin time prolonged, Electrocardiogram QT corrected interval prolonged, INR increased, Acidosis, Alkalosis, Hypercalcemia, Hyperglycemia, Hyperkalemia, Hypermagnesemia, Hypernatremia, Hypertriglyceridemia, Hyperuricemia, Hypoalbuminemia, Hypocalcemia, Hypoglycemia, Hypokalemia, Hypomagnesemia, Hyponatremia, Hypophosphatemia, Acute kidney injury, Chronic kidney disease, Hemoglobinuria, Alanine aminotransferase increased, Alkaline phosphatase increased, Aspartate aminotransferase increased, Blood bilirubin increased, Cardiac troponin I increased, Cardiac troponin T increased, CD4 lymphocytes decreased, Cholesterol high, CPK increased, Creatinine increased, Fibrinogen decreased, GGT increased, Haptoglobin decreased, Hemoglobin increased, Lipase increased, Lymphocyte count decreased, Lymphocyte count increased, Neutrophil count decreased, Platelet count decreased, Serum amylase increased, White blood cell decreased and Proteinuria.

Common terminology criteria for adverse events (CTCAE) may be found online at http://evs.nci.nih.gov/ftp1/CTCAE/About.html.

Although higher radiation doses to a tumor, such as a lung tumor are associated with improved local control and overall survival, the current radiation practice is based on reducing the risk of developing secondary complication in the lungs (such as dyspnea) during or after radiation therapy to approximately 5-15%. However, in practice only a small number of patients are hypersensitive to radiation. Thus, identifying patients that are hypersensitive to radiation would allow for an improved treatment of the non-hypersensitive subjects wherein an increased dose may be given to patients that are not at risk of developing radiation induced toxicity such as RILT thereby increasing their chances on a complete cure.

We found that mitochondrial DNA variation can successfully predict whether lung cancer patients will develop radiation induced toxicity such as lung toxicity (RILT) after ionizing radiotherapy. We use the definition of RILT according to the CTCAEv3.0 scoring system. In essence, RILT is defined as an increase in dyspnea (shortness of breath) score. Approximately 15% of all lung cancer patients develop RILT after radiation therapy.

Even more importantly, the risk of radiation induced toxicity such as RILT may be used to determine the total dose to be applied in the therapy of a given patient. Since the success of the therapy depends on the dose provided, the treating physician is always looking for the highest possible dose to be administered.

The more accurate a prediction of radiation induced toxicity is, the higher the dose can be, thereby improving the success rate of the treatment.

We present herein a predictive model based on mitochondrial DNA (mtDNA) data that achieves an accuracy of 77%, exceeding the accuracy of existing models which achieve an accuracy of about 50-60%, which is hardly better than random.

Mitochondrial DNA is the DNA located in organelles called mitochondria, structures within eukaryotic cells that convert the chemical energy from food into a form that cells can use, adenosine triphosphate (ATP). Most of the rest of the DNA present in eukaryotic cells can be found in the cell nucleus.

In humans, mitochondrial DNA can be regarded as the smallest chromosome coding for only 37 genes and containing only about 16,600 base pairs. Human mitochondrial DNA was the first significant part of the human genome to be sequenced. In most species, including humans, mtDNA is inherited solely from the mother.

The DNA sequence of mtDNA has been determined from a large number of organisms and individuals (including some organisms that are extinct), and the comparison of those DNA sequences represents a mainstay of phylogenetics, in that it allows biologists to elucidate the evolutionary relationships among species. It also permits an examination of the relatedness of populations, and so has become important in anthropology and field biology.

For human mitochondrial DNA 100-10,000 separate copies of mtDNA are usually present per cell. In mammals, each double-stranded circular mtDNA molecule consists of 15,000-17,000 base pairs. The two strands of mtDNA are differentiated by their nucleotide content with the guanine rich strand referred to as the heavy strand or H-strand, and the cytosine rich strand referred to as the light strand or L-strand. The heavy strand encodes 28 genes, and the light strand encodes 9 genes for a total of 37 genes. Of the 37 genes, 13 are for proteins (polypeptides), 22 are for transfer RNA (tRNA) and two are for the small and large subunits of ribosomal RNA (rRNA).

The revised Cambridge Reference Sequence (rCRS) of the human mitochondrial DNA is used herein as published under NCBI number NC_012920 (Genbank's RefSeq database). It is the Cambridge Reference Sequence as provided by Anderson et al., Nature (1981) 290; 457-465 as revised by Andrews et al., Nature Genetics (1999) 2; 147. The rCRS is referred herein as the reference sequence and the nucleotide sequence of its L-strand is provided herein as SEQ ID NO: 1.

Variation in the mtDNA sequence is common and every individual carries between 10 and 60 variants. We now found that certain types of mtDNA variants are associated with radiation induced toxicity such as lung toxicity (RILT). We also found that certain types of mtDNA variations were indicative for the risk of developing radiation induced toxicity.

The terms "predicting the risk" "indicative of the risk" or "risk factor" or equivalent, as used herein, refer to assessing the probability according to which the subject as referred to herein will experience radiation induced toxicity, such as RILT. More preferably, the risk/probability of developing radiation induced toxicity within a certain time window is predicted. In a preferred embodiment of the present invention, the predictive window, preferably, is an interval of at least 3 months, such as at least 6 months, at least 9 months, at least 1 year, at least 2 years, at least 3 years, at least 4 years, at least 5 years, at least 10 years, at least 15 years or any intermitting time range. In a particular preferred embodiment of the present invention, the predictive window, preferably, is an interval of at least 6 months. Preferably, said predictive window is calculated from the time point at which radiotherapy started.

Mitochondrial DNA, extracted from blood lymphocytes of patients before they underwent radiotherapy, was sequenced using the commercially available Affymetrix Mitochondrial DNA resequencing chip 2.0 (Mitochip). The sequence obtained was compared with the reference sequence (SEQ ID NO:1) and variants were scored and validated.

It was found that in particular the non-synonymous variants in any of the protein encoding genes in the mitochondrial genome correlated with the development of radiation induced toxicity such as RILT upon radiation.

The mtDNA may be obtained from all kinds of samples by using standard techniques, commercially available in the art. Preferred is the use of samples containing nucleated cells, preferably blood cells, more preferably, white blood cells. Also preferred is the use of a sample obtained before the start of the irradiation.

The sequence of the mtDNA may be determined with routine methods which are commercially available in the art. The obtained mtDNA sequences may then be compared with a reference sequence, such as SEQ ID NO:1, and variations may be determined.

Due to the degeneracy of the genetic code, not every variation in the nucleotide sequence will automatically lead to an alteration or variant in the protein sequence. The variations in the nucleotide sequence that lead to alterations (variants) of the encoded proteins are termed herein "non-synonymous" variations. Silent variations that do not result in the alteration of the primary protein sequence are termed "synonymous" variants or variations.

The 13 mtDNA genes encoding proteins are termed ND1, ND2, CO1, CO2, ATP8, ATP6, CO3, ND3, ND4L, ND4, ND5, ND6, and CytB. Their amino acid sequence (SEQ ID NO: 2 - 14) is provided in tables 1 - 13. The position in the mtDNA genome of these 13 genes is shown in table 14.

A person skilled in the art will be able to determine whether a certain variation in the mtDNA found in a particular subject is within the coding sequence of a mitochondrial gene encoding a protein. For that purpose, he may align the sequence obtained from the subject with the reference sequence and determine whether the variant is within the range between the first and the last coding sequence of any of the genes listed in table 14.

A variant nucleotide is defined herein as a nucleotide that is different from its counterpart nucleotide at the same position in a reference sequence, such as SEQ ID NO: 1. So, a nucleotide G at position 3600 in the sequence of the mtDNA of a subject is a variant nucleotide since position 3600 in the reference sequence (SEQ ID NO:1) is a C.

If the variant nucleotide is within the coding sequence of any of the genes encoding a protein (protein genes) then the triplet encoding the corresponding amino acid may be determined. The skilled person will know how to determine the corresponding amino acid position. In detail: he may first determine the position of the variant (N) relative to the start codon by subtracting the first coding nucleotide position (FCNP) position from the position number of N. For example, a variation at position N=3340 is within the ND1 gene (Table 14) at relative position 3340-3308 = 32.

Next, he may determine the triplet codon number for that position by dividing the absolute value of the relative position number by 3, adding 1 to the outcome and rounding down the value towards zero. For example, relative positions 30 to 32 are within triplet number 11 of the ND1 gene. Triplet 11 encodes amino acid number 11 of the ND1 gene.

A formula for that calculation in Microsoft Excel would read: ROUNDDOWN(((ABS(N-FNP))/3+1);0). More suitable computer programs for converting nucleotide variations into amino acid variations are available in the art and are preferred.

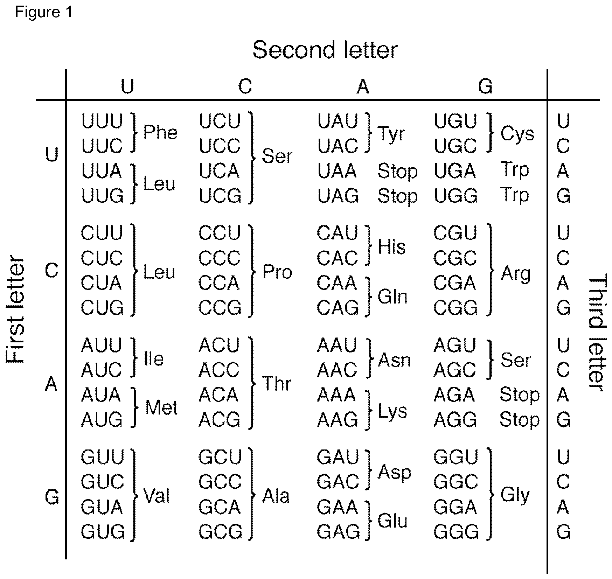

Once the triplet codon number has been determined, it may be determined whether the nucleotide variation will result in an amino acid variation by comparing the encoded amino acid with the reference sequence (SEQ ID NO: 1) and the sequence in tables 1 - 13. Care is to be taken in this process to use the genetic code for mitochondrial DNA which is slightly different from the code for nuclear DNA. The codon usage table for mitochondrial DNA is known and provided herein in figure 1. If the nucleotide variation does not alter the amino acid encoded, the variation is referred to as a synonymous variation, otherwise it is considered a non-synonymous variation.

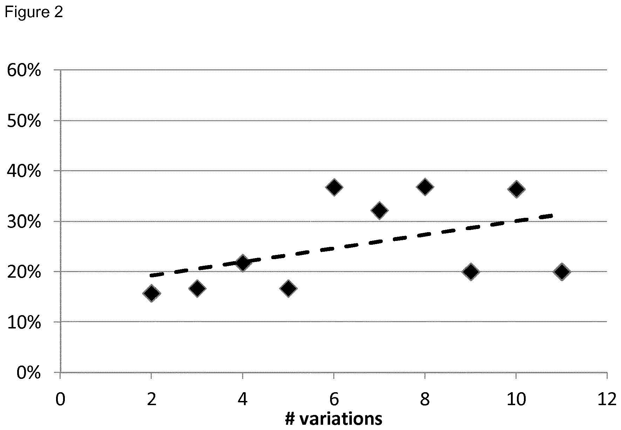

We found that there is a positive correlation between the number of non-synonymous variations and the risk of developing radiation induced toxicity such as RILT after radiation. The raw data obtained with the largest population of 321 lung cancer patients are shown in Table 16 below. The results obtained with the smaller population of 66 lung cancer patients were comparable if not identical. It is shown in table 16 that the number of non-synonymous variations in these patients ranges from 0 to 14. When the patients were grouped according to their number of non-synonymous variations, and groups with less than 3 members were left out, it can be seen that the risk on RILT increases with increasing number of non-synonymous variations (table 17 and figure 2).

**Table 17. The risk on RILT increases with increasing number of non-synonymous variations.**

| **# non-synonymous variations** | **% patients developing RILT** |
|---|---|
| 2 | 16% |
| 3 | 17% |
| 4 | 22% |
| 5 | 17% |
| 6 | 37% |
| 7 | 32% |
| 8 | 37% |
| 9 | 20% |
| 10 | 36% |
| 11 | 20% |

In a ROC curve produced with our data as disclosed herein, the use of this "marker" in a method according to the invention resulted in an accuracy of 60% which is an improvement over prior art methods.

Hence, the invention relates to a method for predicting the risk of developing radiation induced toxicity (such as RILT) comprising the steps of obtaining mitochondrial DNA from a sample of a subject and determining the number of non-synonymous variations present in at least one gene encoding a mitochondrial protein, wherein a higher number of non-synonymous variations corresponds to a higher risk of developing radiation induced toxicity, such as lung toxicity.

When this method is combined with methods employing other or further risk factors, this may advantageously be done in a so-called nomogram. Therein, a value is attributed to a certain risk factor (such as the number of non-synonymous variations in the mitochondrial DNA) that is then converted in a risk factor, wherein a higher value indicates a higher risk.

The invention therefore also relates to a method for predicting the risk of developing radiation induced toxicity comprising the steps of obtaining mitochondrial DNA from a sample of a subject, determining the number of non-synonymous variations present in at least one gene encoding a mitochondrial protein and attributing a value to the number of non-synonymous variations, wherein a higher value corresponds to a higher risk of developing radiation induced toxicity, such as lung toxicity, as defined in the claims. We further found that the method could be improved by scoring only those non-synonymous variations that occurred in the mtDNA genome at positions that were less conserved in evolution. For the determination of the evolutionary conservation of the individual positions in the mtDNA genome, we used a database provided online at http://mtsnp.tmig.or.jp/mtsnp/search_mtSAP_evaluation_e.html. Therein, the entire mtDNA genome of 61 mammalian species can be found. We used those data to determine the evolutionary conservation of each amino acid encoded by the reference sequence of the mtDNA (SEQ ID NO:1). The results are shown in tables 1 - 13 in the column Percentage Conservation (%cons).

Particularly good results were thus obtained when the non-synonymous variants that were well conserved in the reference sequence were excluded from the analysis. It was found that amino acid variants on positions that were evolutionary less conserved in the reference sequence were better pedictors for radiation induced toxicity, such as RILT than the variants on well conserved positions.

For example, amino acid number 11 of the ATP6 gene (Table 1) is 13.1% conserved and would therefore have a better predictive value than amino acid number 1 which is 98.4% conserved.

The invention therefore relates to a method as described above wherein said non-synonymous variations are selected from the group of variants that are less than 100% conserved.

Less than 100% in the present context means 99% or less, such as 98%, 97%, 96%, 95%, 94%, 93%, 92%, 91%, 90%, 89%, 88%, 87%, 86%, 85%, 84%, 83%, 82%, 81%, 80% or less conserved, such as 79%, 78%, 77%, 76%, 75%, 74%, 73%, 72%, 71%, 70%, 69%, 68%, 67%, 66%, 65%, 64%, 63%, 62%, 61%, 60%, 59%, 58%, 57%, 56%, 55%, 54%, 53%, 52%, 51%, 50% or less, including any number between 49 and 10.

It was found that there is an optimum number of conservation to be used in the present invention. If the percentage of conservation decreases, the number of non-synonymous amino acid variations that can be used in the analysis also decreases, making the prediction increasingly more unreliable. On the other hand, the predictive value of the individual variant increases with decreasing percentage of conservation. That mechanism results in an optimal percentage of conservation that yields the optimal predictive value. In our test population that optimal value was found to be between less than 85-95%, such as less than or equal to 90% (<=90%).

Since our test population is sufficiently large, this percentage conservation is expected to be an adequate cut-off value for performing the method in any other population.

In a preferred embodiment, the invention therefore relates to a method as described above wherein said non-synonymous variations occur at positions that are at most 90% conserved (<=90% conserved).

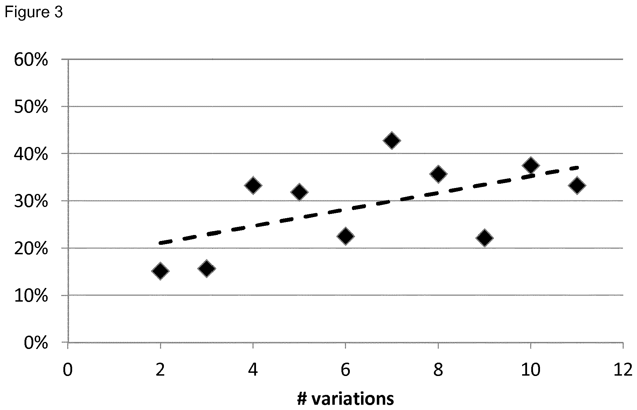

This is illustrated by the data presented in Table 18 and figure 3.

**Table 18: The risk on RILT increases with increasing number of non-synonymous variations at positions <=90% conserved.**

| **# non-synonymous variations at positions <=90% conserved** | **% patients developing RILT** |
|---|---|
| 2 | 15% |
| 3 | 16% |
| 4 | 33% |
| 5 | 32% |
| 6 | 23% |
| 7 | 43% |
| 8 | 36% |
| 9 | 22% |
| 10 | 38% |
| 11 | 33% |

The method was found to be particularly useful in a population of patients suffering from lung cancer. Hence, in a preferred embodiment, the invention relates to a method as described above wherein the subject is diagnosed with lung cancer.

The predictive value of the method as disclosed above, could even be further improved when the value attributed to the number of non-synonymous variations was added to a value obtained from clinical parameters. The most useful clinical parameter in this respect was the baseline dyspnea score.

The term baseline dyspnea score in this context means the shortness-of-breath before the start of the radiation therapy, scored qualitatively on a 0-5 scale according to the Common Toxicity Criteria version 3.0 (CTCAEv3.0). Of course, different CTC versions may also be used, as well as different approaches to score dyspnea, such as scoring according to other schemes (example: RTOG) or using quantitative measures to score dyspnea (such as measuring the movement of patients though time, using for instance a pedometer device).

The invention therefore relates to a method as described above, additionally comprising a step of attributing a value to the baseline dyspnea score of the subject and adding that value to the value obtained from the number of non-synonymous variations to obtain an aggregated value, wherein a higher aggregated value corresponds to a higher risk of developing radiation induced toxicity, such as lung toxicity.

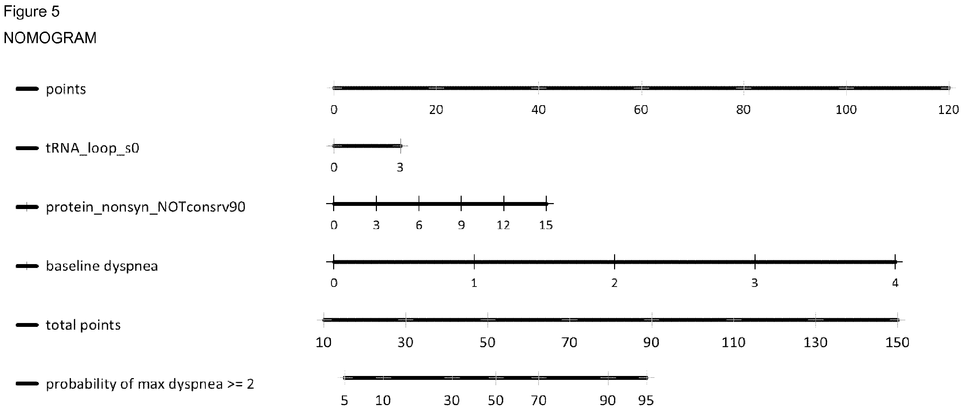

The values attributed to the number of non-synonymous variations and the value attributed to the baseline dyspnea score of the subject may advantageously be represented in a nomogram. Such a nomogram is shown in figure 5.

A nomogram is a tool to estimate outcome probability by assigning points (upperscale) to each predictor value. The sum of these scores corresponds to an event probability (bottom two scales).

We also found that a method as described above could even be further improved when variations in the genes encoding tRNAs were also taken into account, in particular those variations that occur in the loops of the tRNAs.

Table 15 shows the nucleotide positions of loops of tRNAs. There are three loops; the D-loop, the T-loop and the anti-CD loop. The D-loop in tRNA contains the modified nucleotide dihydrouridine. It is composed of 7 to 11 bases and is closed by a Watson Crick base pair. The TψC-loop (generally called the T-loop) contains thymine, a base usually found in DNA and pseudouracil (ψ). The D-loop and T-loop form a tertiary interaction in tRNA. The anticodon loop consists of 7 nucleotides that encompass the three nucleotides that correspond to the three bases of the codon of the mRNA.

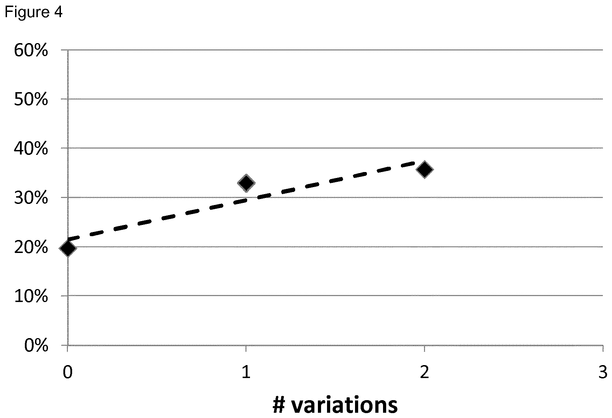

The correlation between number of variants in the loops of tRNA genes is shown in table 19 and figure 4.

**Table 19: The risk on RILT increases with increasing number of variations at positions encoding the loops of tRNAs.**

| **# variations at positions encoding tRNA loops** | **% patients developing RILT** |
|---|---|
| 0 | 20% |
| 1 | 33% |
| 2 | 36% |

In a preferred embodiment, the invention therefore relates to a method as described above wherein the number of variant nucleotides in any of the three loops is counted and a value attributed to the number of variants. The higher the number of variants, the higher the attributed value. The value thus obtained may then be added to the aggregated value obtained from the non-synonymous variants and the baseline dyspnea score.

In a particularly preferred embodiment, the invention therefore relates to a method as described above, additionally comprising a step of determining the number of variations in tRNA loop positions, attributing a value to the number of variations in tRNA loop positions and adding that value to the value obtained from the number of non-synonymous variations or the aggregated value.

In an even more preferred embodiment, the treatment status of the subject is also taken into account. Patients treated with chemotherapy had a higher risk of radiation induced toxicity such as RILT after the radiation treatment. This risk increased with the dose of chemotherapy. Treatment may therefore also successfully be integrated in a method according to the invention. For that purpose, a value is attributed to the treatment status, proportional to the dose of chemotherapy and that value is added to the value obtained from the number of non-synonymous variations or the aggregated value.

As detailed above, the mitochondrial genome consists of 13 genes encoding 13 different proteins. The method may be performed by considering only the non-synonymous variations in one of these genes, at each position or at some selected positions, such as less conserved positions. However, the accuracy and reliability of the method improves when more than one gene is included in the analysis, such as 2 genes or more than 2 genes, such as 3 or 4, 5, 6, 7, 8, 9, 10, 11, 12 or all protein encoding genes.

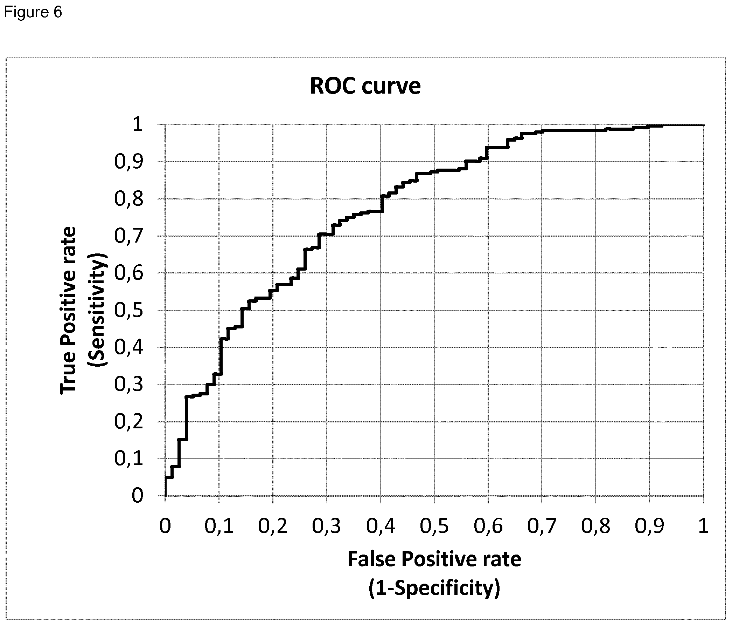

Figure 6 shows a ROC curve obtained when a preferred method according to the invention was applied to the population of the present study. At a certain point in the ROC curve, 64 patients are predicted to not have RILT after radiation treatment whereas 5 patients do.

Therefore, when using this point for predicting whether a patient could receive higher radiation doses, these patients have a relative risk of 8% for developing RILT. This is significant improvement (p = 0.012, Chi-square equality-of-proportions test) from the 24% of patients developing RILT in the whole training dataset. Furthermore, at another point in the ROC curve, 23 patients were predicted to develop RILT and could be selected for treatment with expensive radio-protectors, such as amifostine, or proton therapy. Although at that point 5 (22%) patients were wrongly predicted to be radiosensitive, this proportion is significantly less (p<0.001, Chi-square test) than the 76% of patients not developing RILT in the whole training dataset. The expenses of (over)treating such small proportion of patients are substantially smaller than when all patients would be included (increased relative benefit).

The model as described above does not only work for lung cancer patients or radiation induced lung toxicity. When we applied the same model to patients diagnosed with breast cancer, we found that the model predicted the occurrence of radiation induced toxicity with great precision and accuracy.

In a population of 21 patients with breast cancer, we obtained mitochondrial DNA from fibroblasts of each patient before the start of the radiotherapy and determined the number of non-synonymous variations present in at least one gene encoding a mitochondrial protein, wherein said non-synonymous variations occur at positions that are less than 90% conserved. We correlated these findings with the radiation induced toxicity data from these patients, in this case measured as fibroblast induced fibrosis, scored quantitatively according to the LENT/SOMA criteria (Herskind et al., Radiother. Oncol. 1998; 47: 263-269, Pavy JJ, Int J Radiat Oncol Biol Phys. 1995 Mar 30;31 (5):1049-91.). Endpoint was a grade of fibrosis of at least 2 (>=2).

We found an excellent correlation between the prediction of the model and the actual fibrosis observed in the patient group (Table 20).

**Table 20: Correlation between the prediction of the method according to the invention and the radiation induced toxicity observed in the patient group.**

| | Method of Invention | | |
|---|---|---|---|
| Fibrosis | High risk | Low risk | Total |
| >=2 | 8 | 2 | 10 |
| <2 | 2 | 9 | 11 |
| Total | 10 | 11 | 21 |

From the data provided in table 20, it may be concluded that the accuracy of the method according to the invention is 17/21 or 81%. The positive predictive value is 8/10 or 80%, whereas the negative predictive value is 9/11 or 82%. Specificity of the method in this group was 9/11 or 82% and sensitivity was 8/10 or 80%.

The possibility to predict if patients will be hypersensitive to radiotherapy or not will allow more optimal delivery and ultimately the usage of higher radiation doses (and thus better tumor control and outcome) in patients that are not hypersensitive. This invention will predominantly be useful in radiotherapy centers and clinics that treat cancer patients, such as lung cancer patients and breast cancer patients. The invention may also be useful in the area of radiation protection, such as screening of subjects for working or living in an area with increased radiation, such as for the screening of pilots, astronauts or workers in a radiation contaminated area or nuclear plant.

### Legend to the figures

Figure 1. Codon table for mitochondrial DNA.
Figure 2. Scatter plot with trendline. The figure shows the data presented in table 17. It can be seen that the risk on RILT increases with increasing number of non-synonymous variations.
Figure 3. Scatter plot with trendline. The figure shows the data presented in table 18. It can be seen that the risk on RILT increases with increasing number of non-synonymous variations at positions that are 90% or less conserved.
Figure 4. Scatter plot with trendline. The figure shows the data presented in table 19. It can be seen that the risk on RILT increases with increasing number variations in the genes encoding tRNAs at position encoding the tRNA loops.
Figure 5. The nomogram is a model visualizing a diagram for determining the probability of RILT. The figure shows a model for determining the probability of dyspnea >= 2. The uppermost horizontal line indicates the number of points to be attributed to a certain parameter. For example: if 0 variations are found in the tRNA loop of an individual, then 0 points are scored. When 15 non-synonymous variations are found at positions less than 90% conserved, this accounts for an additional 42 points. A baseline dyspnea score of 1 accounts for 27 points. The total points for this individual would then be 42 plus 14 plus 0 is 56 total points. This individual would then score a probability of about 60% of RILT, calculated based on the total points horizontal line and the respective value of max. dyspnea >=2 line.
Figure 6: ROC CURVE obtained from the population of lung cancer patients tested herein.
Figure 7: ROC CURVE obtained from the population of breast cancer patients tested herein.

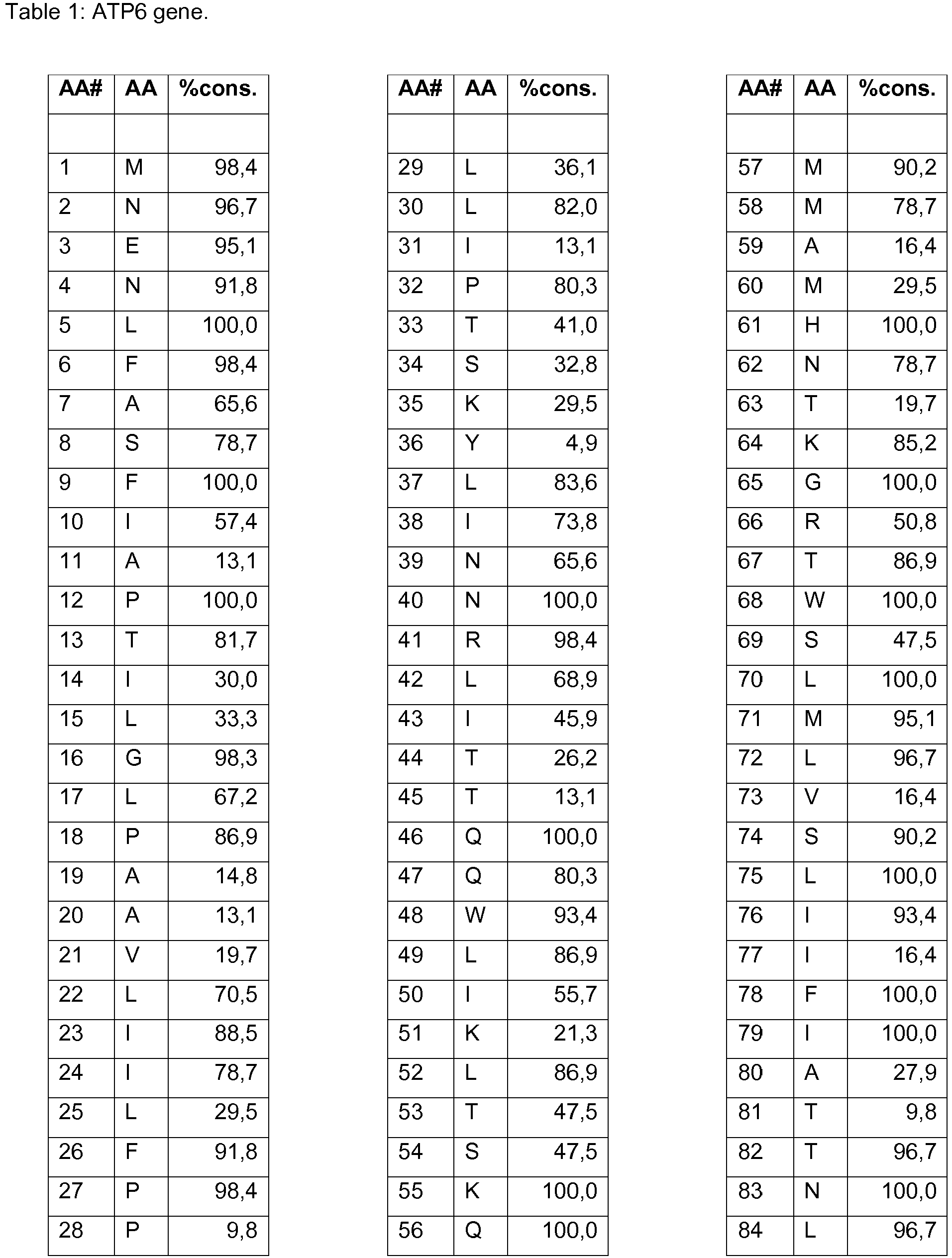

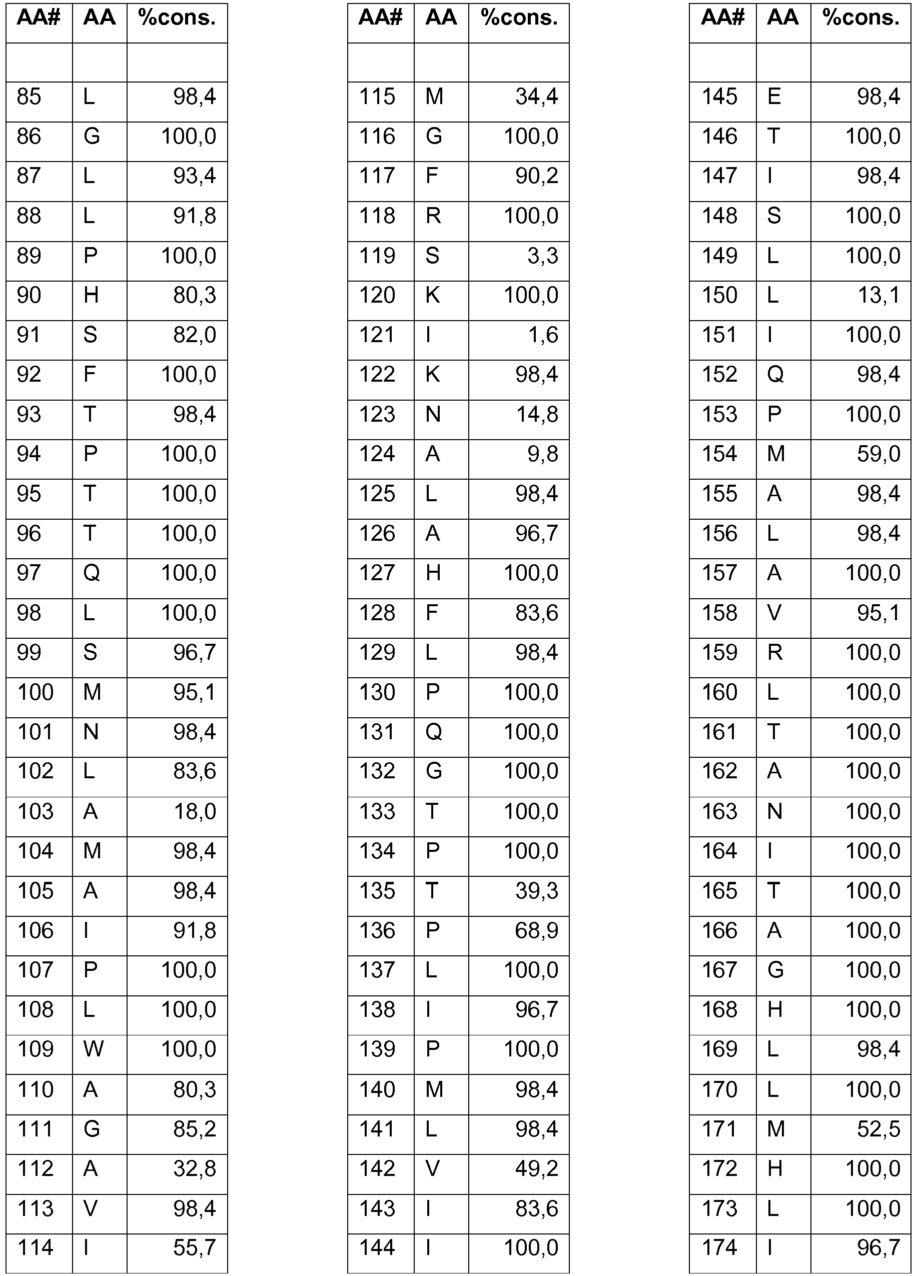

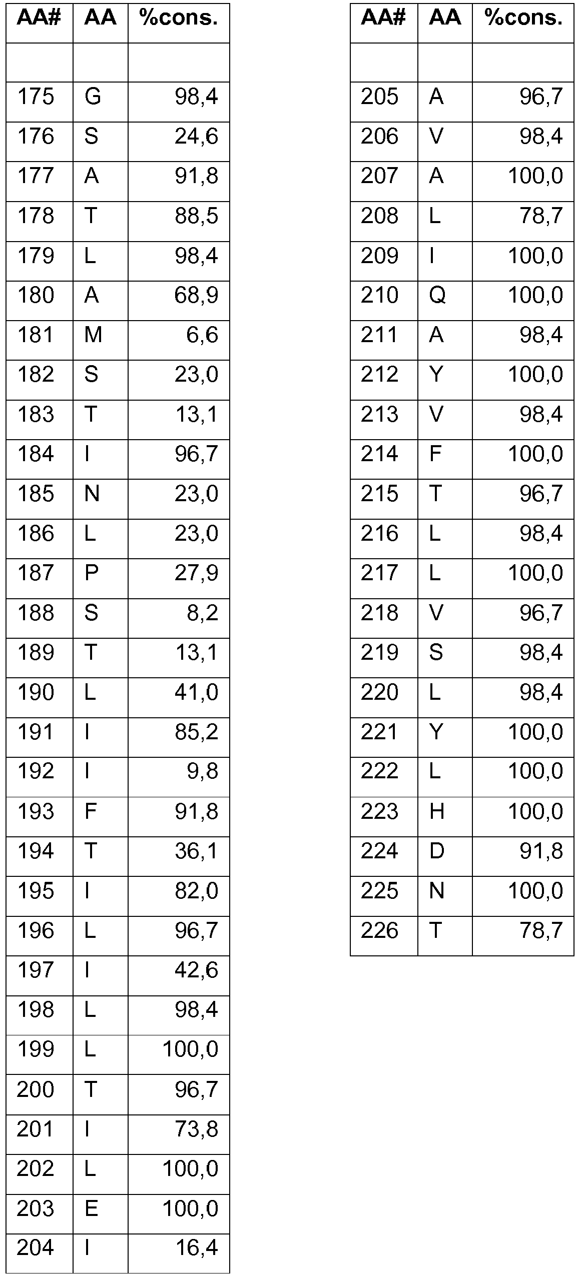

**Table 2: ATP 8 gene**

| **AA#** | **AA** | **%cons.** |
|---|---|---|
| | | |
| 1 | M | 100,0 |
| 2 | P | 98,4 |
| 3 | Q | 98,4 |
| 4 | L | 98,4 |
| 5 | N | 16,4 |
| 6 | T | 96,7 |
| 7 | T | 11,5 |
| 8 | V | 8,2 |
| 9 | W | 100,0 |
| 10 | P | 11,5 |
| 11 | T | 29,5 |
| 12 | M | 14,8 |
| 13 | I | 95,1 |
| 14 | T | 14,8 |
| 15 | P | 9,8 |
| 16 | M | 85,2 |
| 17 | L | 29,5 |
| 18 | L | 57,4 |
| 19 | T | 70,5 |
| 20 | L | 95,1 |
| 21 | F | 86,9 |
| 22 | L | 16,4 |
| 23 | I | 31,1 |
| 24 | T | 13,1 |
| 25 | Q | 95,1 |
| 26 | L | 86,9 |
| 27 | K | 96,7 |
| 28 | M | 19,7 |
| | | |
| 29 | L | 21,3 |
| 30 | N | 27,9 |
| 31 | T | 6,6 |
| 32 | N | 16,4 |
| 33 | Y | 62,3 |
| 34 | H | 18,0 |
| 35 | L | 16,4 |
| 36 | P | 16,4 |
| 37 | P | 82,0 |
| 38 | S | 16,4 |
| 39 | P | 42,6 |
| 40 | K | 67,2 |
| 41 | P | 16,4 |
| 42 | M | 18,0 |
| 43 | K | 55,7 |
| 44 | M | 13,1 |
| 45 | K | 14,8 |
| 46 | N | 9,8 |
| 47 | Y | 6,6 |
| 48 | N | 26,2 |
| 49 | K | 11,5 |
| 50 | P | 91,8 |
| 51 | W | 100,0 |
| 52 | E | 77,0 |
| 53 | P | 11,5 |
| 54 | K | 93,4 |
| 55 | W | 100,0 |
| 56 | T | 98,4 |
| | | |
| 57 | K | 100,0 |
| 58 | I | 77,0 |
| 59 | C | 6,6 |
| 60 | S | 41,0 |
| 61 | L | 9,8 |
| 62 | H | 32,8 |
| 63 | S | 82,0 |
| 64 | L | 78,6 |
| 65 | P | 83,9 |
| 66 | P | 21,4 |
| 67 | Q | 68,6 |
| 68 | S | 68,4 |

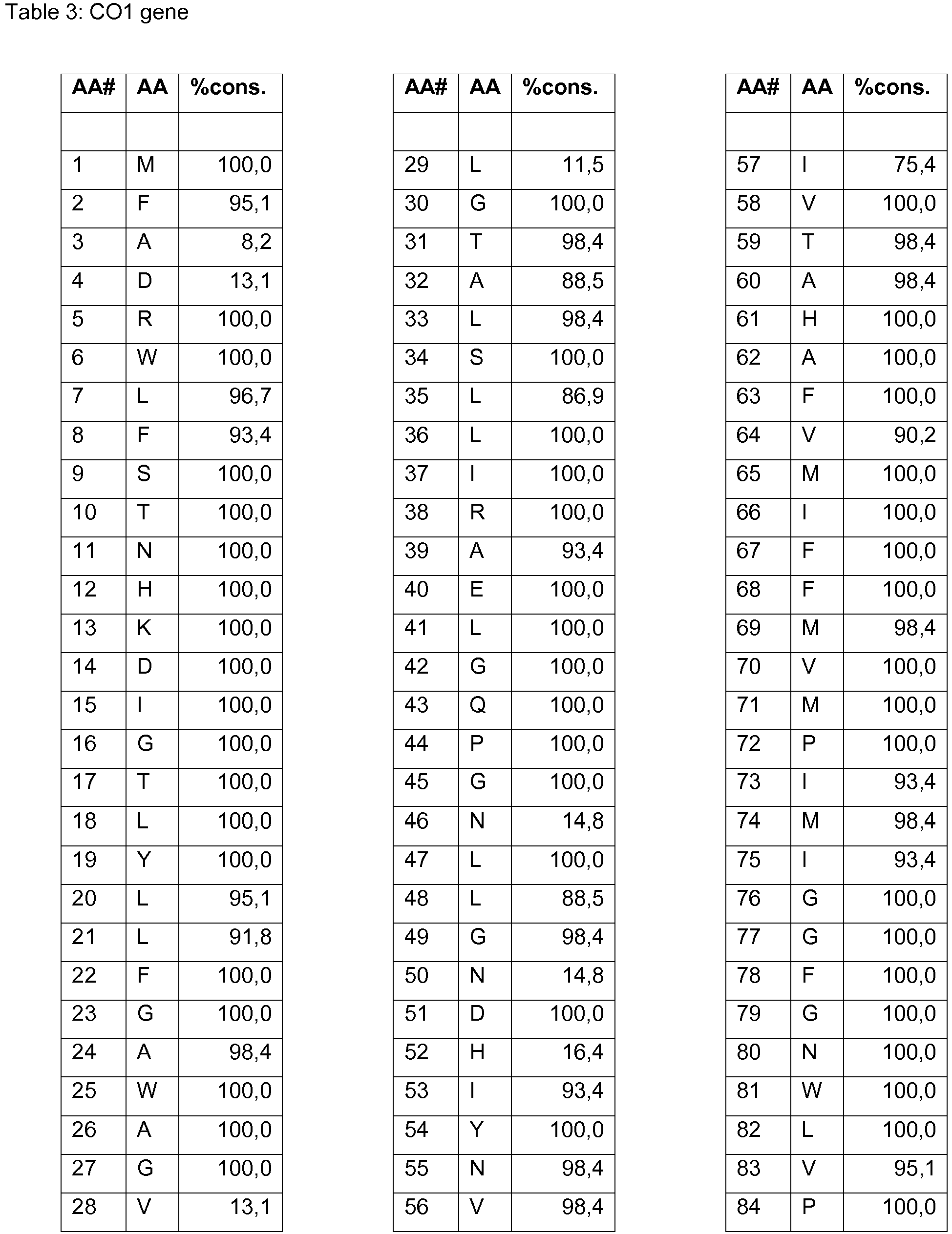

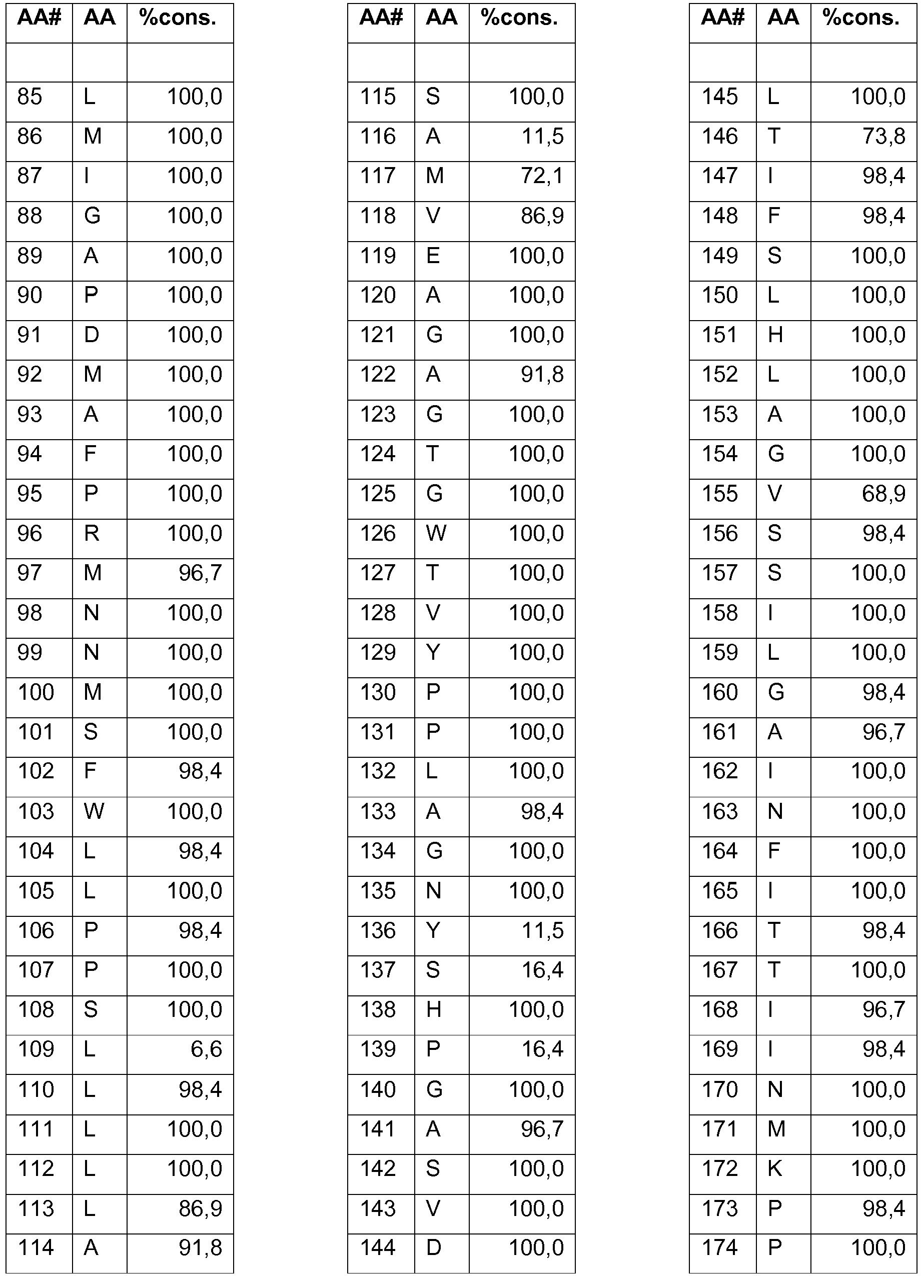

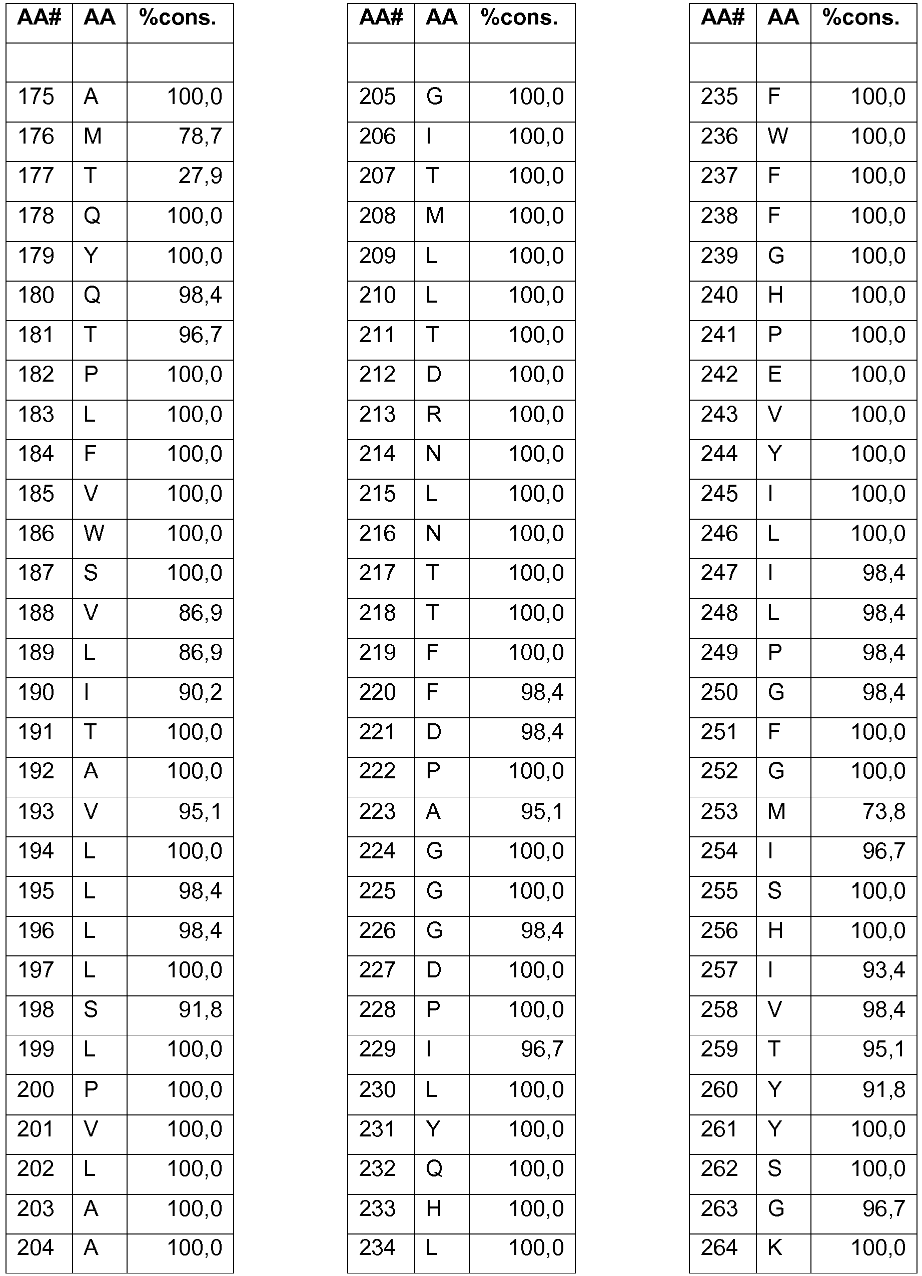

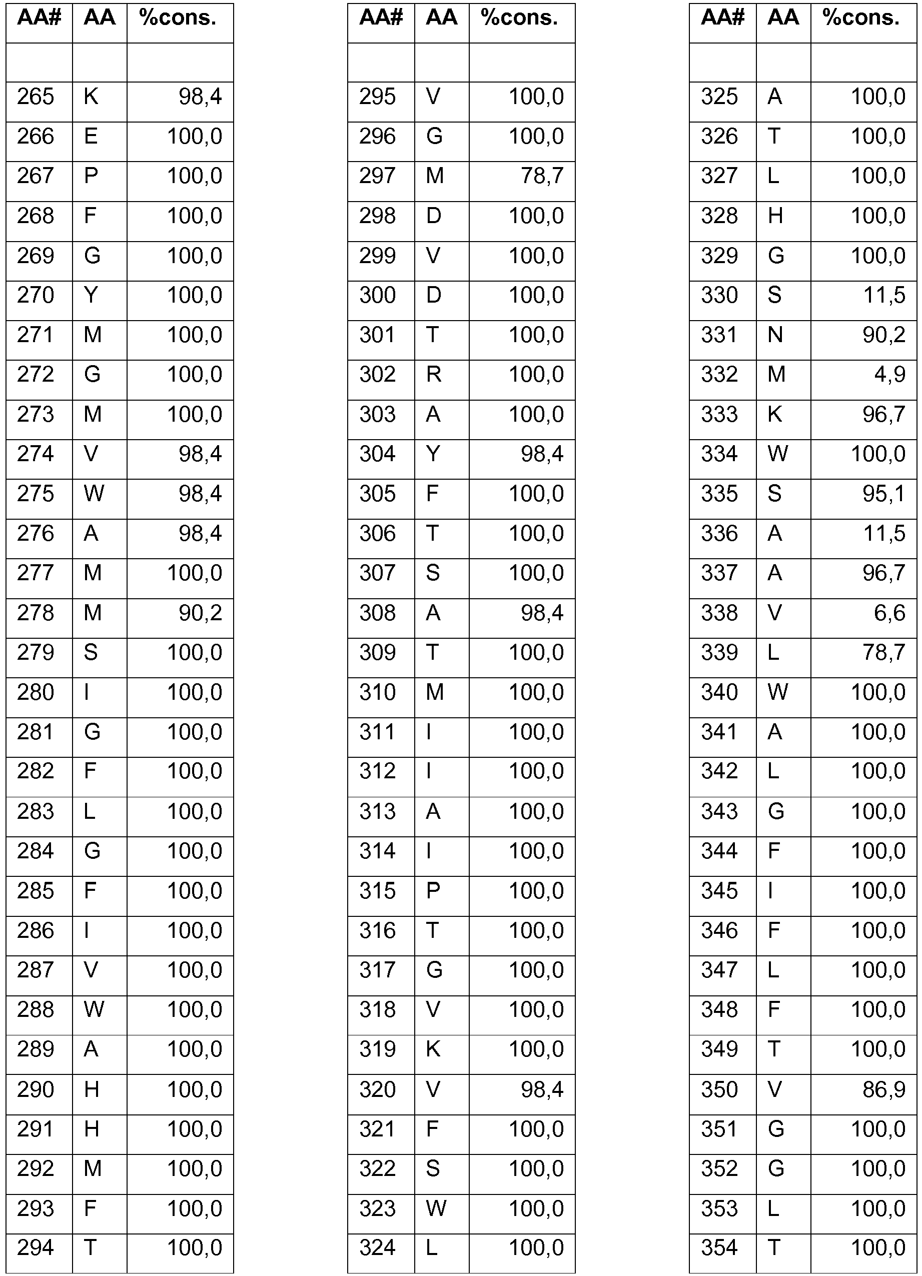

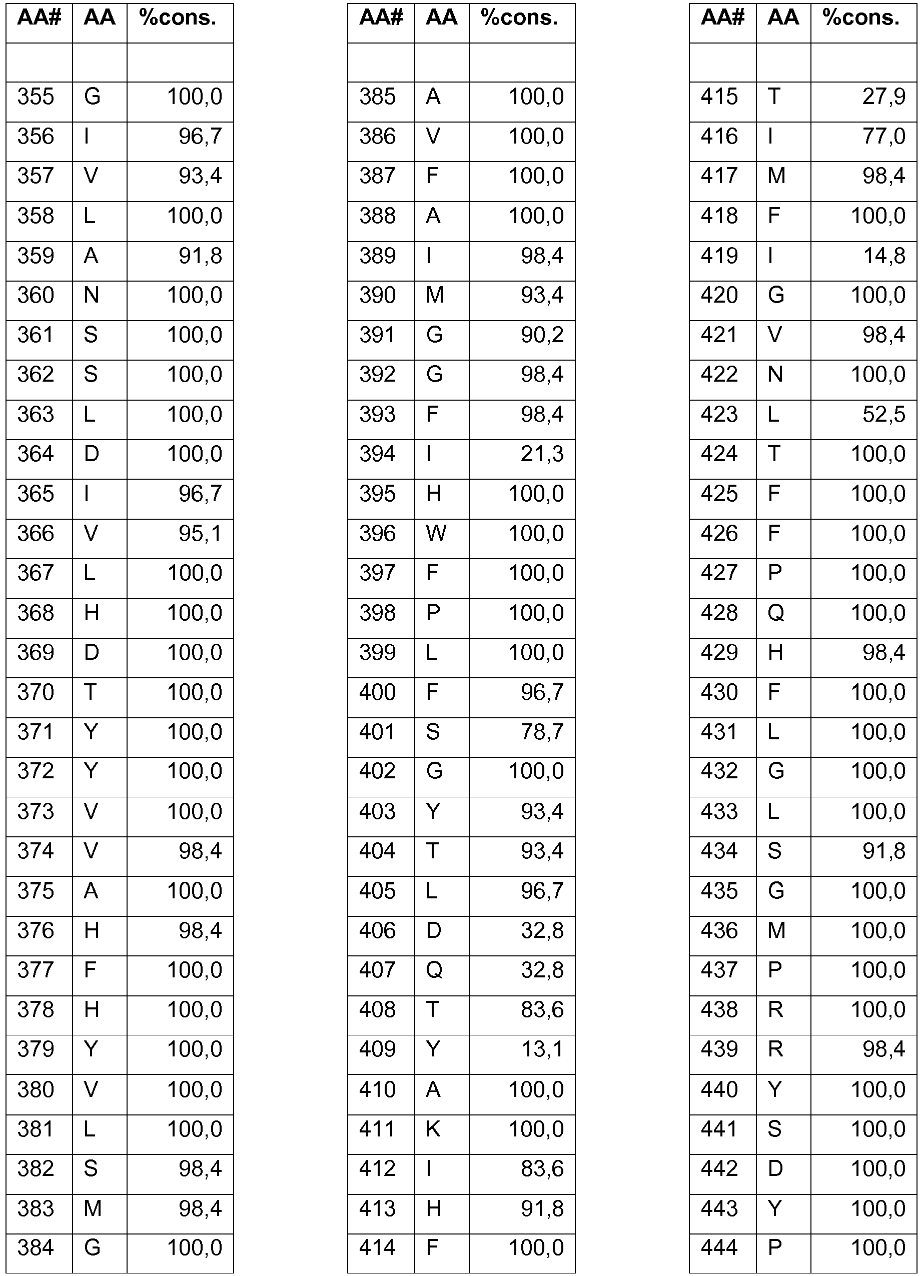

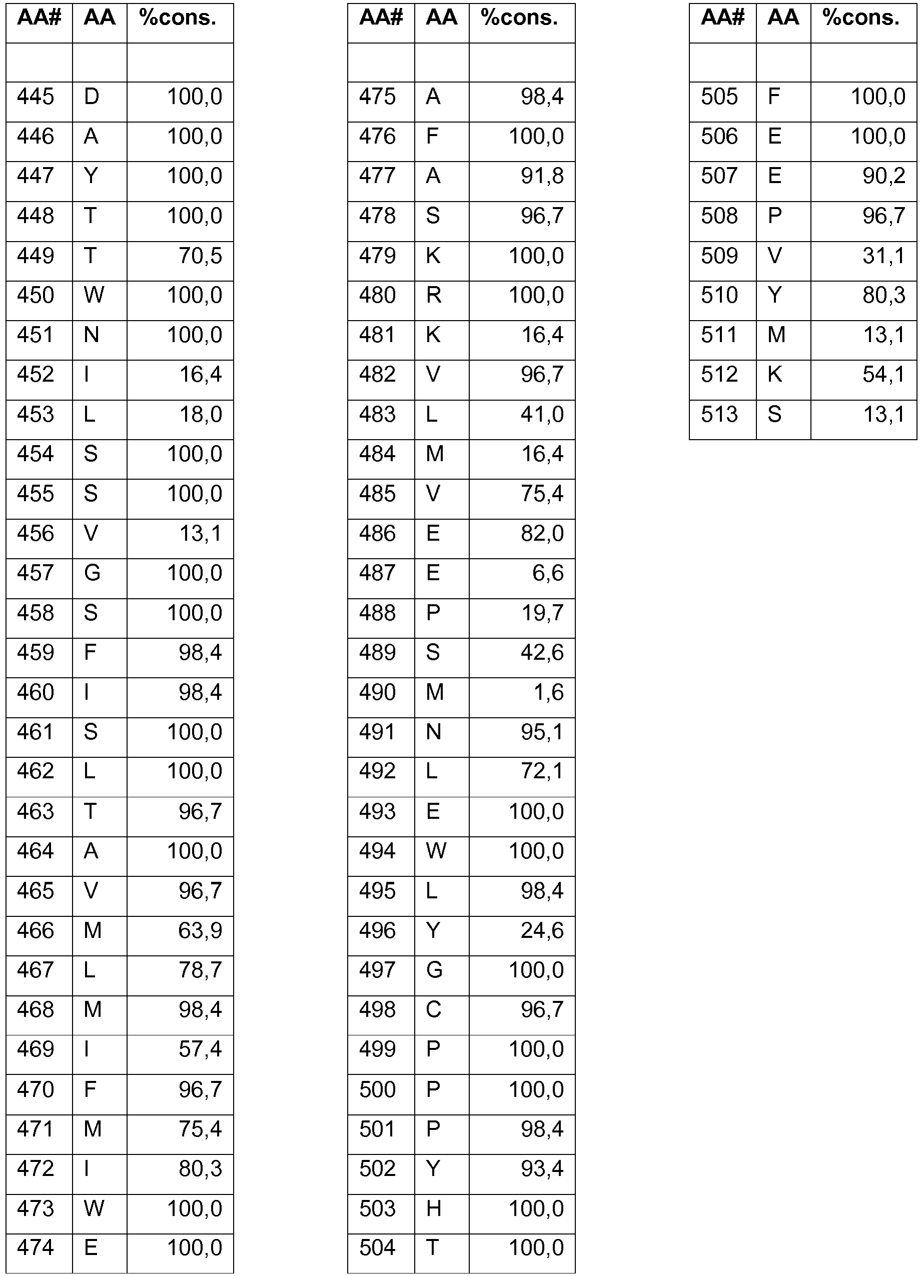

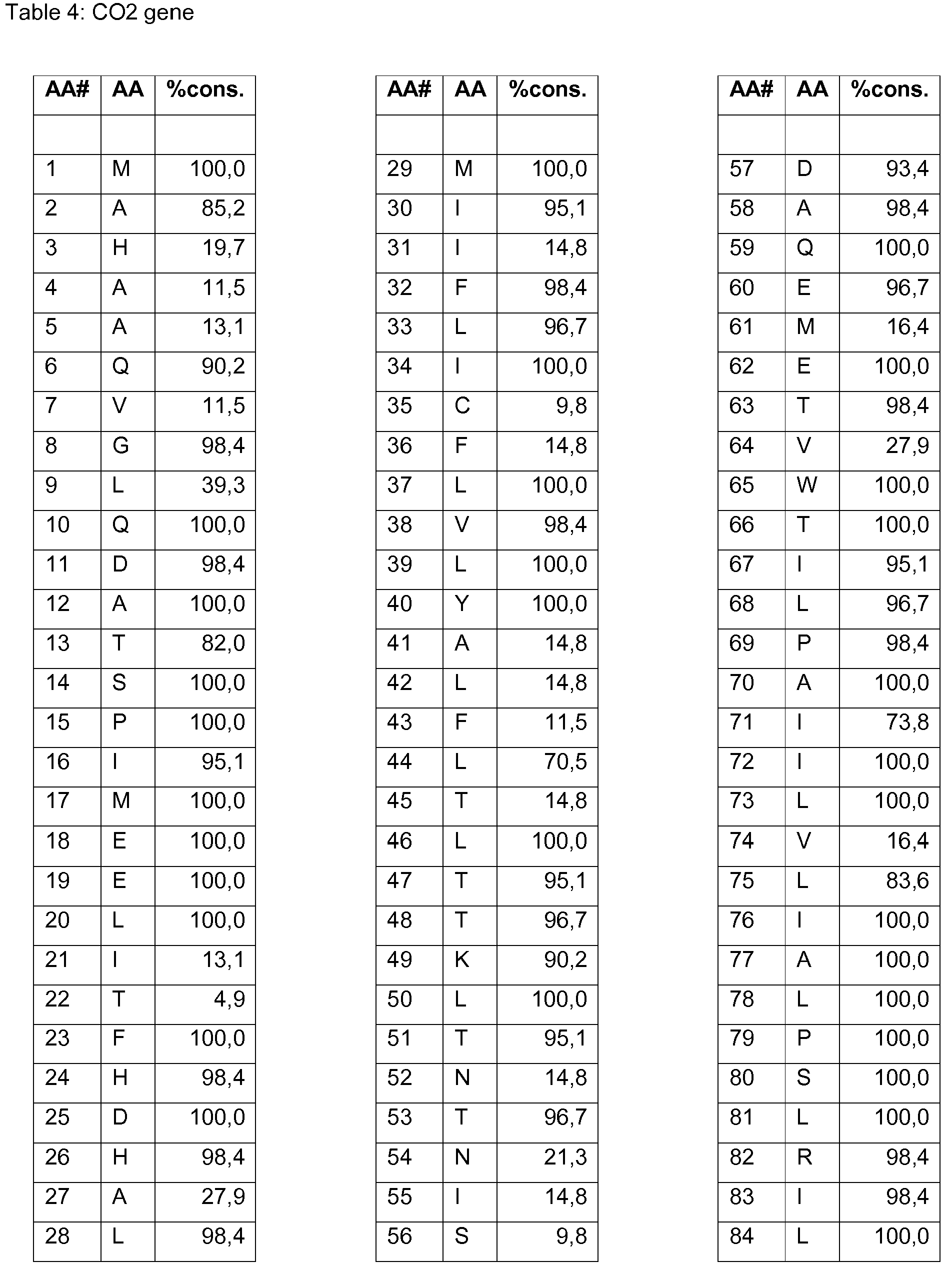

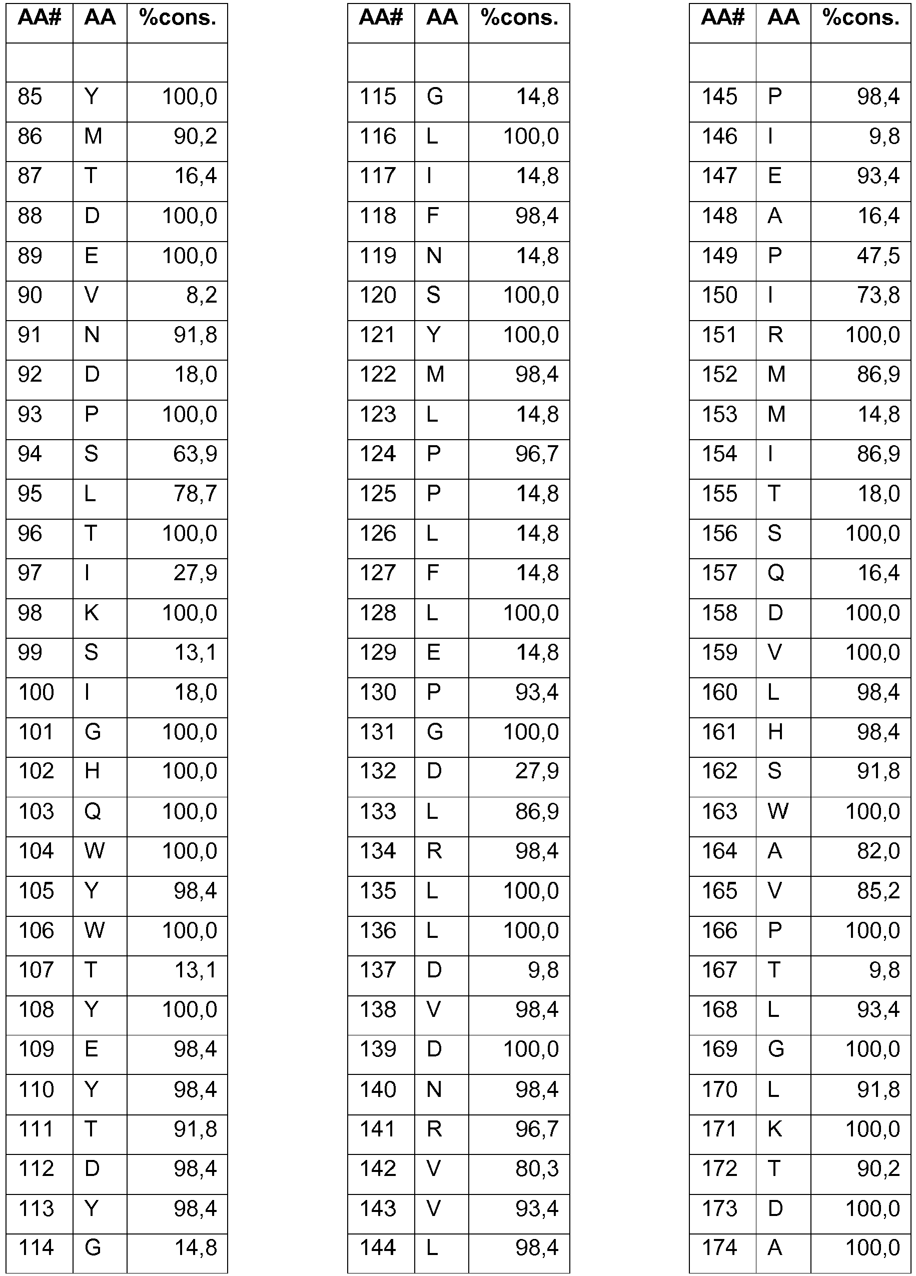

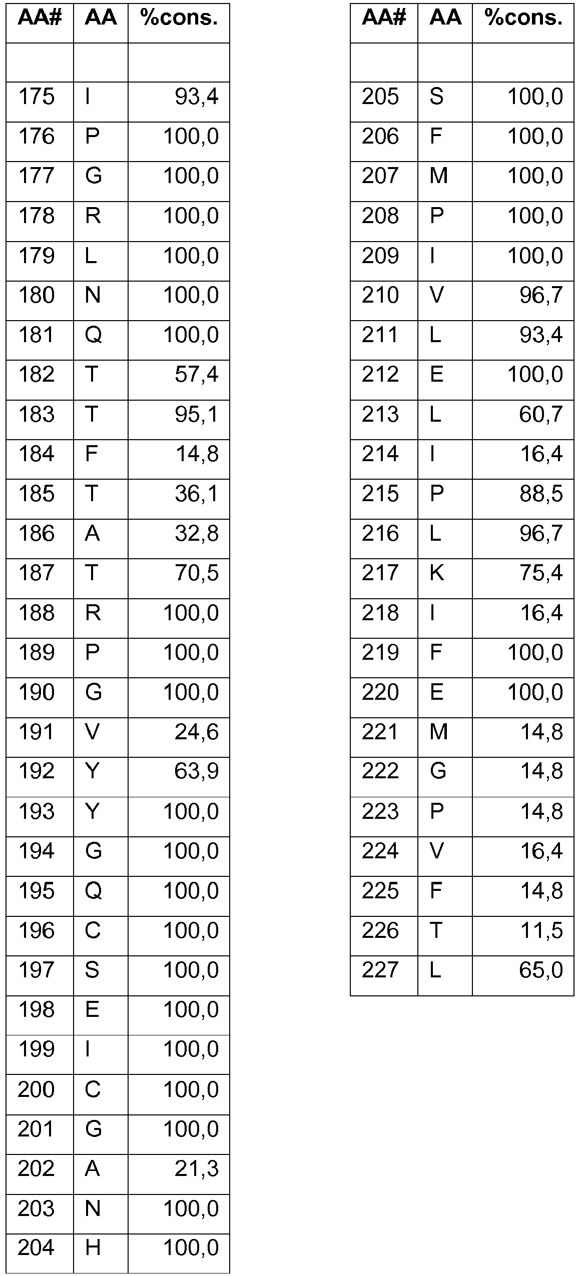

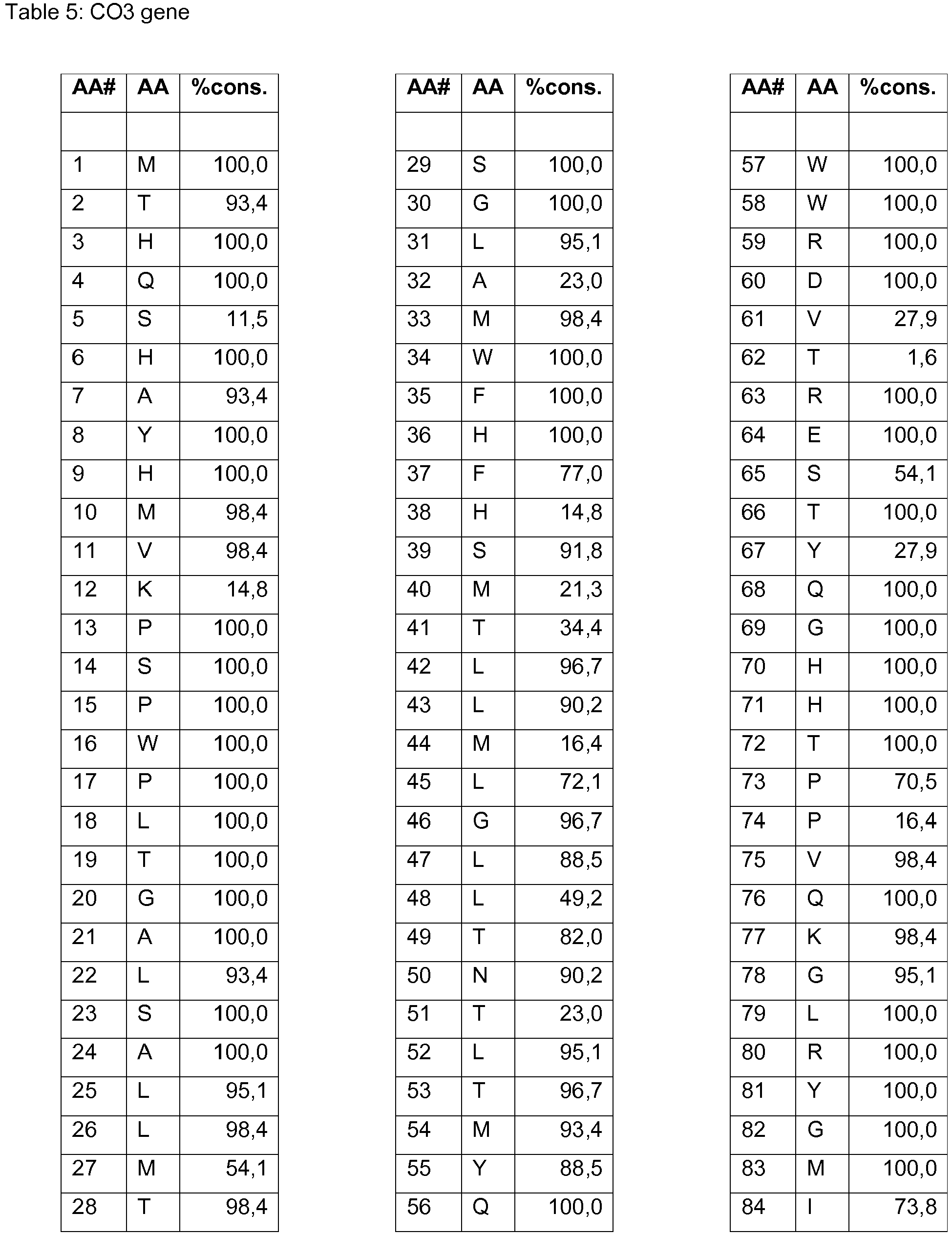

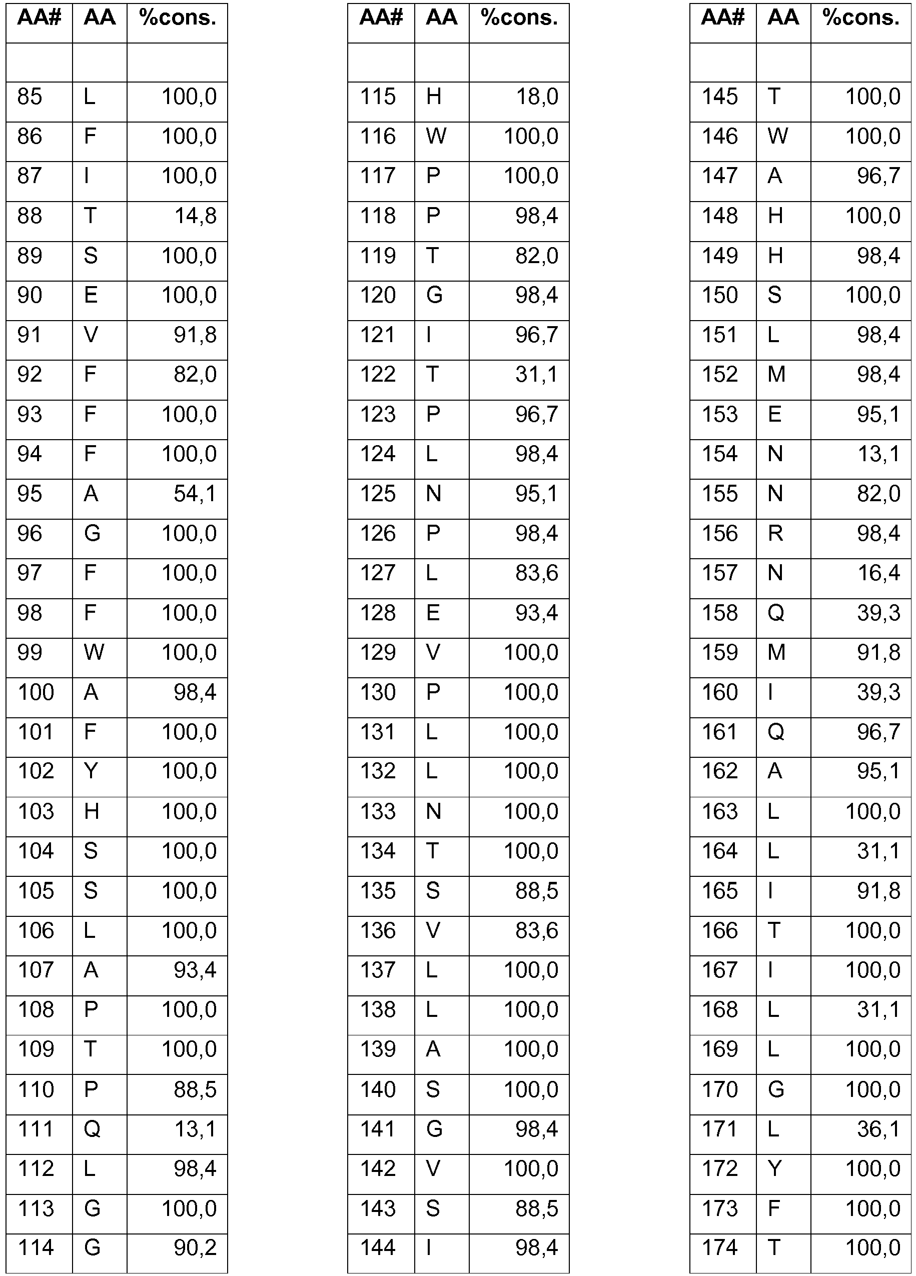

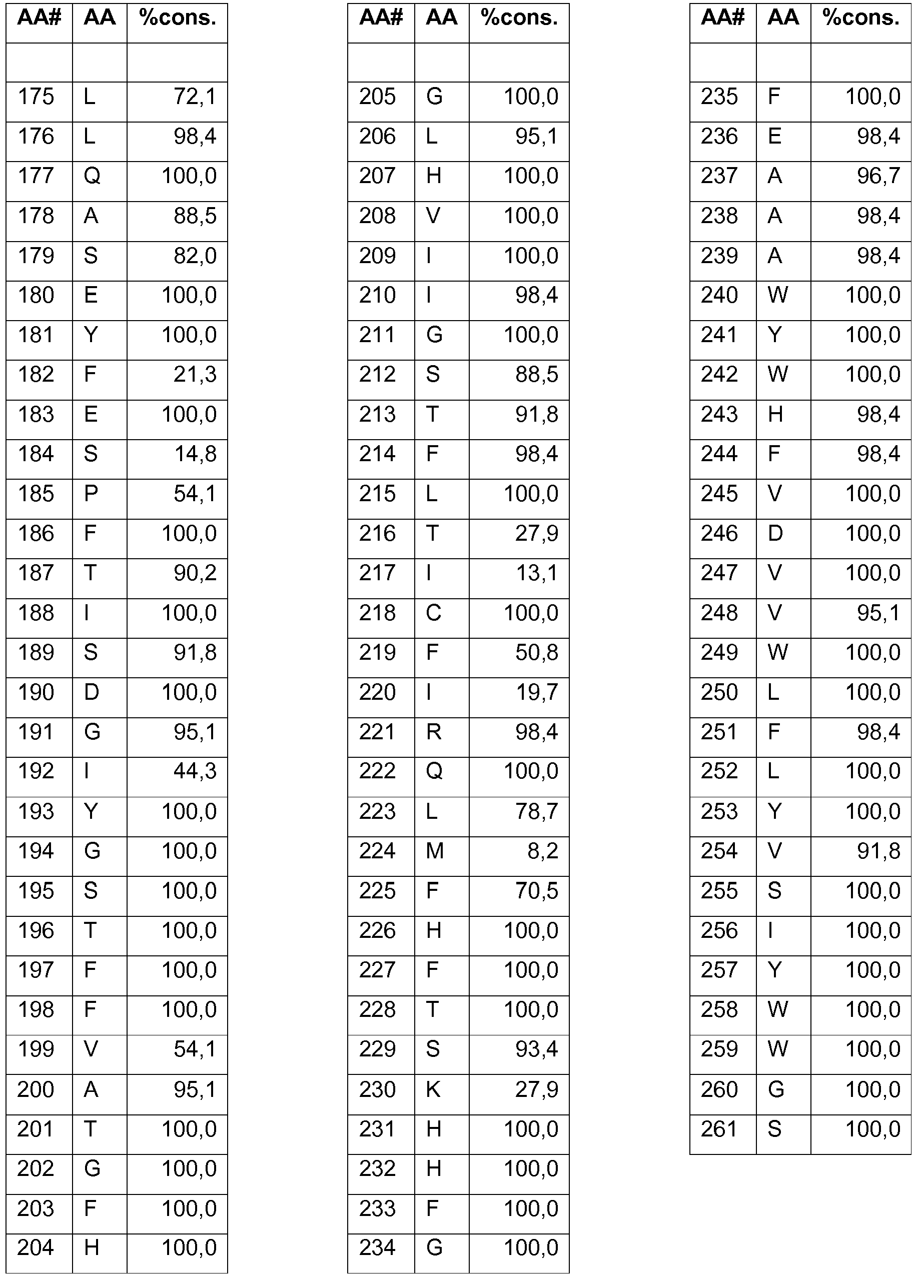

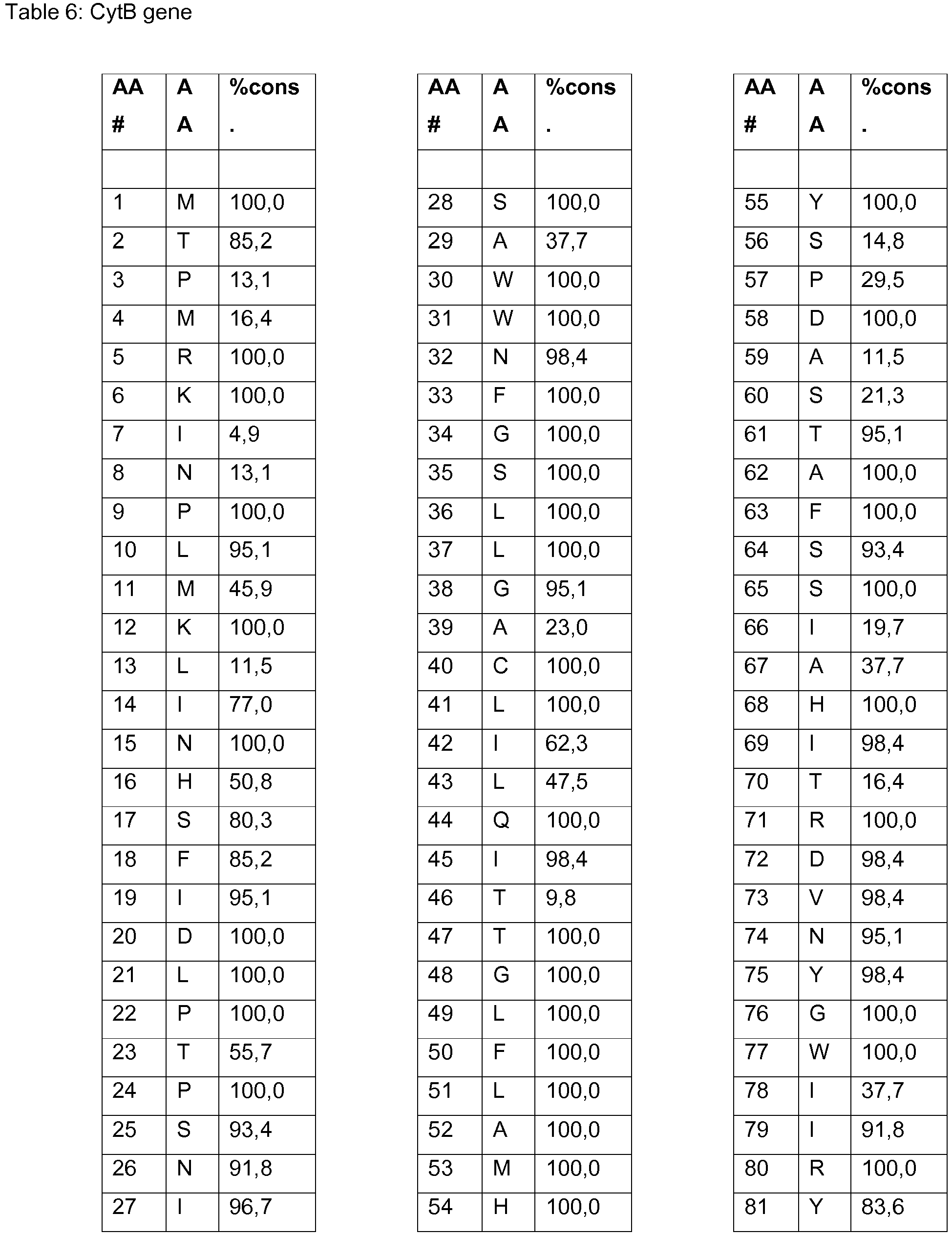

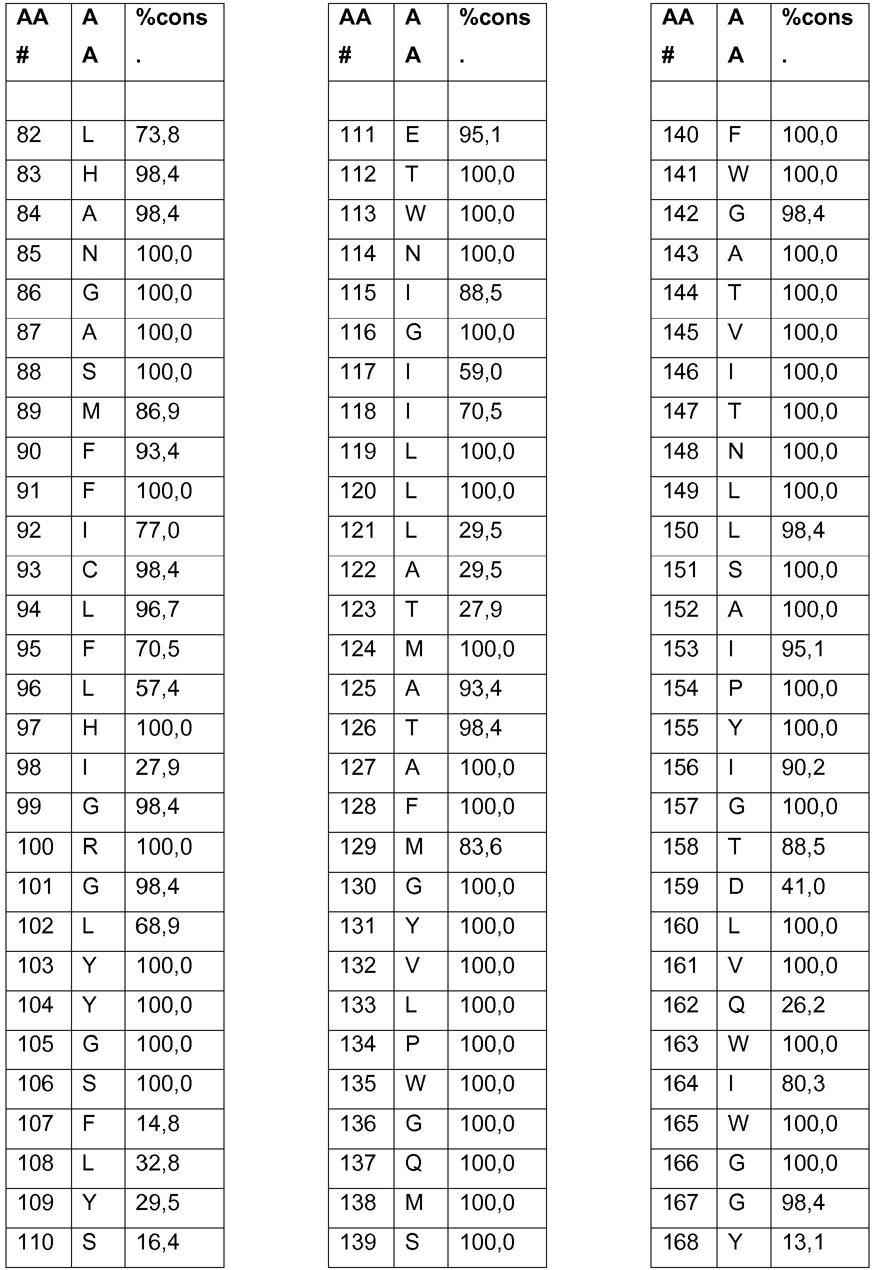

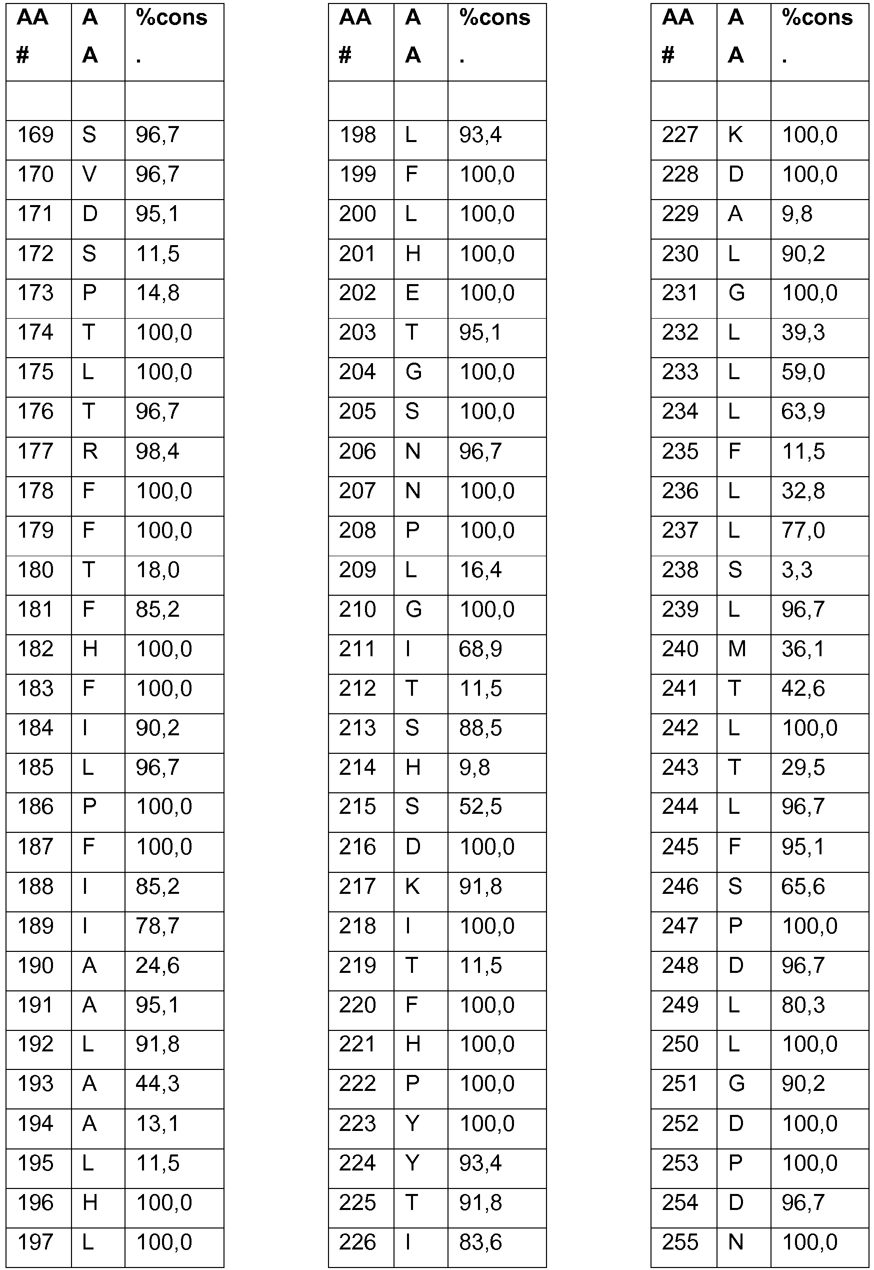

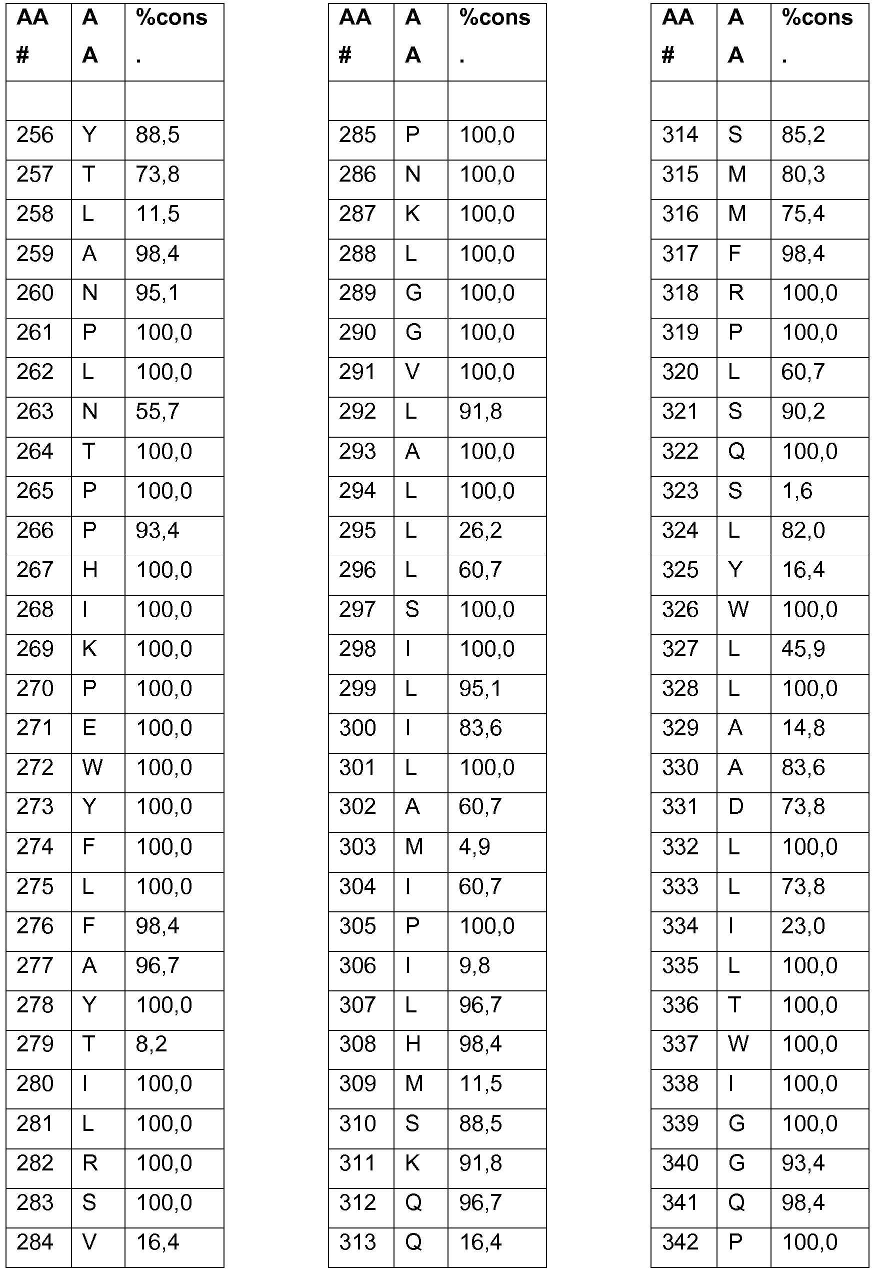

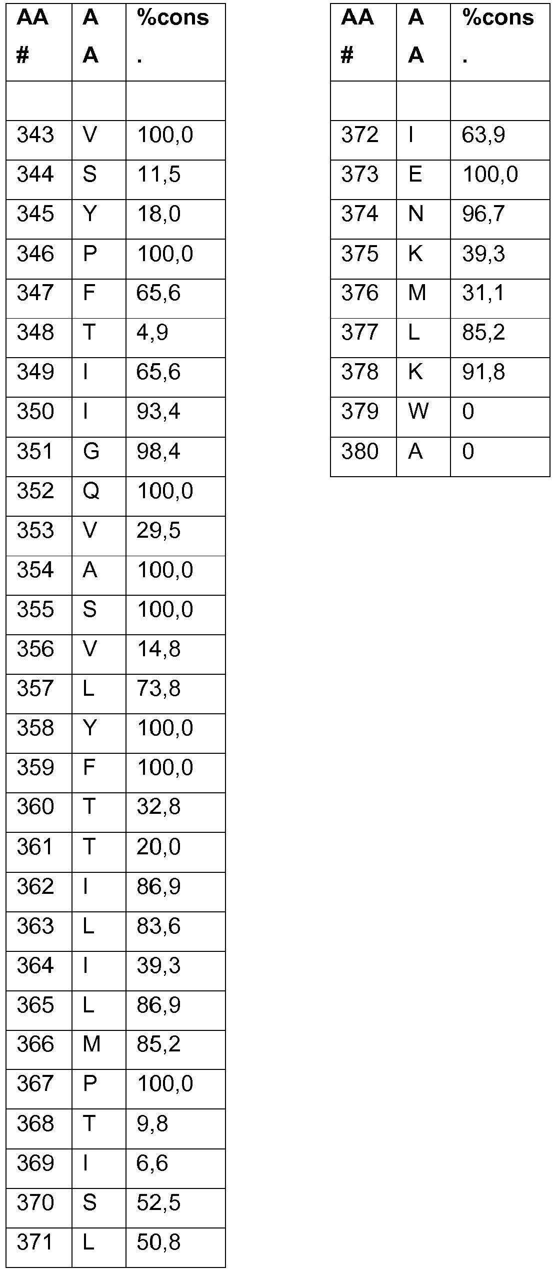

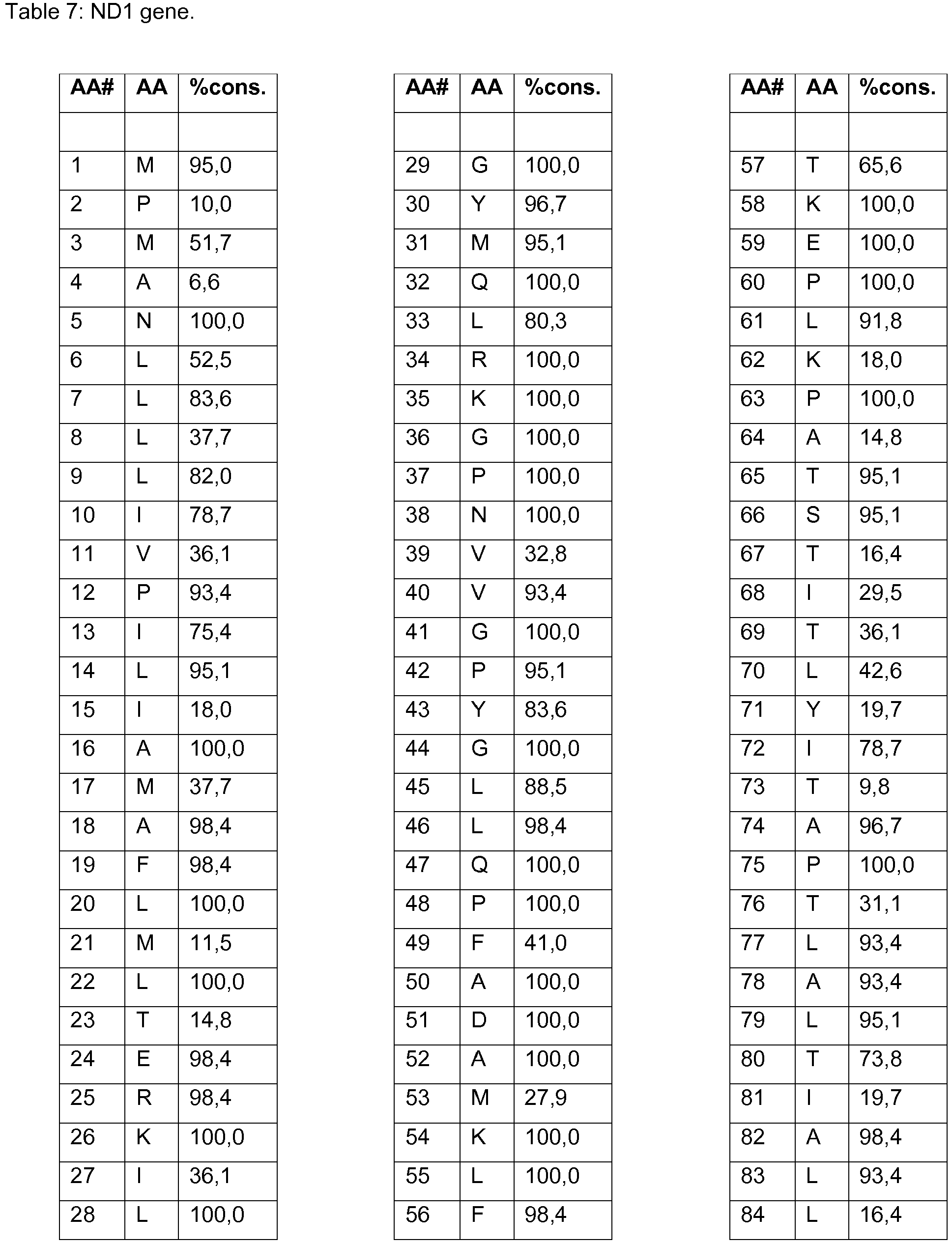

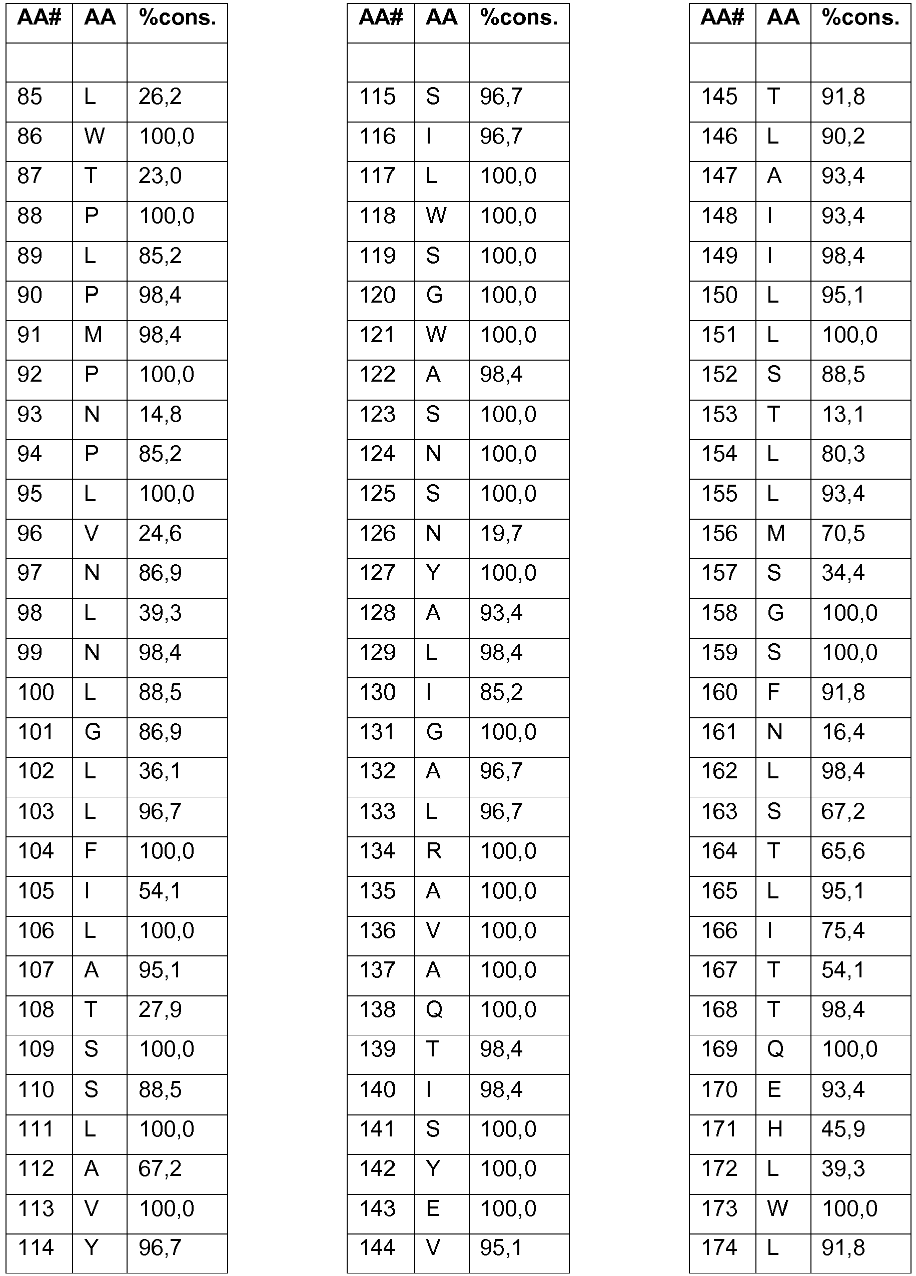

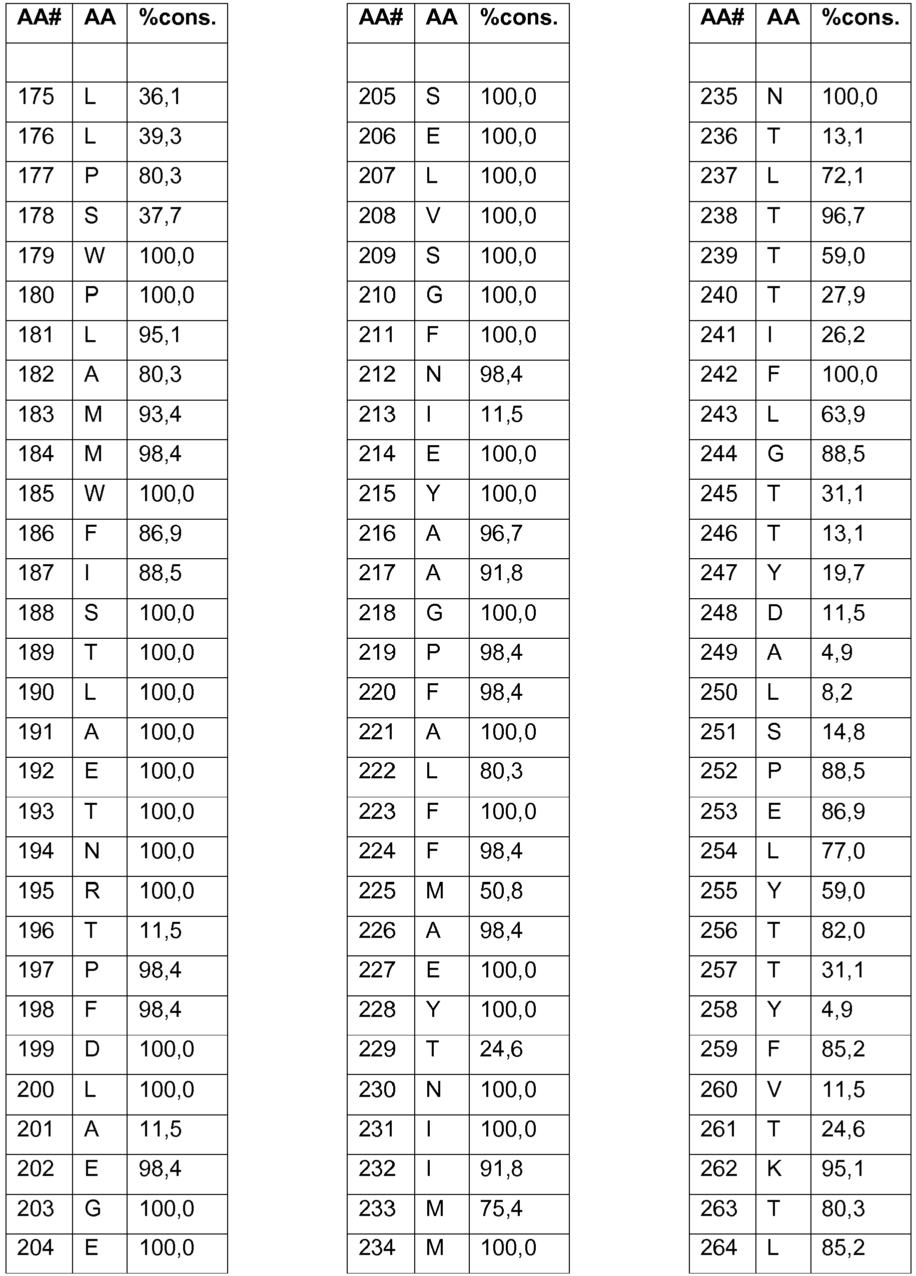

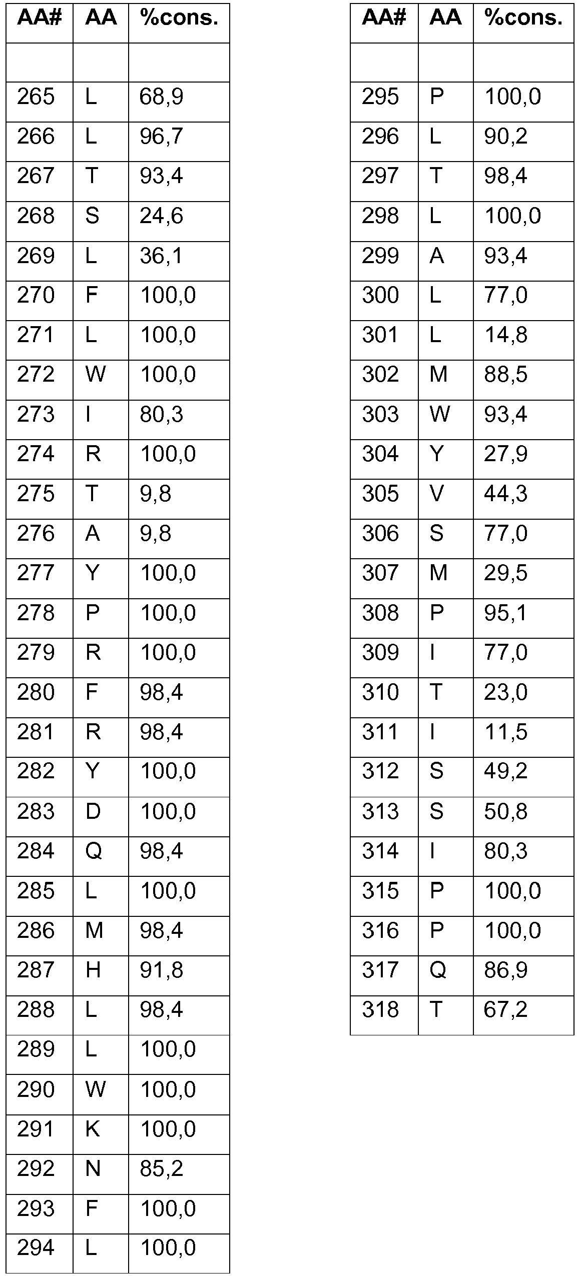

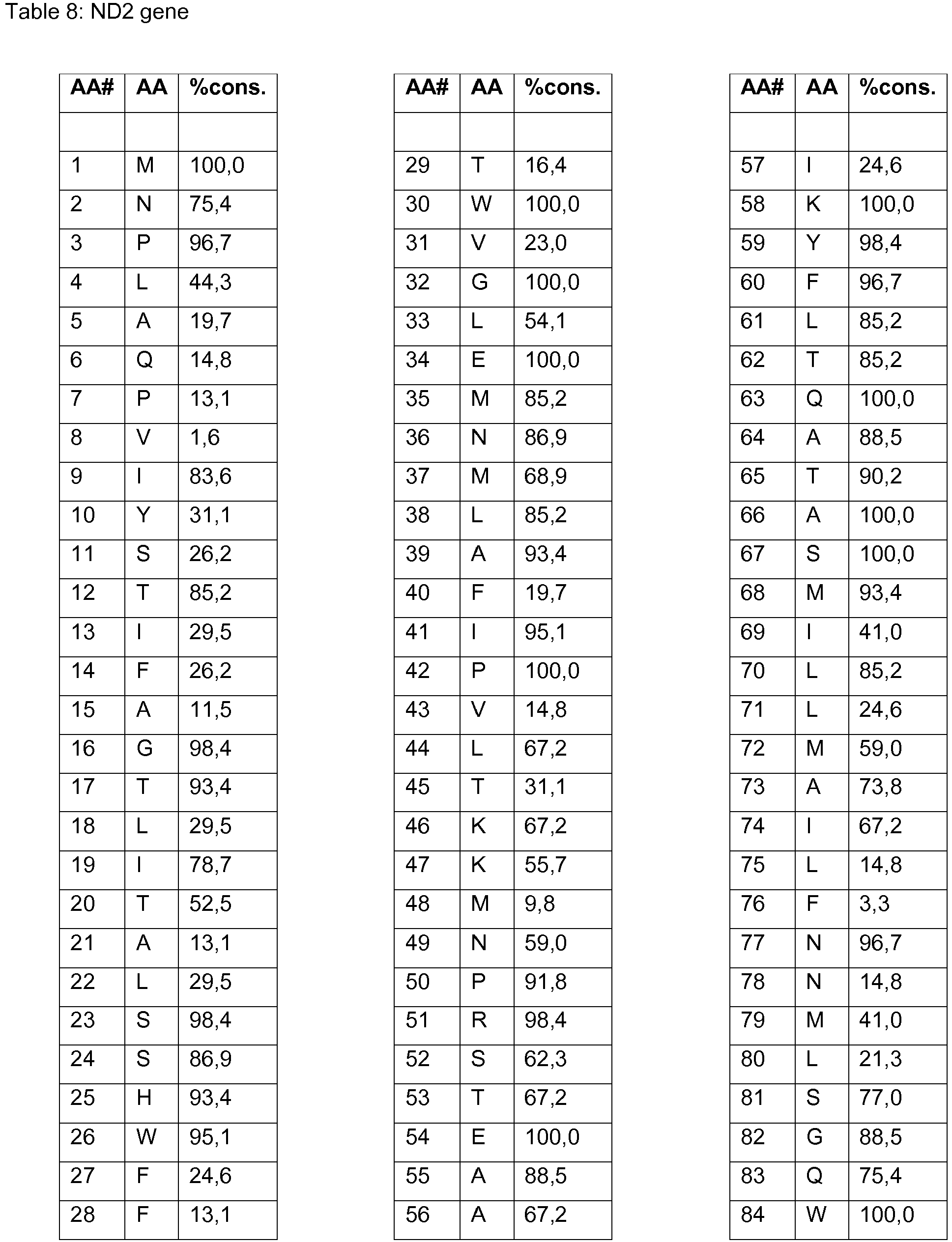

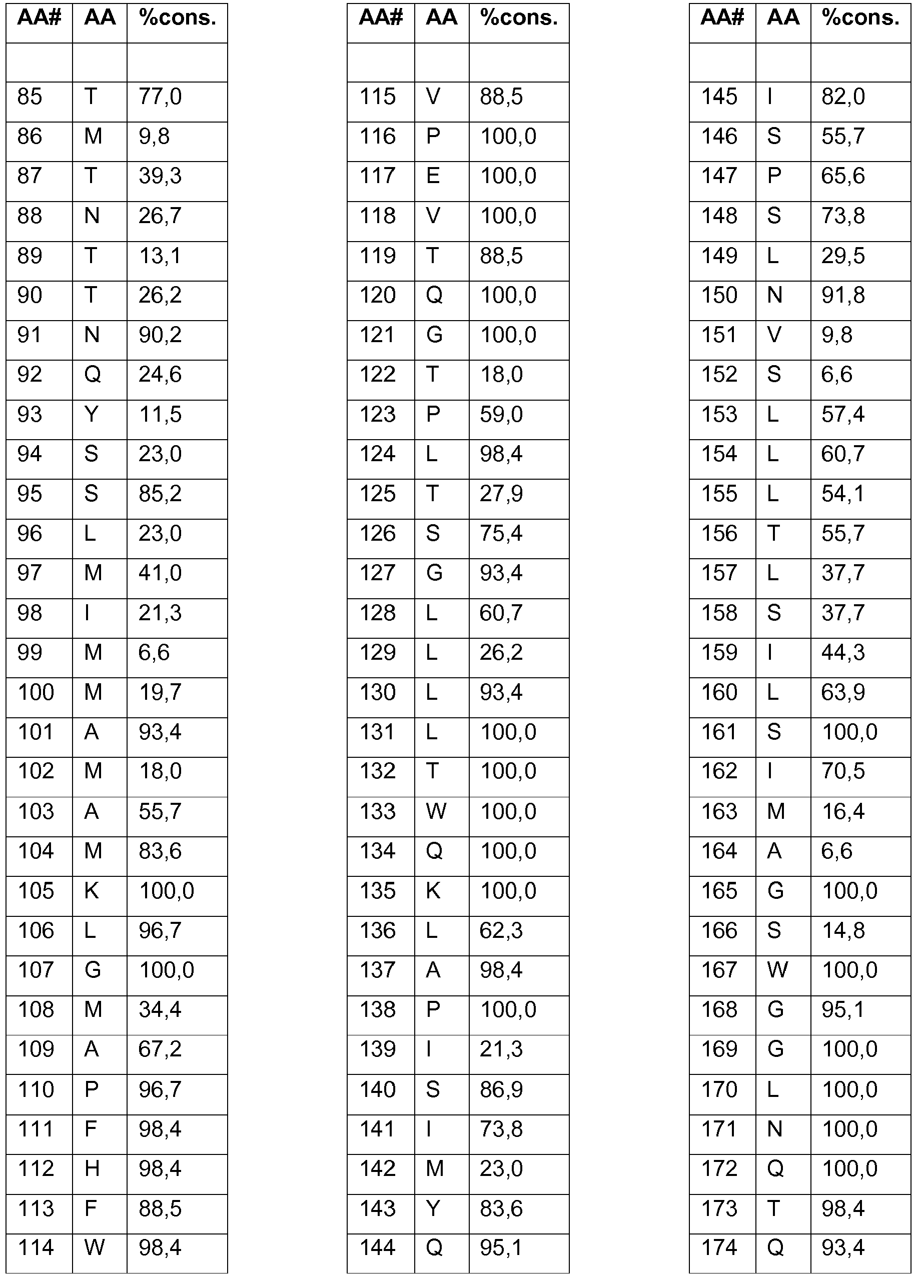

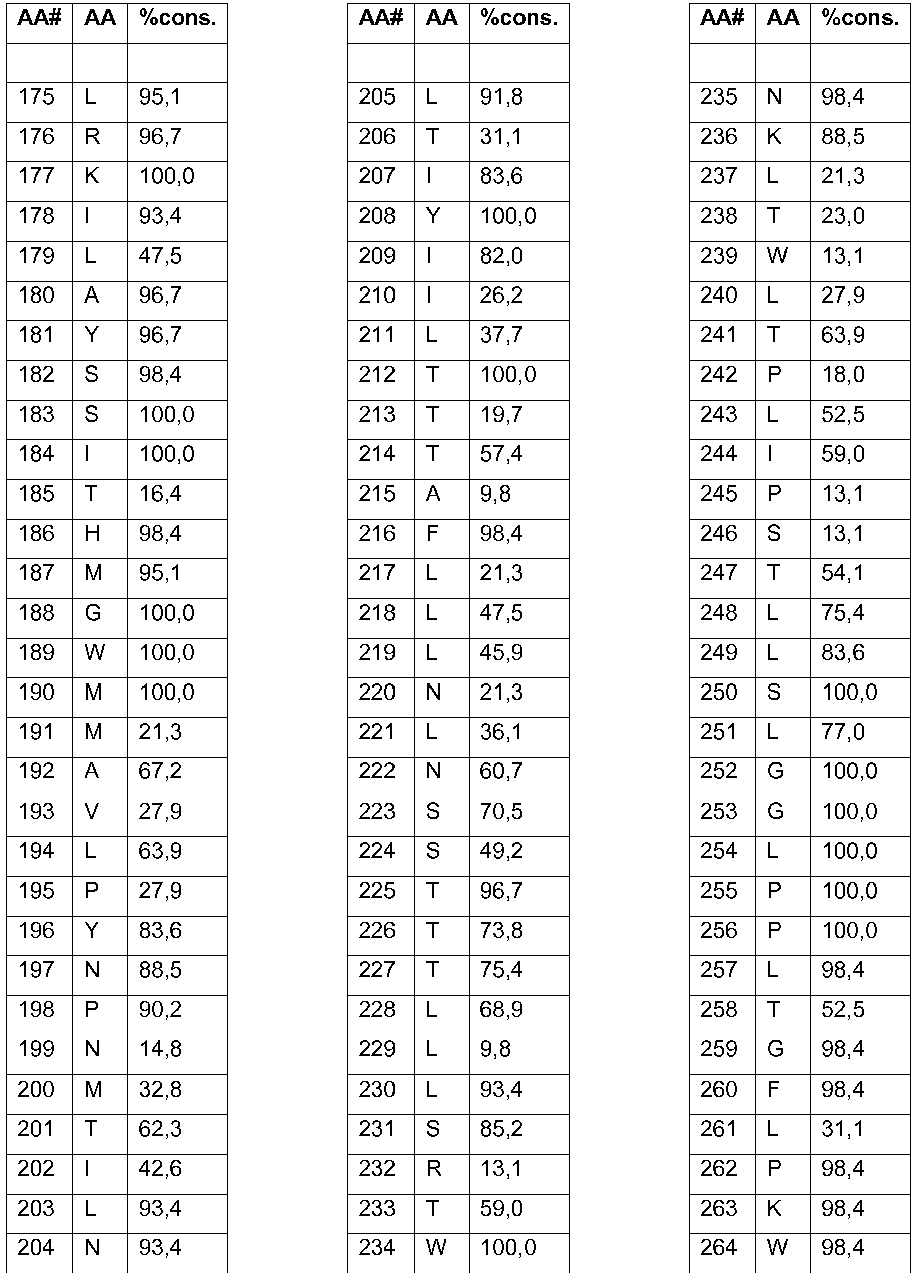

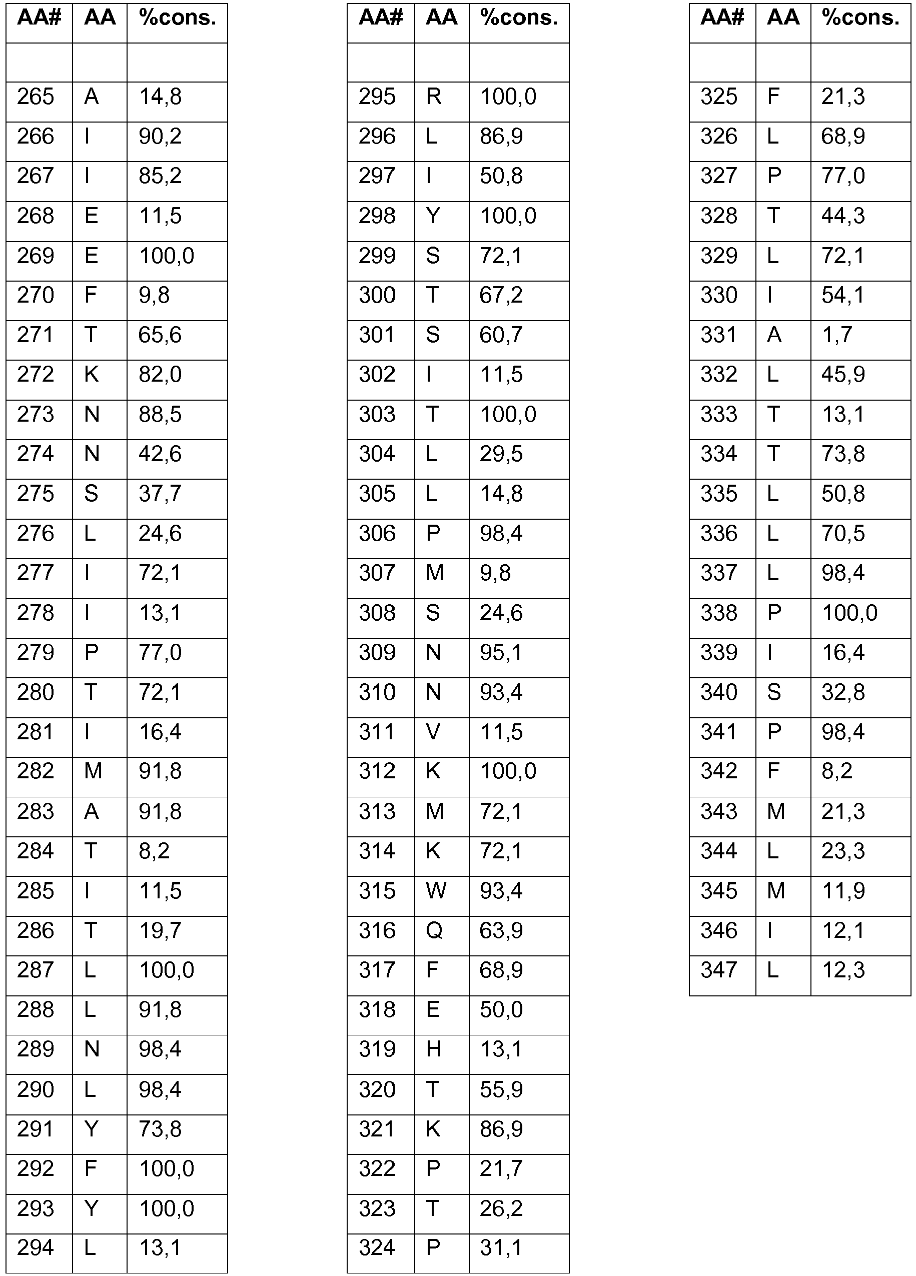

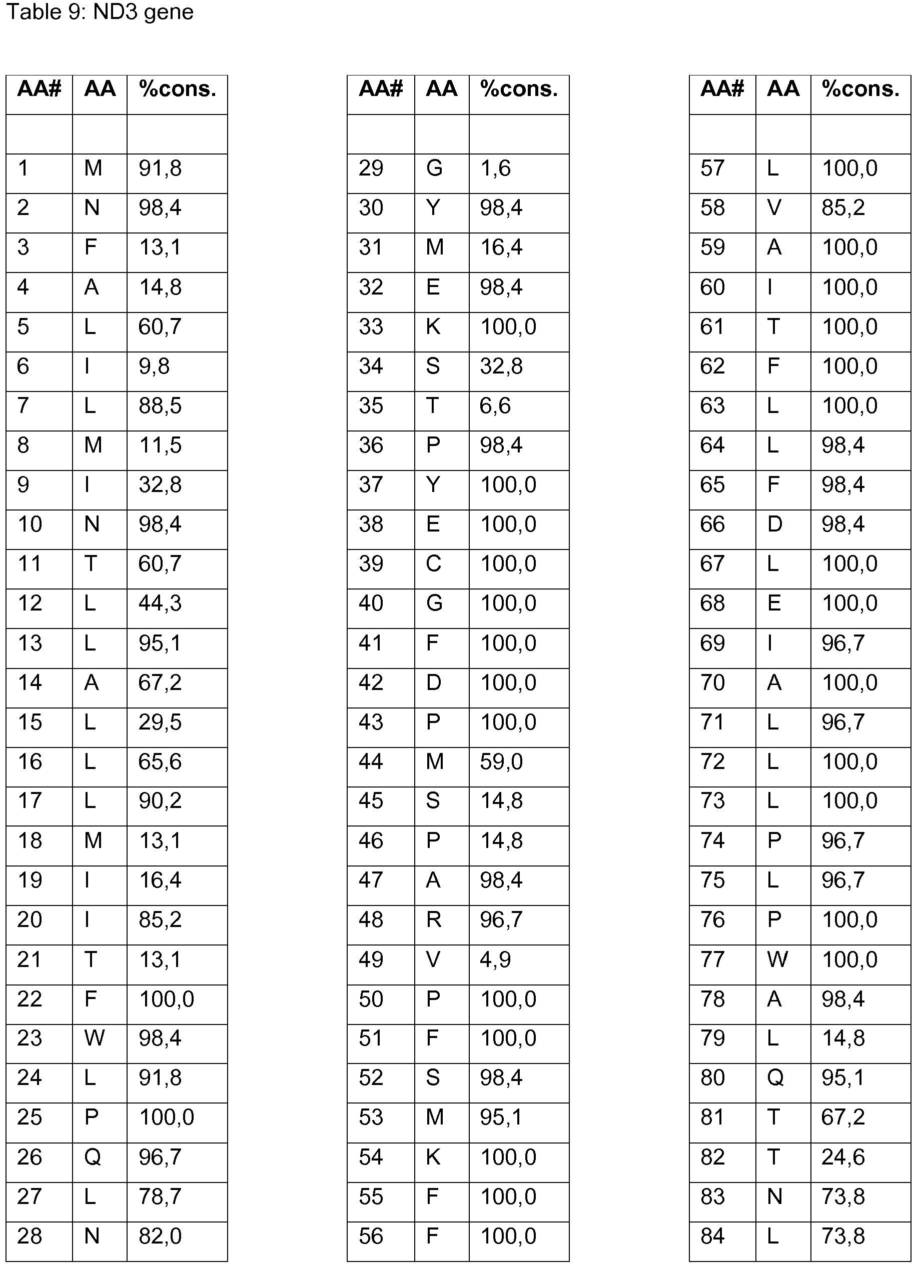

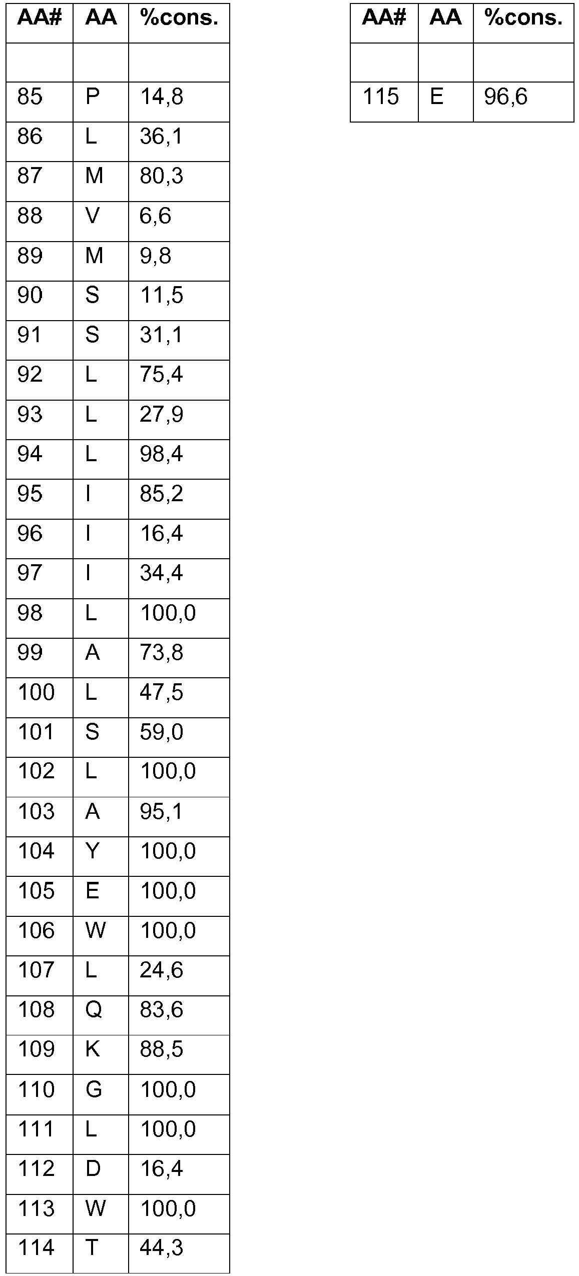

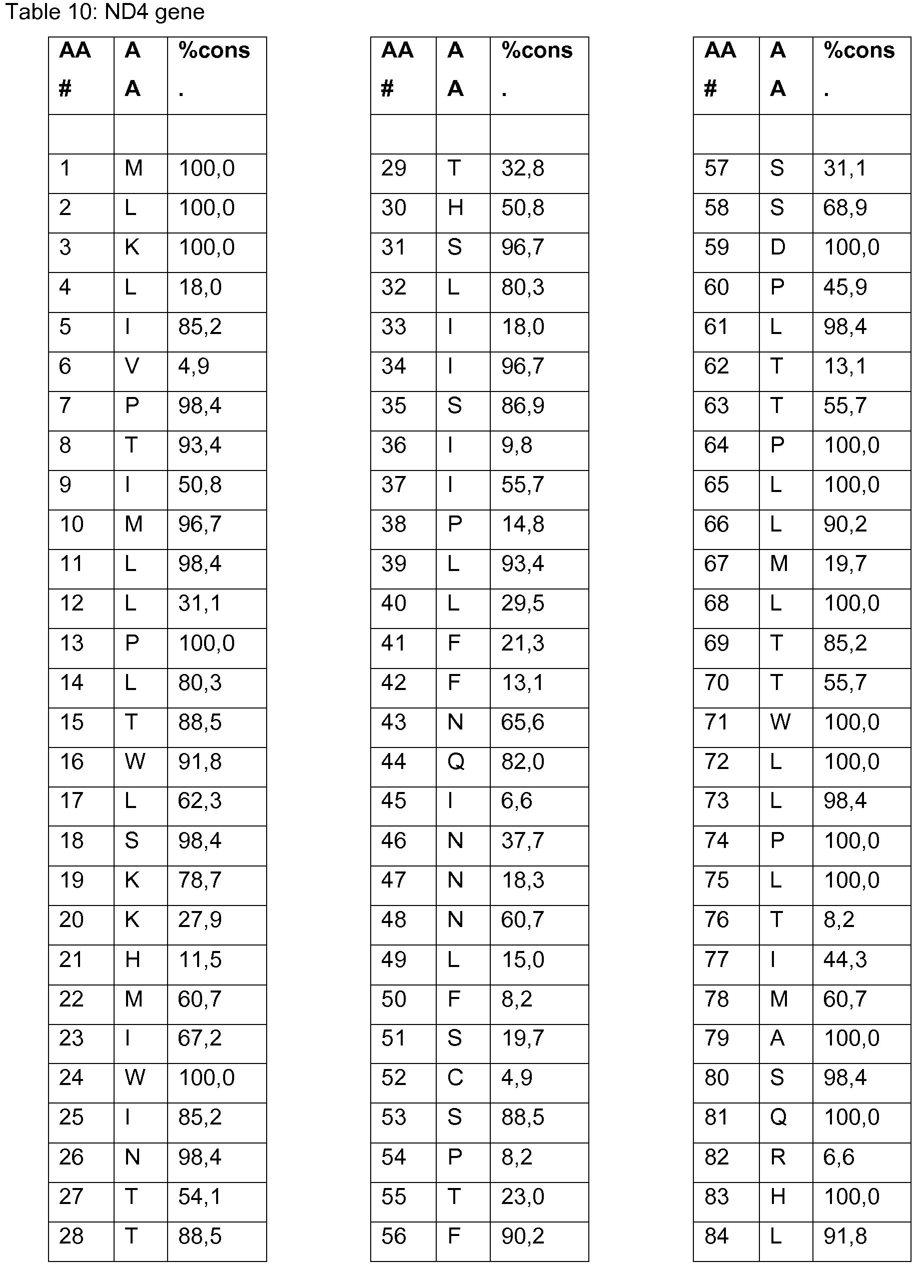

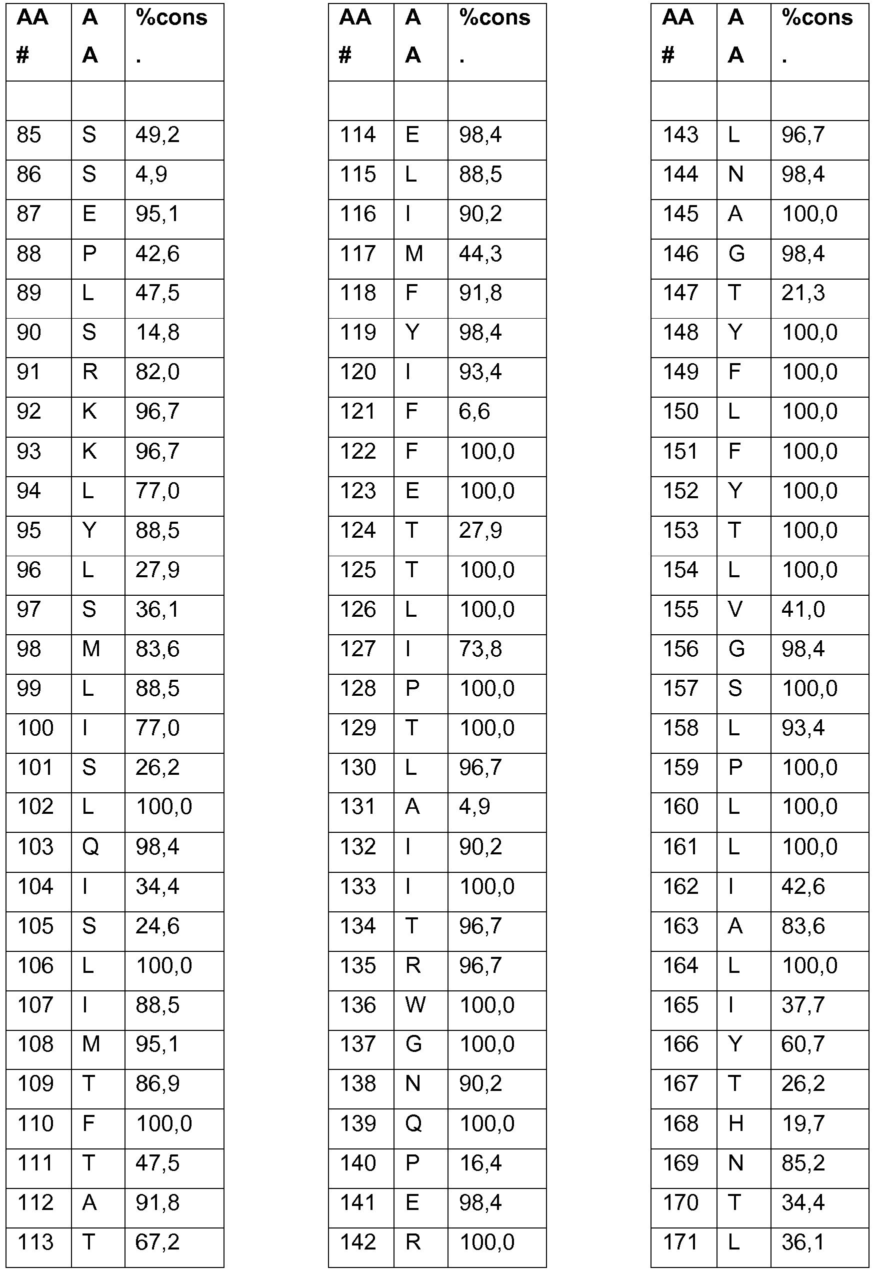

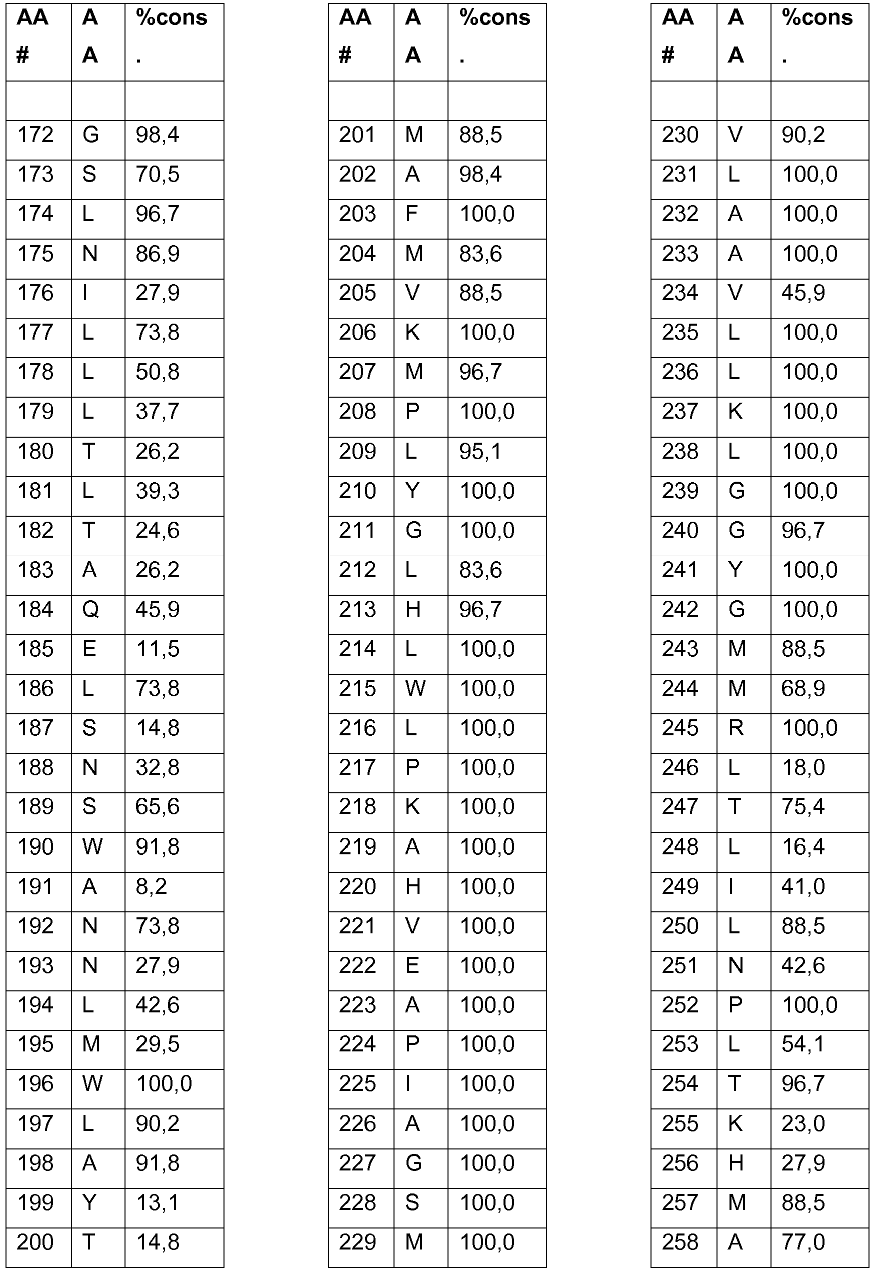

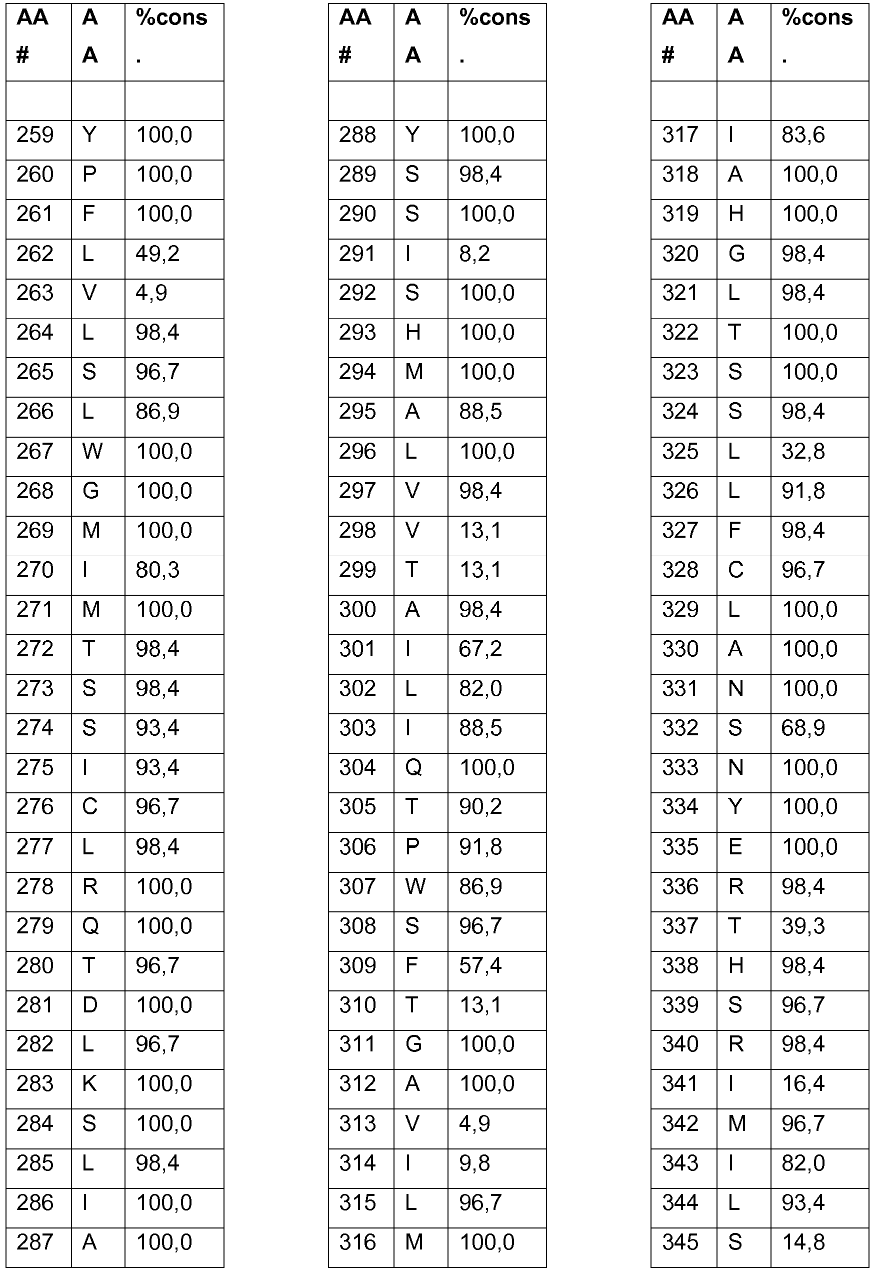

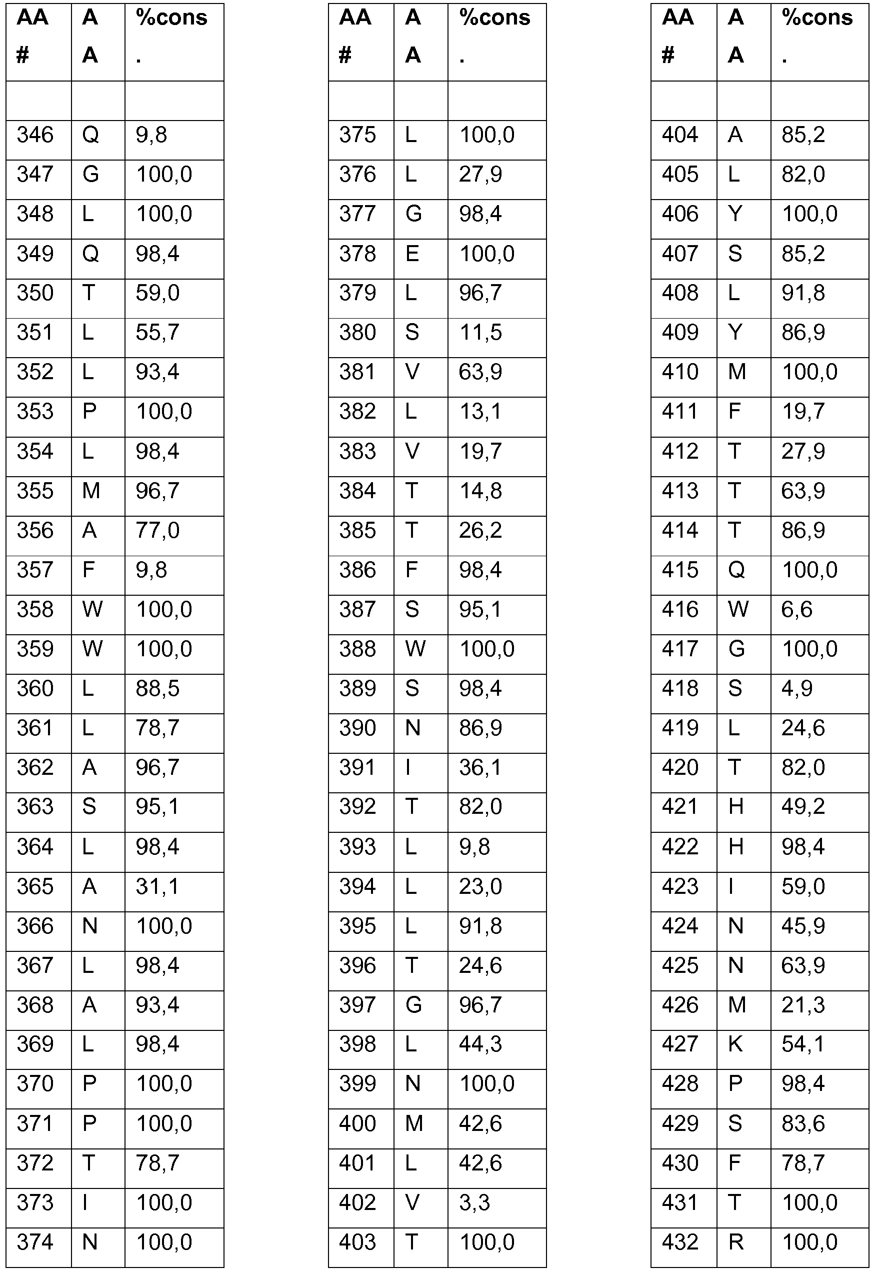

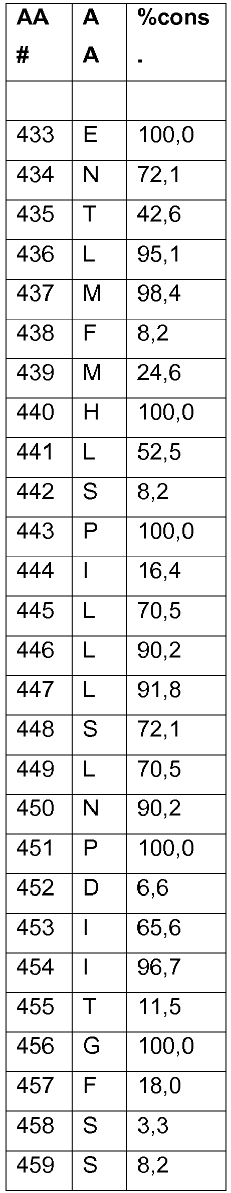

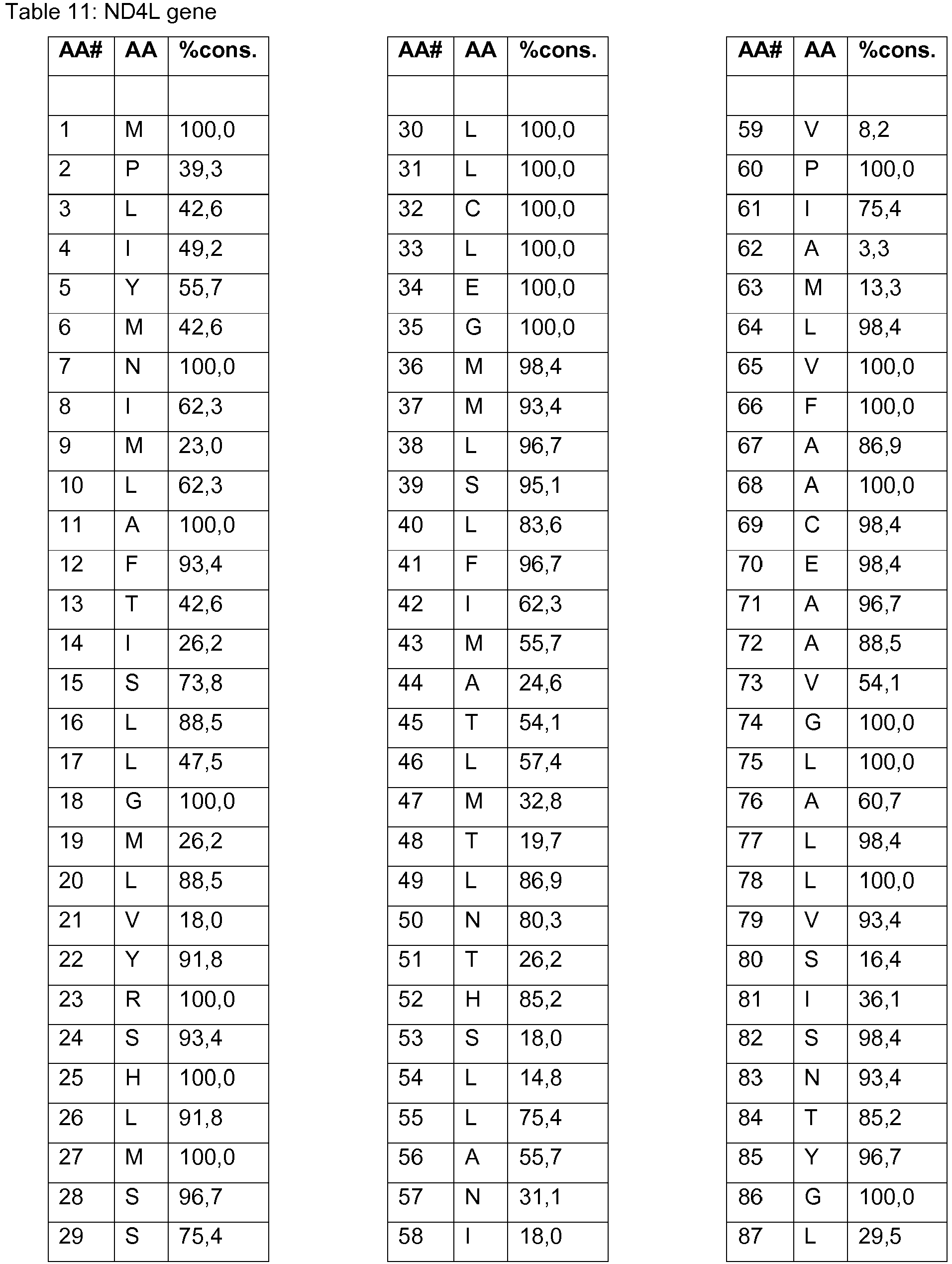

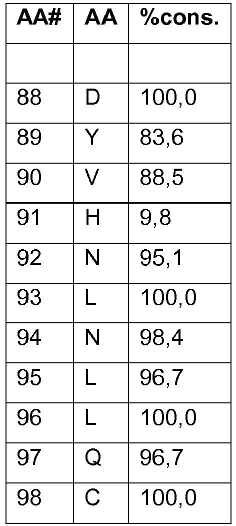

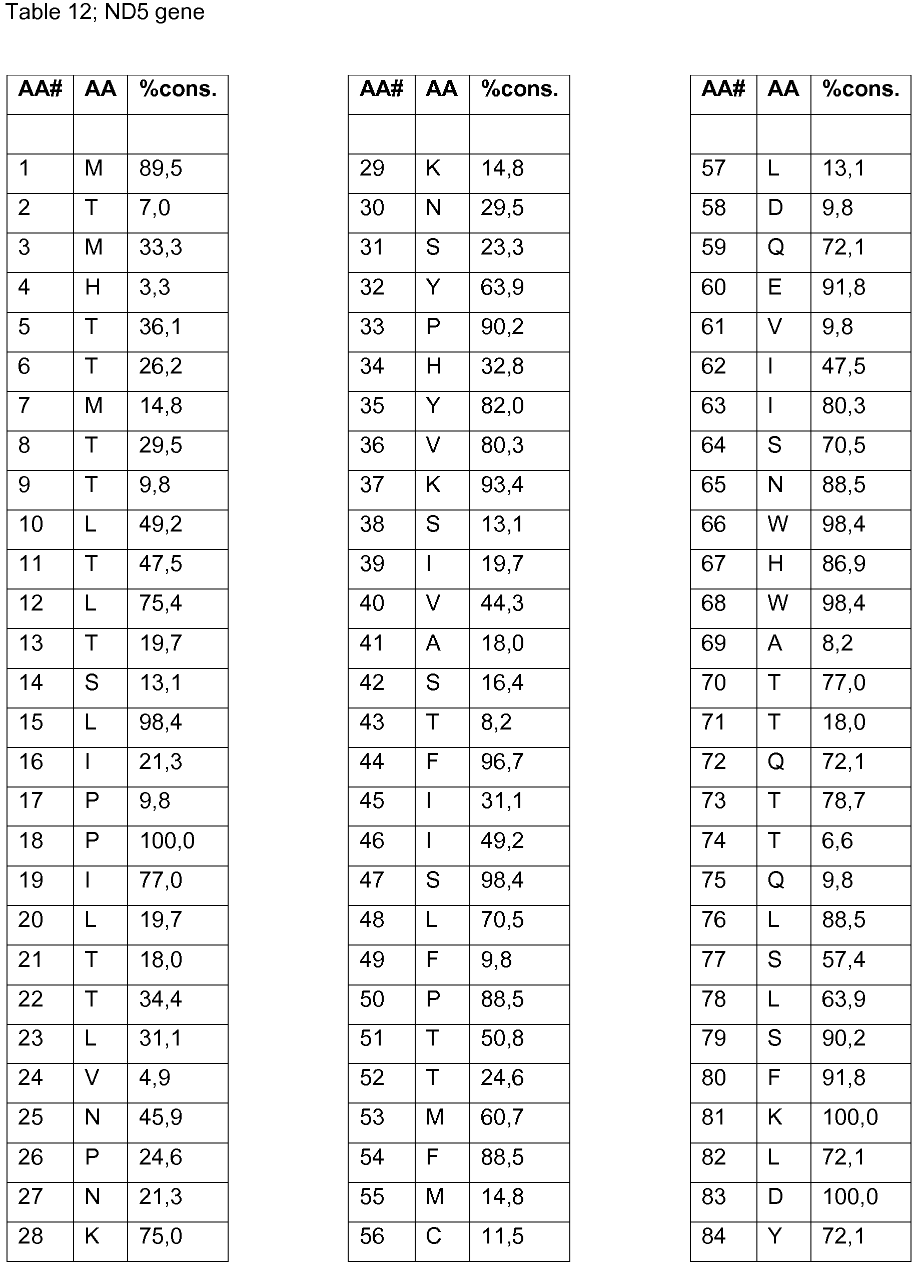

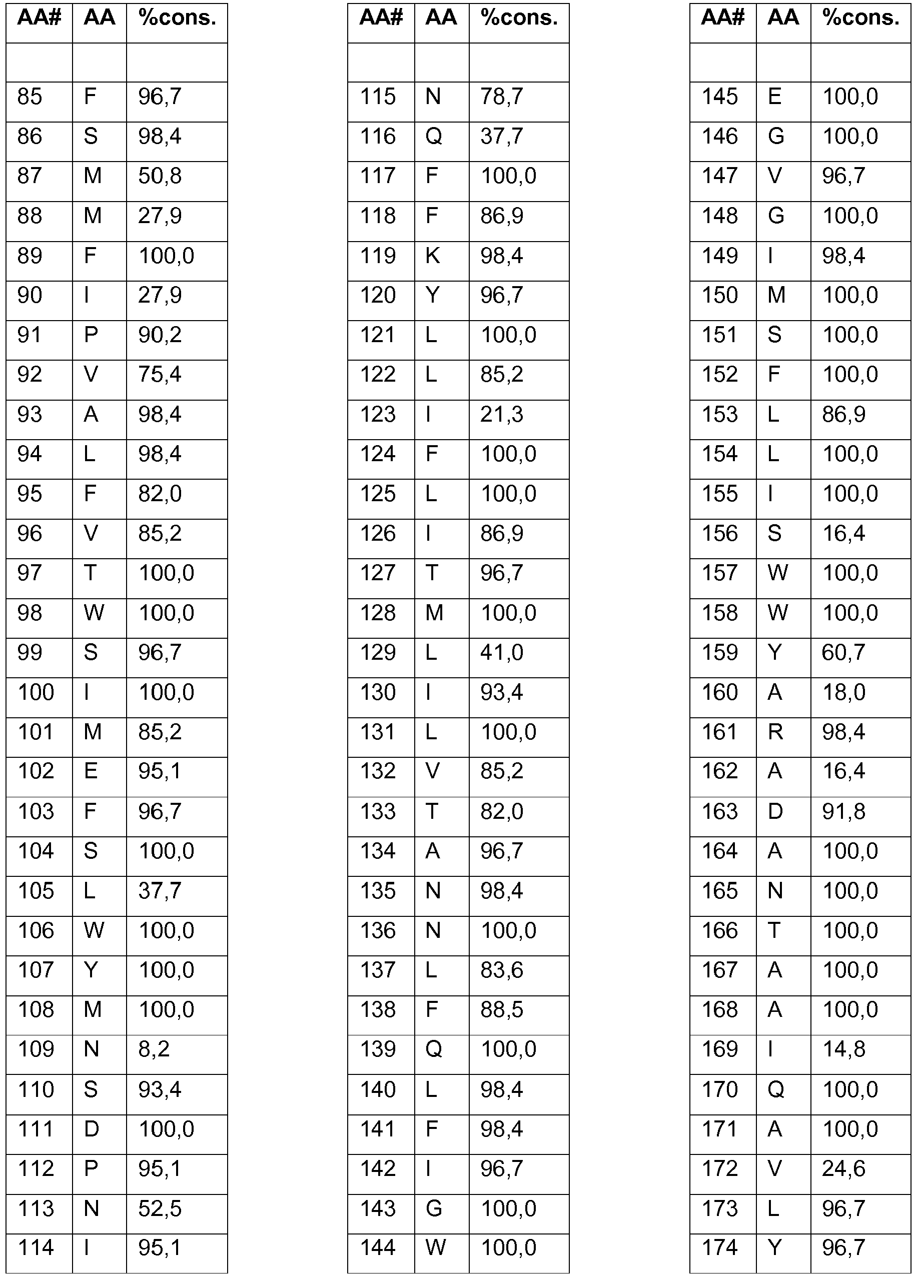

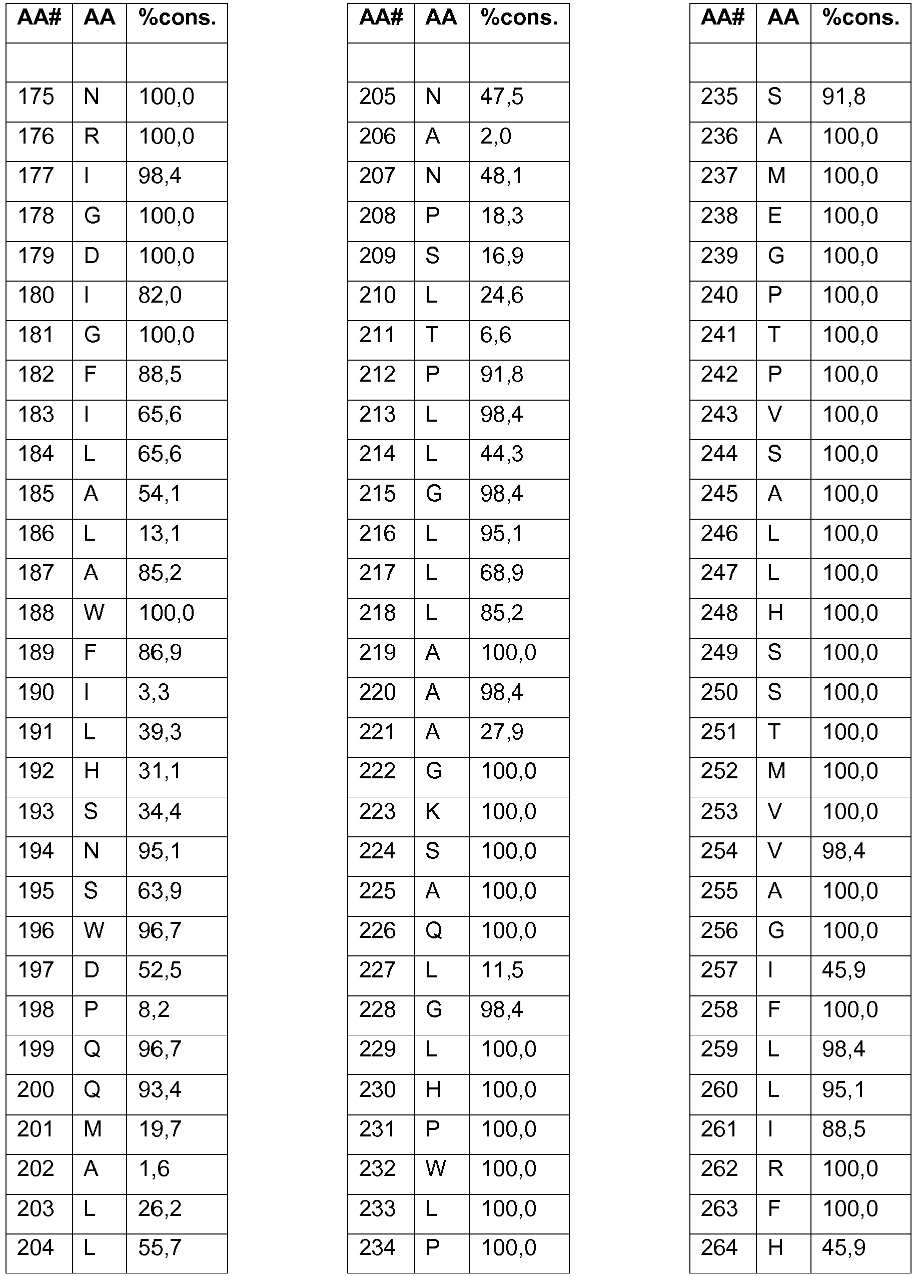

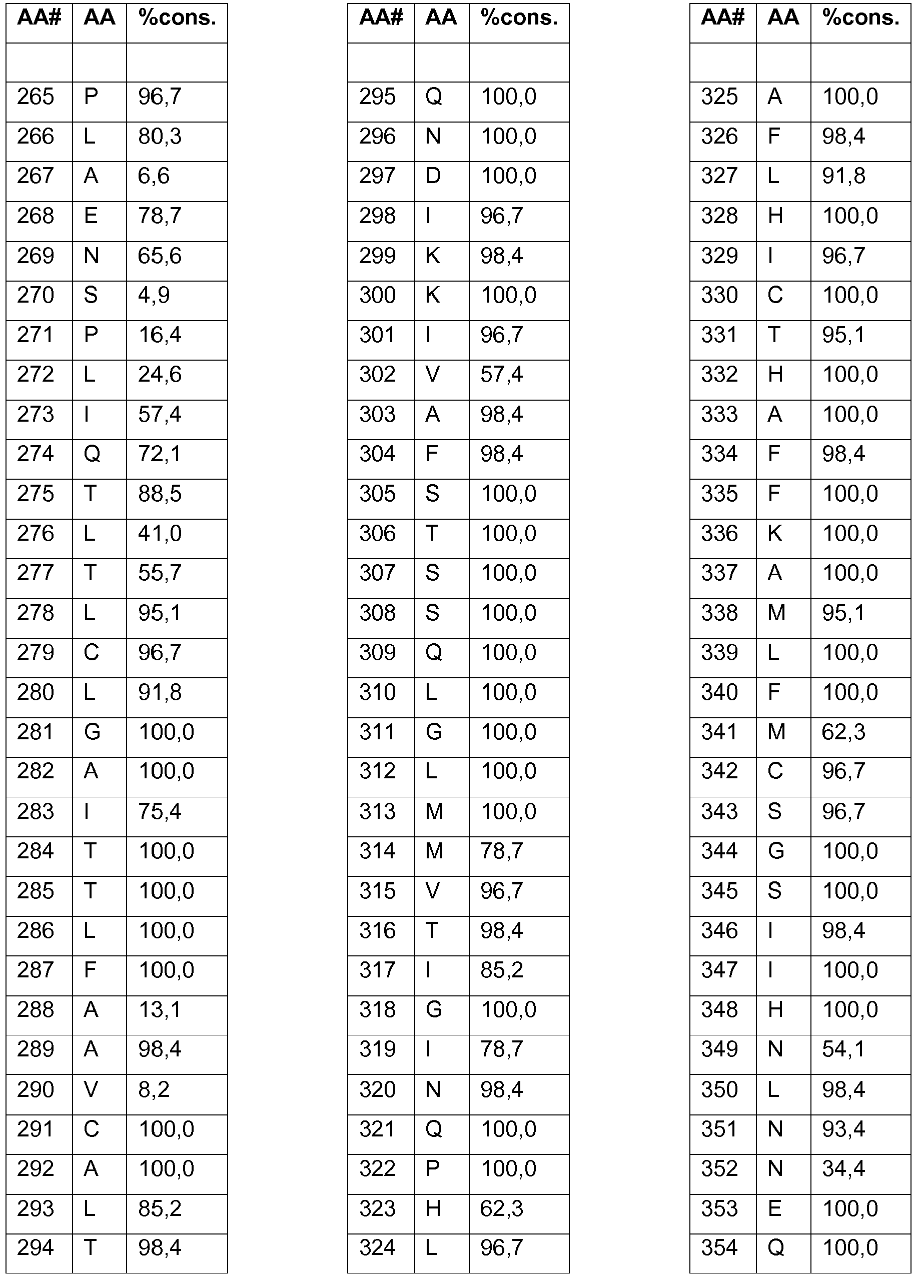

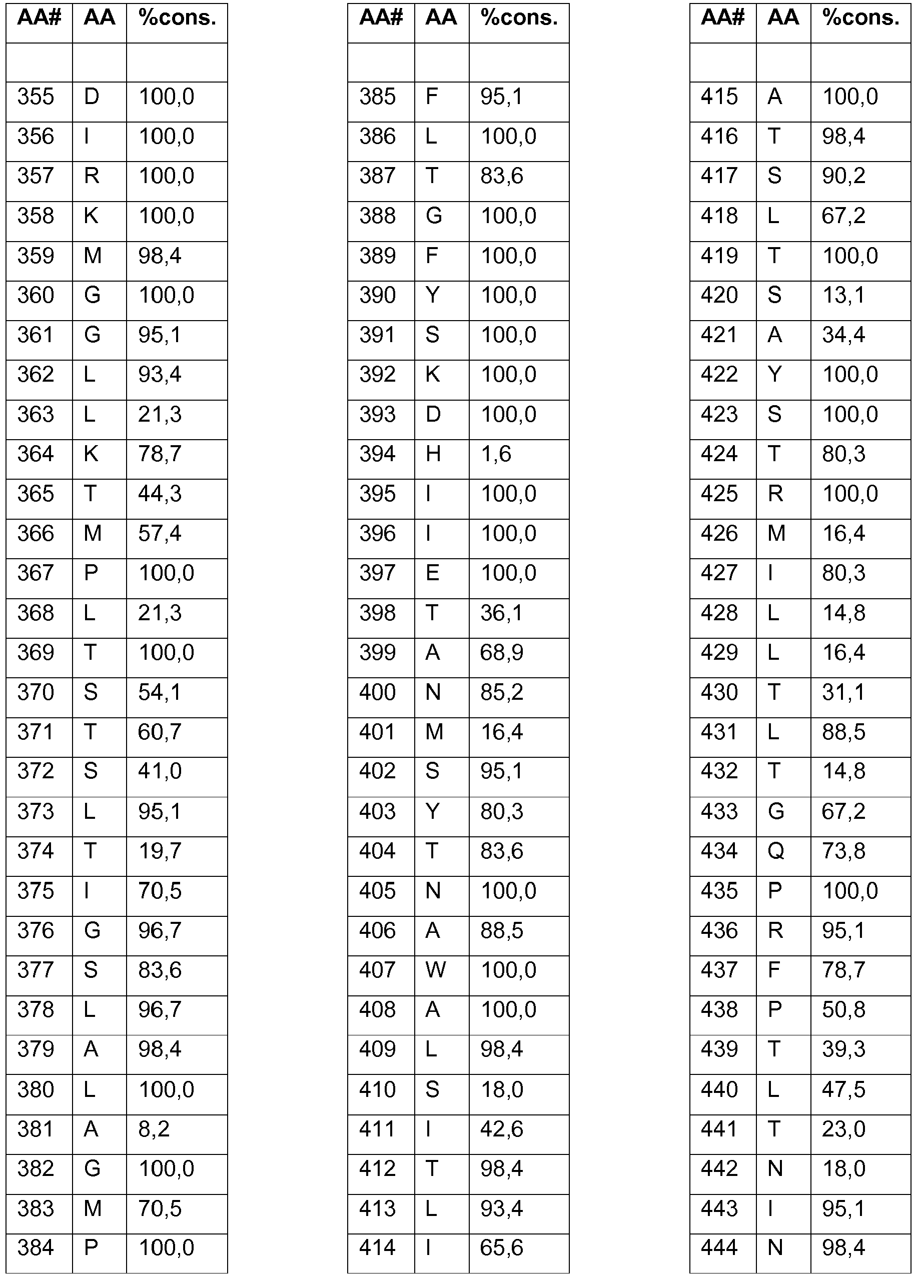

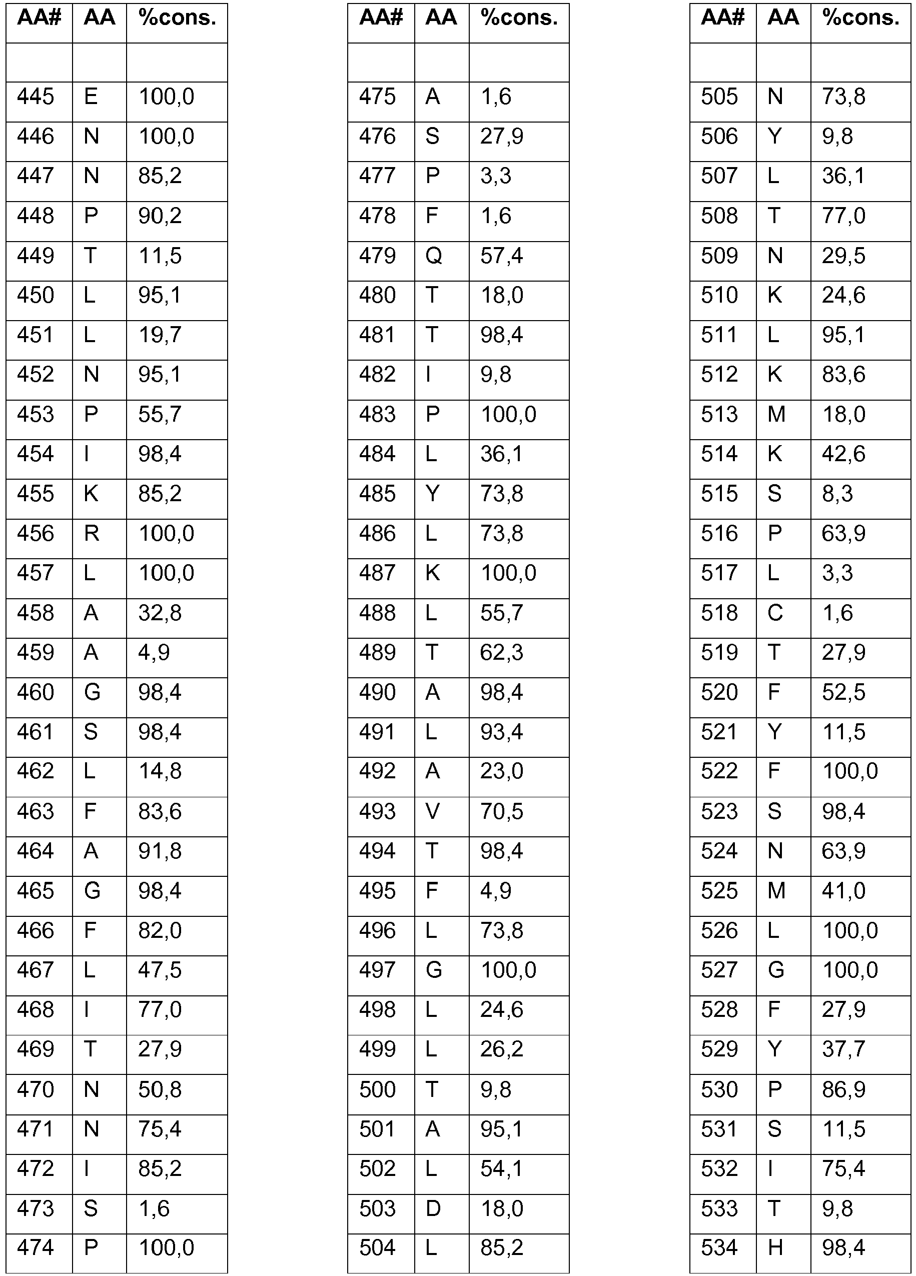

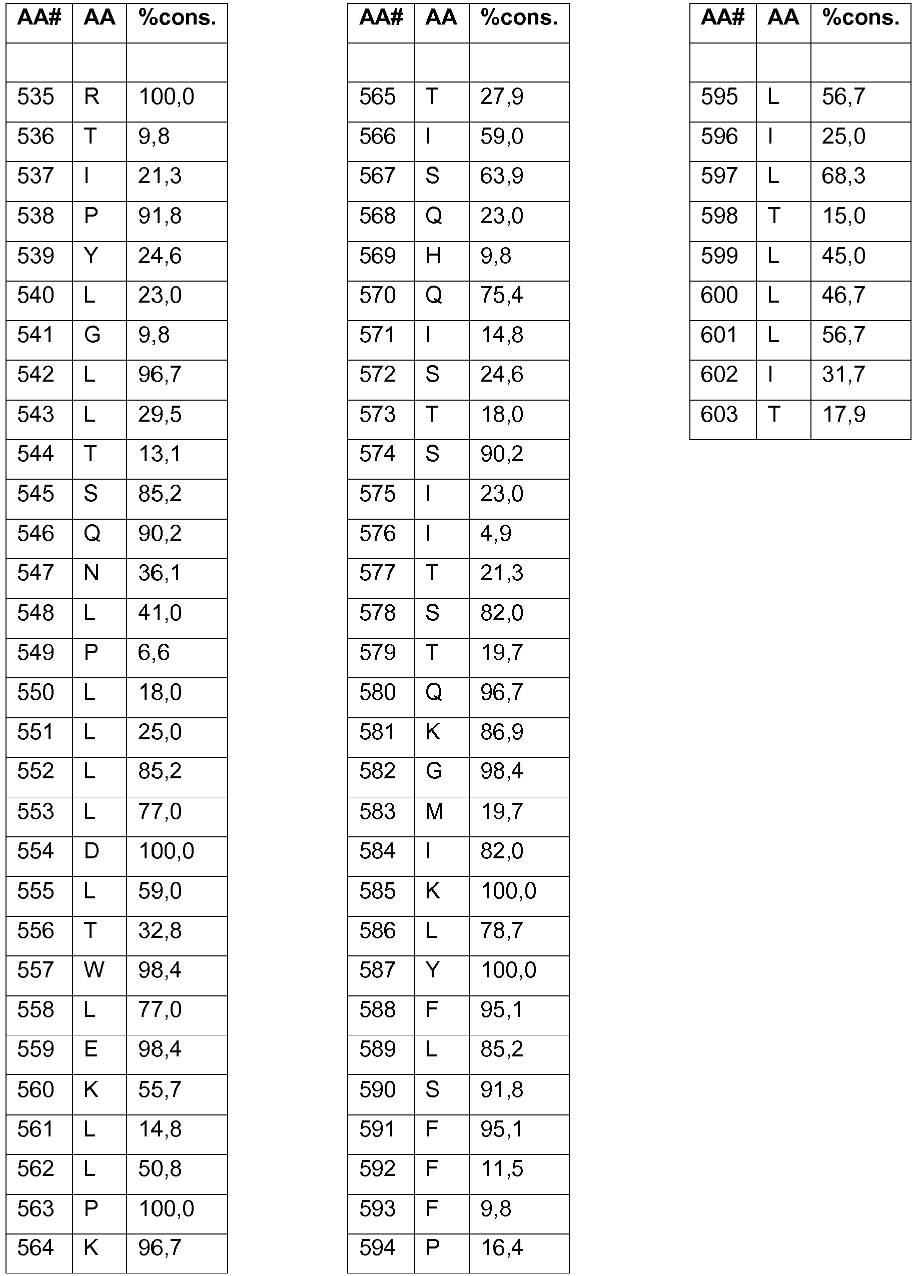

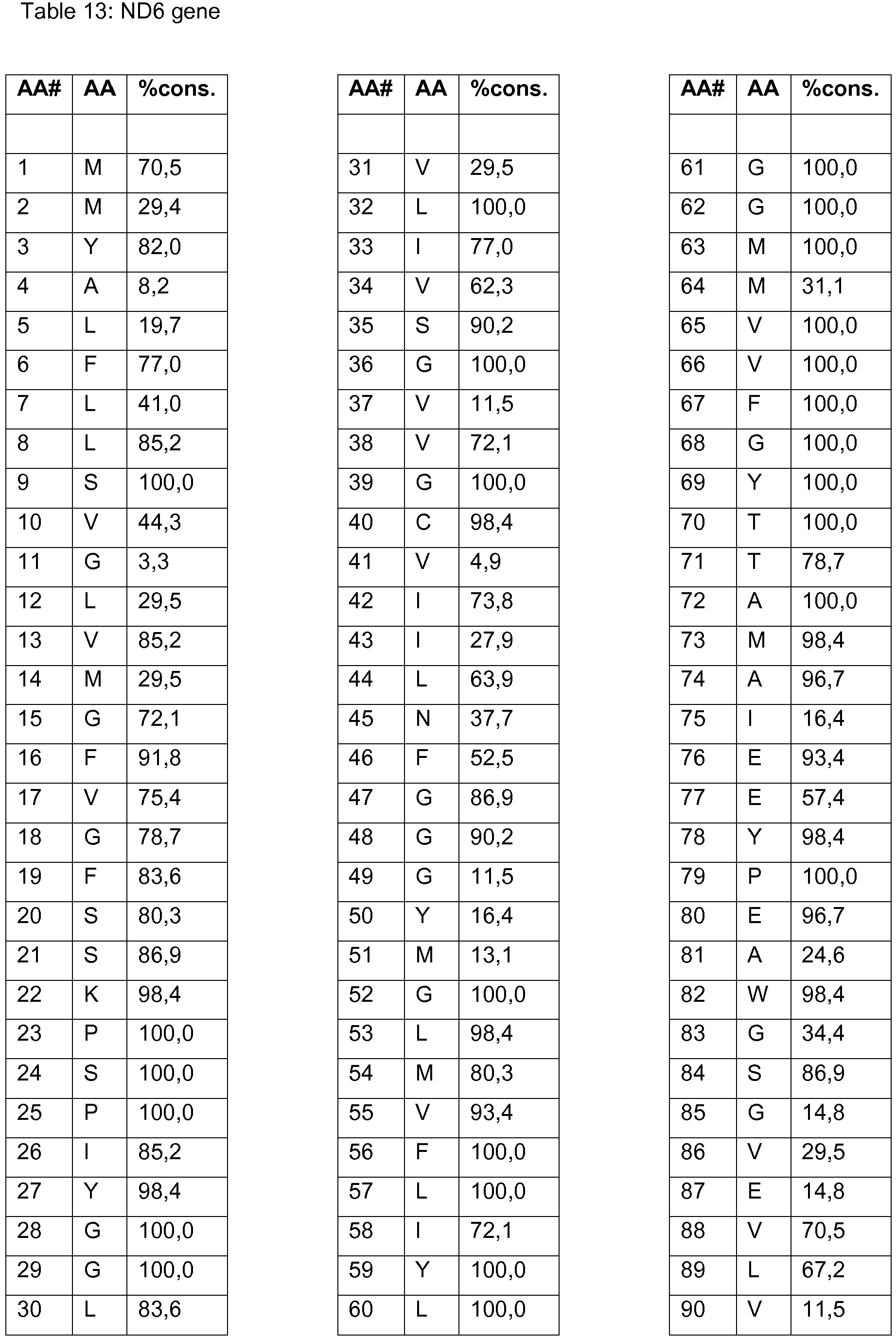

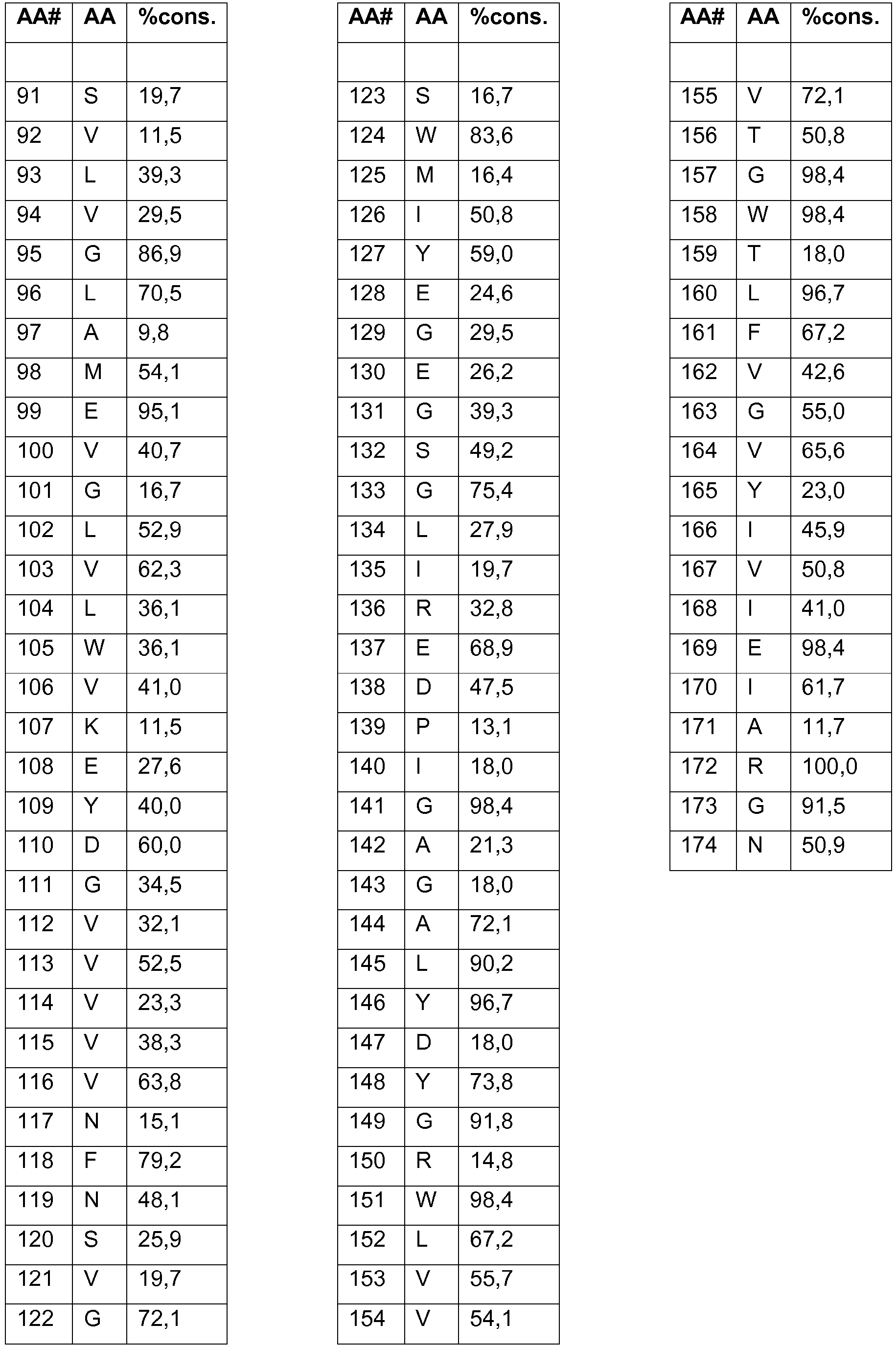

**Table 14; Determination of amino acid positions in a mitochondrial gene.**

| Gene Name | First Coding Nucleotide Position (FCNP) | Last Coding Nucleotide Position | Amino Acids | SEQ ID NO: |
|---|---|---|---|---|
| ND1 | 3307 | 4260 | 1 - 318 | 2 |
| ND2 | 4470 | 5510 | 1 - 347 | 3 |
| CO1 | 5904 | 7442 | 1 - 513 | 4 |
| CO2 | 7586 | 8266 | 1 - 227 | 5 |
| ATP8 | 8366 | 8569 | 1 - 68 | 6 |
| ATP6 | 8527 | 9204 | 1 - 226 | 7 |
| CO3 | 9207 | 9989 | 1 - 261 | 8 |
| ND3 | 10059 | 10403 | 1 - 115 | 9 |
| ND4L | 10470 | 10763 | 1 - 98 | 10 |
| ND4 | 10760 | 12136 | 1 - 459 | 11 |
| ND5 | 12337 | 14145 | 1 - 603 | 12 |
| ND6 | 14673 | 14152 | 1 - 174 | 13 |
| CytB | 14747 | 15886 | 1 - 380 | 14 |

**Table 15 Nucleotide positions of loops of tRNAs**

| **tRNA** | **D-loop** | **Anti-CD loop** | **T-loop** |
|---|---|---|---|
| Phe | 590-598 | 609-615 | 628-636 |
| Val | 1615-1620 | 1631-1637 | 1652-1657 |
| Ile | 4275-4280 | 4290-4296 | 4312-4318 |
| Met | 4415-4419 | 4430-4436 | 4451-4456 |
| Trp | 5525-5531 | 5542-5548 | 5561-5568 |
| Asp | 7531-7535 | 7546-7552 | 7567-7572 |
| Lys | 8307-8311 | 8321-8327 | 8343-8351 |
| Gly | 10004-10008 | 10019-10025 | 10039-10046 |
| Arg | 10418-10422 | 10433-10439 | 10453-10457 |
| His | 12151-12155 | 12166-12172 | 12187-12193 |
| Ser (AGY) | 12214-12217 | 12224-12230 | 12245-12252 |
| Thr | 15900-15907 | 15917-15932 | 15937-15941 |
| Pro | 16010-16005 | 15994-15998 | 15974-15968 |
| Glu | 14729-14725 | 14714-14708 | 14693-14687 |
| Tyr | 5879-5874 | 5864-5858 | 5843-5839 |
| Cys | 5814-5810 | 5800-5794 | 5780-5773 |
| Asn | 5717-5708 | 5698-5692 | 5676-5670 |
| Ala | 5642-5638 | 5627-5621 | 5606-5600 |
| Leu (UUR) | 3243-3252 | 32633269 | 3285-3291 |
| Leu (CUN) | 12279-12285 | 12296-12302 | 12317-12323 |
| Ser (UCN) | 7502-7498 | 7486-7480 | 7465-7459 |
| Gln | 43874380 | 4369-4363 | 4384-4342 |

**Table 16: Raw data from 321 patients**

| **Patient #** | **# non-synonymous variants** | **# non-synonymous variants in genes <=90% conserved** | **# variants tRNA genes** | **Baseline dyspnea score** | **RILT** |
|---|---|---|---|---|---|
| 1 | 3 | 3 | 0 | 2 | YES |
| 2 | 3 | 2 | 0 | 0 | NO |
| 3 | 2 | 2 | 0 | 0 | NO |
| 4 | 0 | 0 | 0 | 3 | YES |
| 5 | 3 | 3 | 0 | 1 | NO |
| 6 | 8 | 7 | 0 | 1 | YES |
| 7 | 6 | 5 | 0 | 0 | NO |
| 8 | 3 | 2 | 0 | 1 | NO |
| 9 | 9 | 8 | 0 | 1 | NO |
| 10 | 2 | 2 | 0 | 1 | YES |
| 11 | 4 | 4 | 0 | 0 | NO |
| 12 | 2 | 2 | 0 | 3 | YES |
| 13 | 4 | 3 | 1 | 0 | NO |
| 14 | 8 | 5 | 1 | 3 | YES |
| 15 | 4 | 4 | 0 | 1 | NO |
| 16 | 8 | 7 | 0 | 0 | NO |
| 17 | 6 | 5 | 1 | 0 | NO |
| 18 | 3 | 3 | 0 | 0 | NO |
| 19 | 6 | 6 | 1 | 2 | YES |
| 20 | 2 | 2 | 0 | 2 | NO |
| 21 | 2 | 2 | 0 | 1 | NO |
| 22 | 10 | 9 | 1 | 0 | NO |
| 23 | 7 | 6 | 1 | 0 | NO |
| 24 | 3 | 2 | 0 | 0 | NO |
| 25 | 4 | 4 | 1 | 1 | YES |
| 26 | 5 | 5 | 1 | 1 | NO |
| 27 | 10 | 10 | 0 | 0 | NO |
| 28 | 7 | 7 | 1 | 1 | YES |
| 29 | 2 | 2 | 0 | 1 | NO |
| 30 | 9 | 8 | 1 | 0 | NO |
| 31 | 6 | 5 | 0 | 1 | NO |
| 32 | 2 | 2 | 0 | 0 | NO |
| 33 | 2 | 2 | 0 | 0 | NO |
| 34 | 2 | 2 | 0 | 1 | NO |
| 35 | 6 | 5 | 1 | 1 | NO |
| 36 | 2 | 2 | 0 | 1 | NO |
| 37 | 2 | 2 | 0 | 1 | YES |
| 38 | 4 | 4 | 0 | 0 | NO |
| 39 | 4 | 3 | 0 | 1 | YES |
| 40 | 10 | 9 | 1 | 2 | YES |
| 41 | 8 | 8 | 0 | 1 | YES |
| 42 | 7 | 7 | 0 | 1 | NO |
| 43 | 3 | 3 | 0 | 1 | NO |
| 44 | 2 | 2 | 0 | 1 | NO |
| 45 | 2 | 2 | 0 | 1 | NO |
| 46 | 6 | 6 | 2 | 0 | NO |
| 47 | 8 | 7 | 0 | 0 | NO |
| 48 | 2 | 2 | 0 | 0 | NO |
| 49 | 7 | 7 | 0 | 0 | NO |
| 50 | 9 | 8 | 1 | 0 | NO |
| 51 | 2 | 2 | 0 | 0 | NO |
| 52 | 8 | 8 | 0 | 2 | YES |
| 53 | 6 | 6 | 0 | 1 | NO |
| 54 | 3 | 3 | 0 | 1 | NO |
| 55 | 4 | 4 | 1 | 1 | NO |
| 56 | 9 | 9 | 0 | 0 | NO |
| 57 | 2 | 2 | 0 | 1 | NO |
| 58 | 7 | 6 | 0 | 1 | NO |
| 59 | 3 | 2 | 0 | 1 | NO |
| 60 | 3 | 3 | 0 | 0 | NO |
| 61 | 6 | 5 | 1 | 1 | NO |
| 62 | 7 | 6 | 1 | 0 | NO |
| 63 | 11 | 11 | 0 | 1 | NO |
| 64 | 4 | 4 | 1 | 0 | NO |
| 65 | 10 | 10 | 1 | 1 | YES |
| 66 | 3 | 3 | 0 | 0 | NO |
| 67 | 5 | 5 | 1 | 2 | YES |
| 68 | 6 | 5 | 0 | 1 | YES |
| 69 | 3 | 2 | 0 | 1 | YES |
| 70 | 2 | 2 | 1 | 0 | NO |
| 71 | 3 | 3 | 0 | 1 | NO |
| 72 | 9 | 9 | 0 | 1 | NO |
| 73 | 2 | 2 | 0 | 0 | NO |
| 74 | 3 | 2 | 0 | 0 | YES |
| 75 | 6 | 5 | 1 | 0 | NO |
| 76 | 6 | 5 | 1 | 1 | YES |
| 77 | 2 | 2 | 0 | 0 | NO |
| 78 | 7 | 6 | 0 | 1 | NO |
| 79 | 3 | 3 | 0 | 0 | NO |
| 80 | 3 | 2 | 0 | 0 | NO |
| 81 | 11 | 10 | 0 | 0 | YES |
| 82 | 3 | 3 | 0 | 1 | NO |
| 83 | 6 | 5 | 0 | 0 | NO |
| 84 | 3 | 3 | 0 | 0 | NO |
| 85 | 6 | 6 | 0 | 1 | NO |
| 86 | 2 | 2 | 0 | 1 | NO |
| 87 | 10 | 10 | 0 | 1 | NO |
| 88 | 7 | 7 | 1 | 2 | YES |
| 89 | 10 | 11 | 0 | 3 | YES |
| 90 | 2 | 2 | 0 | 1 | NO |
| 91 | 6 | 6 | 0 | 3 | YES |
| 92 | 6 | 5 | 0 | 1 | YES |
| 93 | 3 | 4 | 1 | 0 | NO |
| 94 | 2 | 2 | 0 | 0 | NO |
| 95 | 2 | 2 | 0 | 1 | NO |
| 96 | 14 | 13 | 0 | 0 | YES |
| 97 | 3 | 3 | 0 | 1 | NO |
| 98 | 3 | 3 | 0 | 0 | NO |
| 99 | 4 | 3 | 0 | 0 | NO |
| 100 | 8 | 8 | 1 | 0 | NO |
| 101 | 9 | 9 | 1 | 1 | NO |
| 102 | 4 | 3 | 0 | 0 | NO |
| 103 | 2 | 2 | 0 | 0 | NO |
| 104 | 3 | 3 | 0 | 0 | NO |
| 105 | 7 | 6 | 2 | 0 | NO |
| 106 | 2 | 2 | 0 | 2 | NO |
| 107 | 2 | 2 | 0 | 0 | NO |
| 108 | 3 | 2 | 0 | 0 | NO |
| 109 | 2 | 2 | 1 | 1 | YES |
| 110 | 3 | 3 | 0 | 1 | NO |
| 111 | 10 | 9 | 1 | 0 | NO |
| 112 | 6 | 6 | 0 | 0 | NO |
| 113 | 3 | 4 | 0 | 0 | YES |
| 114 | 8 | 7 | 0 | 0 | NO |
| 115 | 2 | 2 | 0 | 2 | YES |
| 116 | 2 | 2 | 0 | 1 | NO |
| 117 | 6 | 5 | 0 | 0 | NO |
| 118 | 2 | 2 | 0 | 1 | YES |
| 119 | 2 | 2 | 1 | 1 | YES |
| 120 | 6 | 5 | 1 | 1 | NO |
| 121 | 6 | 5 | 1 | 1 | YES |
| 122 | 6 | 5 | 1 | 2 | NO |
| 123 | 2 | 2 | 0 | 1 | YES |
| 124 | 4 | 3 | 0 | 1 | NO |
| 125 | 4 | 4 | 0 | 1 | NO |
| 126 | 3 | 3 | 1 | 1 | YES |
| 127 | 7 | 6 | 1 | 0 | NO |
| 128 | 3 | 2 | 0 | 1 | NO |
| 129 | 9 | 6 | 1 | NA | NO |
| 130 | 2 | 2 | 0 | 0 | NO |
| 131 | 10 | 10 | 0 | 0 | NO |
| 132 | 3 | 3 | 0 | 0 | NO |
| 133 | 5 | 4 | 2 | 0 | NO |
| 134 | 2 | 2 | 0 | 0 | NO |
| 135 | 2 | 2 | 0 | 0 | NO |
| 136 | 3 | 3 | 0 | 0 | NO |
| 137 | 3 | 3 | 0 | 1 | NO |
| 138 | 7 | 6 | 1 | 0 | NO |
| 139 | 3 | 2 | 0 | 0 | NO |
| 140 | 8 | 7 | 0 | 1 | NO |
| 141 | 6 | 5 | 0 | 2 | YES |
| 142 | 2 | 2 | 1 | 0 | NO |
| 143 | 6 | 5 | 1 | 3 | NO |
| 144 | 5 | 3 | 2 | 2 | NO |
| 145 | 8 | 7 | 0 | 0 | NO |
| 146 | 3 | 3 | 0 | 1 | NO |
| 147 | 4 | 4 | 1 | 0 | NO |
| 148 | 2 | 2 | 0 | NA | NO |
| 149 | 6 | 5 | 0 | 0 | NO |
| 150 | 2 | 2 | 0 | 0 | NO |
| 151 | 8 | 8 | 0 | 0 | NO |
| 152 | 3 | 2 | 0 | 2 | NO |
| 153 | 2 | 2 | 0 | 1 | NO |
| 154 | 2 | 2 | 0 | 1 | NO |
| 155 | 5 | 5 | 1 | 1 | NO |
| 156 | 14 | 13 | 0 | 1 | NO |
| 157 | 7 | 6 | 1 | 0 | NO |
| 158 | 9 | 7 | 1 | 1 | NO |
| 159 | 3 | 3 | 0 | 0 | NO |
| 160 | 3 | 2 | 0 | 0 | NO |
| 161 | 2 | 2 | 0 | 0 | NO |
| 162 | 2 | 2 | 1 | 1 | NO |
| 163 | 3 | 2 | 0 | 0 | NO |
| 164 | 6 | 5 | 0 | 0 | YES |
| 165 | 6 | 5 | 1 | 1 | NO |
| 166 | 3 | 3 | 0 | 1 | NO |
| 167 | 6 | 5 | 0 | 1 | NO |
| 168 | 6 | 5 | 0 | 0 | NO |
| 169 | 6 | 5 | 1 | 1 | NO |
| 170 | 3 | 2 | 0 | 1 | NO |
| 171 | 7 | 6 | 0 | 0 | NO |
| 172 | 2 | 2 | 0 | 1 | NO |
| 173 | 7 | 7 | 0 | 2 | YES |
| 174 | 2 | 2 | 0 | 1 | NO |
| 175 | 8 | 7 | 1 | 0 | NO |
| 176 | 3 | 3 | 0 | 1 | NO |
| 177 | 9 | 8 | 1 | 2 | YES |
| 178 | 6 | 5 | 1 | 1 | YES |
| 179 | 3 | 2 | 0 | 1 | NO |
| 180 | 7 | 6 | 2 | 1 | YES |
| 181 | 2 | 2 | 0 | 2 | YES |
| 182 | 2 | 2 | 0 | 1 | NO |
| 183 | 3 | 3 | 0 | 0 | NO |
| 184 | 2 | 2 | 0 | 1 | NO |
| 185 | 2 | 2 | 1 | 1 | NO |
| 186 | 8 | 8 | 0 | 1 | NO |
| 187 | 2 | 2 | 0 | 0 | NO |
| 188 | 3 | 3 | 1 | 0 | YES |
| 189 | 6 | 5 | 0 | 2 | NO |
| 190 | 6 | 5 | 0 | 0 | NO |
| 191 | 2 | 2 | 0 | 1 | NO |
| 192 | 2 | 2 | 0 | 4 | YES |
| 193 | 2 | 2 | 0 | 1 | YES |
| 194 | 8 | 7 | 1 | 1 | YES |
| 195 | 2 | 2 | 0 | 0 | NO |
| 196 | 2 | 2 | 0 | 3 | NO |
| 197 | 3 | 2 | 0 | 1 | YES |
| 198 | 3 | 2 | 0 | 1 | NO |
| 199 | 4 | 4 | 1 | 3 | YES |
| 200 | 8 | 7 | 0 | 0 | YES |
| 201 | 11 | 8 | 0 | 0 | NO |
| 202 | 3 | 3 | 0 | 1 | NO |
| 203 | 7 | 6 | 1 | 0 | NO |
| 204 | 2 | 2 | 0 | NA | NO |
| 205 | 9 | 8 | 1 | 1 | YES |
| 206 | 4 | 4 | 1 | 0 | NO |
| 207 | 3 | 3 | 1 | 1 | YES |
| 208 | 9 | 8 | 1 | NA | NO |
| 209 | 6 | 5 | 1 | 1 | NO |
| 210 | 6 | 5 | 2 | 2 | YES |
| 211 | 10 | 10 | 0 | 1 | YES |
| 212 | 3 | 2 | 0 | 0 | NO |
| 213 | 2 | 2 | 0 | 1 | NO |
| 214 | 2 | 2 | 0 | 1 | NO |
| 215 | 2 | 2 | 0 | 0 | NO |
| 216 | 4 | 4 | 1 | 2 | YES |
| 217 | 4 | 4 | 1 | 0 | NO |
| 218 | 6 | 5 | 0 | 2 | NO |
| 219 | 3 | 2 | 0 | 1 | NO |
| 220 | 2 | 2 | 0 | 2 | NO |
| 221 | 5 | 4 | 1 | 2 | YES |
| 222 | 7 | 7 | 0 | 1 | YES |
| 223 | 8 | 5 | 1 | 1 | NO |
| 224 | 2 | 2 | 0 | 0 | NO |
| 225 | 3 | 3 | 0 | NA | NO |
| 226 | 9 | 9 | 0 | 1 | NO |
| 227 | 2 | 2 | 0 | 0 | NO |
| 228 | 2 | 2 | 1 | 0 | NO |
| 229 | 2 | 2 | 1 | 0 | NO |
| 230 | 7 | 7 | 1 | 3 | YES |
| 231 | 4 | 4 | 0 | 0 | NO |
| 232 | 6 | 5 | 1 | 1 | YES |
| 233 | 2 | 2 | 0 | 1 | NO |
| 234 | 2 | 2 | 0 | 1 | NO |
| 235 | 7 | 6 | 2 | 2 | YES |
| 236 | 2 | 2 | 0 | 0 | NO |
| 237 | 7 | 7 | 1 | 0 | YES |
| 238 | 7 | 6 | 0 | 1 | NO |
| 239 | 2 | 2 | 0 | 0 | NO |
| 240 | 3 | 3 | 0 | 0 | NO |
| 241 | 7 | 5 | 3 | 1 | NO |
| 242 | 2 | 2 | 0 | 0 | NO |
| 243 | 9 | 9 | 0 | 0 | NO |
| 244 | 3 | 2 | 0 | 1 | NO |
| 245 | 6 | 6 | 1 | 1 | YES |
| 246 | 2 | 2 | 0 | 1 | NO |
| 247 | 3 | 2 | 0 | 1 | NO |
| 248 | 3 | 3 | 0 | 0 | NO |
| 249 | 4 | 3 | 0 | 1 | NO |
| 250 | 6 | 4 | 1 | 1 | YES |
| 251 | 6 | 5 | 1 | 1 | YES |
| 252 | 6 | 5 | 0 | 2 | NO |
| 253 | 2 | 2 | 0 | 1 | NO |
| 254 | 9 | 9 | 0 | 3 | YES |
| 255 | 2 | 2 | 0 | 1 | NO |
| 256 | 0 | 0 | 0 | 1 | YES |
| 257 | 6 | 6 | 2 | 4 | YES |
| 258 | 11 | 8 | 0 | 1 | NO |
| 259 | 3 | 3 | 1 | 1 | NO |
| 260 | 4 | 3 | 1 | 1 | NO |
| 261 | 11 | 11 | 0 | 1 | NO |
| 262 | 6 | 5 | 0 | 0 | YES |
| 263 | 2 | 2 | 0 | 1 | YES |
| 264 | 5 | 4 | 2 | 1 | NO |
| 265 | 2 | 2 | 0 | 0 | NO |
| 266 | 7 | 7 | 1 | 0 | NO |
| 267 | 3 | 3 | 0 | 1 | NO |
| 268 | 7 | 6 | 2 | 2 | NO |
| 269 | 6 | 6 | 2 | 1 | NO |
| 270 | 6 | 6 | 2 | 1 | NO |
| 271 | 2 | 2 | 0 | 1 | YES |
| 272 | 2 | 2 | 0 | 1 | NO |
| 273 | 6 | 5 | 0 | 1 | YES |
| 274 | 2 | 2 | 0 | 0 | NO |
| 275 | 2 | 2 | 1 | 2 | NO |
| 276 | 2 | 2 | 0 | 1 | NO |
| 277 | 2 | 2 | 0 | 0 | NO |
| 278 | 5 | 4 | 1 | 1 | NO |
| 279 | 2 | 2 | 0 | 3 | YES |
| 280 | 2 | 2 | 1 | 1 | NO |
| 281 | 7 | 6 | 2 | 1 | NO |
| 282 | 3 | 3 | 0 | 1 | NO |
| 283 | 3 | 2 | 0 | 0 | NO |
| 284 | 2 | 2 | 0 | 0 | NO |
| 285 | 5 | 5 | 1 | 1 | NO |
| 286 | 5 | 5 | 1 | 1 | NO |
| 287 | 2 | 2 | 0 | 1 | NO |
| 288 | 7 | 6 | 1 | 1 | NO |
| 289 | 7 | 6 | 1 | 2 | YES |
| 290 | 8 | 7 | 1 | 0 | NO |
| 291 | 3 | 3 | 0 | 3 | YES |
| 292 | 2 | 2 | 0 | 1 | NO |
| 293 | 6 | 5 | 2 | 1 | YES |
| 294 | 6 | 5 | 1 | 2 | NO |
| 295 | 5 | 5 | 0 | 1 | NO |
| 296 | 3 | 3 | 0 | 0 | NO |
| 297 | 6 | 6 | 0 | 1 | NO |
| 298 | 3 | 3 | 0 | 0 | YES |
| 299 | 10 | 10 | 0 | 1 | NO |
| 300 | 6 | 6 | 0 | 1 | NO |
| 301 | 4 | 3 | 0 | 0 | NO |
| 302 | 3 | 3 | 0 | 0 | YES |
| 303 | 2 | 2 | 0 | 0 | NO |
| 304 | 8 | 8 | 0 | 2 | YES |
| 305 | 3 | 3 | 0 | 0 | NO |
| 306 | 2 | 2 | 0 | 0 | NO |
| 307 | 2 | 2 | 0 | 1 | NO |
| 308 | 10 | 10 | 0 | 0 | NO |
| 309 | 9 | 7 | 1 | 0 | NO |
| 310 | 7 | 5 | 1 | 0 | NO |
| 311 | 3 | 2 | 0 | 0 | NO |
| 312 | 8 | 6 | 1 | 0 | NO |
| 313 | 3 | 3 | 0 | 0 | NO |
| 314 | 2 | 2 | 0 | 1 | NO |
| 315 | 4 | 4 | 0 | 2 | YES |
| 316 | 5 | 5 | 1 | 0 | NO |
| 317 | 3 | 3 | 0 | 0 | NO |
| 318 | 6 | 5 | 1 | 1 | NO |
| 319 | 3 | 3 | 0 | 1 | NO |
| 320 | 4 | 3 | 1 | 0 | NO |
| 321 | 2 | 2 | 0 | 0 | NO |

### Examples

### Example 1: Patient population and DNA isolation.

Patient were recruited from MAASTRO Clinic (n=321) and Ghent University Hospital (n=66). Lung toxicity was scored using the Common Terminology Criteria for Adverse Events version 3.0 for dyspnea before (baseline) and up to six months after (maximum) radiotherapy (RT). The study was approved by the ethics committees of both centers, and all study participants provided written informed consent.

Total (nuclear and mitochondrial) cellular DNA was isolated from blood of patients collected before radiotherapy according to standard procedures.

Samples from patients with breast cancer were obtained from the University Medical Center of Mannheim. The skin biopsies were taken from patients treated in Mannheim as part of the TARGIT A phase 3 clinical trial [Vaidya et al., Lancet 382: 1 -11 (2013)].

The TARGIT A trial was performed between 02/2002 and 12/2008, 305 patients were treated within TARGIT A (Arm A: n = 34 IORT, n = 20 IORT + WBRT for risk factors; Arm B WBRT: n = 55) or received IORT as a planned boost (control group: n = 196). Toxicity was assessed according to the LENT SOMA scales.

Patients were treated with interoperative radiation therapy (20 Gy/50 kV X-rays; INTRABEAM [Carl Zeiss Surgical, Oberkochen, Germany]) to the tumor bed during breast-conserving surgery as a boost followed by whole-breast radiotherapy (WBRT, 46 Gy). They underwent a prospective, predefined follow-up (median, 25 months; range 18-44 months), including clinical examination and breast ultrasound at 6-months and mammographies at 1-year intervals. Toxicities were documented using the common toxicity criteria (CTC)/European Organization for Research and Treatment of Cancer and the LENT-SOMA score. Cosmesis was evaluated with a score from 1 to 4.

### Example 2: Sequence determination.

GeneChip® Mitochondria Resequencing 2.0 Arrays (Affymetrix, Santa Clara, CA, USA) were used to determine the mtDNA sequence of the DNA samples according to the manufacturer's protocol.

After generation of the cell intensity (CEL) files by GeneChip® Operating Software 1.4 (GCOS 1.4), raw sequence data was analyzed by the Sequence Pilot - module SeqC (JSI medical systems) with the standard parameters. The eventual mtDNA sequences were compared with the revised Cambridge reference sequence (SEQ ID NO: 1) to list all homoplasmic variants. The haplogroups were determined as described previously (Voets, A.M., et al. Large scale mtDNA sequencing reveals sequence and functional conservation as major determinants of homoplasmic mtDNA variant distribution. Mitochondrion 11, 964-972 (2011)).

### Example 3: Statistical analysis

Multivariate logistic regression analyses models were built with α = 0.05. The model performance for predicting outcome was evaluated by calculating the area under the curve of the receiver operating characteristic curve using a 10-fold cross-validation (out-of-sample) procedure. The maximum value of the AUC is 1.0, indicating a perfect discrimination, whereas 0.5 indicates a random chance to correctly discriminate outcome with the model. P values for AUCs being different from 0.5 were calculated using 500 bootstraps and a 1-sided Student's t-test. The final model output was represented by a nomogram (lasonos, A., Schrag, D., Raj, G.V. & Panageas, K.S. How to build and interpret a nomogram for cancer prognosis. J Clin Oncol 26, 1364-1370 (2008)).

The endpoint for the model was the occurrence of dyspnea≥2 within six months after RT.

### Example 4: Breast cancer patients

**Table 21**

| Patient # | Probability of radiation induced toxicity |
|---|---|
| 11 | 0,167 |
| 13 | 0,167 |
| 14 | 0,167 |
| 2 | 0,167 |
| 3 | 0,167 |
| 6 | 0,167 |
| 8 | 0,167 |
| 9 | 0,167 |
| 10 | 0,184 |
| 1 | 0,184 |
| 4 | 0,224 |
| 12 | 0,248 |
| 5 | 0,271 |
| 15 | 0,296 |
| 18 | 0,296 |
| 20 | 0,296 |
| 22 | 0,296 |
| 19 | 0,322 |
| 21 | 0,322 |
| 17 | 0,378 |
| 7 | 0,41 |

## Claims

1. In vitro method for predicting the risk of developing radiation induced toxicity comprising the steps of:
a. obtaining mitochondrial DNA from a sample of a subject,
b. determining the number of non-synonymous variations present in at least one gene encoding a mitochondrial protein,
c. determining whether the non-synonymous variations occur at positions that are less than 90% conserved,
d. attributing a value to the number of non-synonymous variations that occur at positions that are less than 90% conserved,
wherein a higher value corresponds to a higher risk for developing radiation induced toxicity.

2. In vitro method according to claim 1 wherein the subject is diagnosed with lung cancer.

3. In vitro method according to claim 1 wherein the subject is diagnosed with breast cancer.

4. In vitro method according to claim 2, additionally comprising a step of attributing a value to the baseline dyspnea score of the subject and adding that value to the value obtained from the number of non-synonymous variations to obtain an aggregated value, wherein a higher aggregated value corresponds to a higher risk of developing radiation induced toxicity, such as lung toxicity.

5. In vitro method according to any one of claims 1 - 4, additionally comprising a step of determining the number of variations in tRNA loop positions, attributing a value to the number of variations in tRNA loop positions and adding that value to the value obtained from the number of non-synonymous variations or the aggregated value.

6. In vitro method according to any one of claims 1 - 5 wherein a value is attributed to the dose of chemotherapy that the subject is receiving wherein the value is proportional to the dose of chemotherapy, and adding that value to the value obtained from the number of non-synonymous variations or the aggregated value.

7. In vitro method according to any one of claims 1 - 6 wherein said at least one gene encoding a mitochondrial protein is 2 genes or more than 2 genes, such as 3 or 4, 5, 6, 7, 8, 9, 10, 11, 12 or all genes encoding a mitochondrial protein.

8. In vitro method according to any one of claims 1 - 7 wherein the sample comprises nucleated cells.

9. In vitro method according to claim 8 wherein the sample is a blood sample or a tissue sample.

10. In vitro method according to any one of claims 1 - 9 wherein the sample is taken from the subject before the start of the radiation therapy.

## Patentansprüche

1. In-vitro-Verfahren zur Vorhersage des Risikos der Entwicklung strahlungsinduzierter Toxizität, umfassend die Schritte:
a. Gewinnen mitochondrialer DNA aus einer Probe von einem Individuum,
b. Bestimmen der Anzahl nicht synonymer Variationen, die in wenigstens einem für ein mitochondriales Protein codierenden Gen vorliegen,
c. Bestimmen, ob die nicht synonymen Variationen an Positionen auftreten, die zu weniger als 90% konserviert sind,
d. Zuordnen eines Werts zur Anzahl nicht synonymer Variationen, die an Positionen auftreten, die zu weniger als 90% konserviert sind,
wobei ein höherer Wert einem höheren Risiko der Entwicklung strahlungsinduzierter Toxizität entspricht.

2. In-vitro-Verfahren nach Anspruch 1, wobei bei dem Individuum eine Lungenkrebsdiagnose vorliegt.

3. In-vitro-Verfahren nach Anspruch 1, wobei bei dem Individuum eine Brustkrebsdiagnose vorliegt.

4. In-vitro-Verfahren nach Anspruch 2, zusätzlich umfassend einen Schritt, bei dem dem Dyspnoebasislinienergebnis des Individuums ein Wert zugeordnet und dieser Wert zu dem aus der Anzahl nicht synonymer Variationen erhaltenen Wert addiert wird, so dass ein Gesamtwert erhalten wird, wobei ein höherer Gesamtwert einem höheren Risiko der Entwicklung strahlungsinduzierter Toxizität, wie etwa Lungentoxizität, entspricht.

5. In-vitro-Verfahren nach einem der Ansprüche 1 - 4, zusätzlich umfassend einen Schritt, bei dem die Anzahl von Variationen in tRNA-Loop-Positionen bestimmt, der Anzahl von Variationen in tRNA-Loop-Positionen ein Wert zugeordnet und dieser Wert zu dem aus der Anzahl nicht synonymer Variationen erhaltenen Wert addiert wird oder zu dem Gesamtwert addiert wird.

6. In-vitro-Verfahren nach einem der Ansprüche 1 - 5, wobei der Chemotherapiedosis, die das Individuum erhält, ein Wert zugeordnet wird, wobei der Wert zur Chemotherapiedosis proportional ist, und dieser Wert zu dem aus der Anzahl nicht synonymer Variationen erhaltenen Wert addiert wird oder zu dem Gesamtwert addiert wird.

7. In-vitro-Verfahren nach einem der Ansprüche 1 - 6, wobei es sich bei dem wenigstens einen für ein mitochondriales Protein codierenden Gen um 2 Gene oder mehr als 2 Gene, wie etwa 3 oder 4, 5, 6, 7, 8, 9, 10, 11, 12 oder alle Gene, die für ein mitochondriales Protein codieren, handelt.

8. In-vitro-Verfahren nach einem der Ansprüche 1 - 7, wobei die Probe kernhaltige Zellen umfasst.

9. In-vitro-Verfahren nach Anspruch 8, wobei es sich bei der Probe um eine Blutprobe oder eine Gewebeprobe handelt.

10. In-vitro-Verfahren nach einem der Ansprüche 1 - 9, wobei die Probe dem Individuum vor dem Beginn der Strahlentherapie entnommen wird.

## Revendications

1. Procédé *in vitro* de prédiction du risque de développer une toxicité induite par rayonnement comprenant les étapes de :
a. obtention d'ADN mitochondrial à partir d'un échantillon d'un sujet,
b. détermination du nombre de variations non synonymes présentes dans au moins un gène codant pour une protéine mitochondriale,
c. détermination que les variations non synonymes sont présentes ou non à des positions qui sont conservées à moins de 90 %,
d. attribution d'une valeur au nombre de variations non synonymes qui sont présentes aux positions qui sont conservées à moins de 90 %,
dans lequel une valeur plus élevée correspond à un risque plus élevé de développer une toxicité induite par rayonnement.

2. Procédé *in vitro* selon la revendication 1 dans lequel le sujet est diagnostiqué avec un cancer du poumon.

3. Procédé *in vitro* selon la revendication 1 dans lequel le sujet est diagnostiqué avec un cancer du sein.

4. Procédé *in vitro* selon la revendication 2, comprenant en outre une étape d'attribution d'une valeur au score de dyspnée de référence du sujet et ajout de cette valeur à la valeur obtenue à partir du nombre de variations non synonymes pour obtenir une valeur agrégée, dans lequel une valeur agrégée plus élevée correspond à un risque plus élevé de développer une toxicité induite par rayonnement, telle qu'une toxicité pulmonaire.

5. Procédé *in vitro* selon l'une quelconque des revendications 1 à 4, comprenant en outre une étape de détermination du nombre de variations dans les positions de boucle d'ARNt, attribution d'une valeur au nombre de variations dans les positions de boucle d'ARNt et ajout de cette valeur à la valeur obtenue à partir du nombre de variations non synonymes ou la valeur agrégée.

6. Procédé *in vitro* selon l'une quelconque des revendications 1 à 5 dans lequel une valeur est attribuée à la dose de chimiothérapie que le sujet reçoit, la valeur étant proportionnelle à la dose de chimiothérapie, et l'ajout de cette valeur à la valeur obtenue à partir du nombre de variations non synonymes ou la valeur agrégée.

7. Procédé *in vitro* selon l'une quelconque des revendications 1 à 6 dans lequel ledit au moins un gène codant pour une protéine mitochondriale est 2 gènes ou plus de 2 gènes, tels que 3 ou 4, 5, 6, 7, 8, 9, 10, 11, 12 ou tous les gènes codant pour une protéine mitochondriale.

8. Procédé *in vitro* selon l'une quelconque des revendications 1 à 7 dans lequel l'échantillon comprend des cellules nucléées.

9. Procédé *in vitro* selon la revendication 8 dans lequel l'échantillon est un échantillon de sang ou un échantillon de tissu.

10. Procédé *in vitro* selon l'une quelconque des revendications 1 à 9 dans lequel l'échantillon est prélevé à partir du sujet avant le début de la radiothérapie.


### SEQUENCE LISTING

<110> Universiteit Maastricht, Academisch Ziekenhuis Maastricht and Stichting Maastricht Radiation Oncology "Maastro-Clinic"
<120> Method for determining the risk of developing radiation-induced toxicity after exposure to radiation.
<130> 229 WO
<160> 14
<170> PatentIn version 3.5
<210> 1
   <211> 16569
   <212> DNA
   <213> homo sapiens
<220>
   <221> b
   <222> (3107)..(3107)
   <223> b is a gap in order to keep this sequence aligned with previous reference sequences.
<400> 1
   gatcacaggt ctatcaccct attaaccact cacgggagct ctccatgcat ttggtatttt      60
   cgtctggggg gtatgcacgc gatagcattg cgagacgctg gagccggagc accctatgtc     120
   gcagtatctg tctttgattc ctgcctcatc ctattattta tcgcacctac gttcaatatt     180
   acaggcgaac atacttacta aagtgtgtta attaattaat gcttgtagga cataataata     240
   acaattgaat gtctgcacag ccactttcca cacagacatc ataacaaaaa atttccacca     300
   aaccccccct cccccgcttc tggccacagc acttaaacac atctctgcca aaccccaaaa    360
   acaaagaacc ctaacaccag cctaaccaga tttcaaattt tatcttttgg cggtatgcac     420
   ttttaacagt caccccccaa ctaacacatt attttcccct cccactccca tactactaat     480
   ctcatcaata caacccccgc ccatcctacc cagcacacac acaccgctgc taaccccata     540
   ccccgaacca accaaacccc aaagacaccc cccacagttt atgtagctta cctcctcaaa     600
   gcaatacact gaaaatgttt agacgggctc acatcacccc ataaacaaat aggtttggtc     660
   ctagcctttc tattagctct tagtaagatt acacatgcaa gcatccccgt tccagtgagt     720
   tcaccctcta aatcaccacg atcaaaagga acaagcatca agcacgcagc aatgcagctc     780
   aaaacgctta gcctagccac acccccacgg gaaacagcag tgattaacct ttagcaataa     840
   acgaaagttt aactaagcta tactaacccc agggttggtc aatttcgtgc cagccaccgc     900
   ggtcacacga ttaacccaag tcaatagaag ccggcgtaaa gagtgtttta gatcaccccc     960
   tccccaataa agctaaaact cacctgagtt gtaaaaaact ccagttgaca caaaatagac    1020
   tacgaaagtg gctttaacat atctgaacac acaatagcta agacccaaac tgggattaga    1080
   taccccacta tgcttagccc taaacctcaa cagttaaatc aacaaaactg ctcgccagaa 1140
   cactacgagc cacagcttaa aactcaaagg acctggcggt gcttcatatc cctctagagg 1200
   agcctgttct gtaatcgata aaccccgatc aacctcacca cctcttgctc agcctatata 1260
   ccgccatctt cagcaaaccc tgatgaaggc tacaaagtaa gcgcaagtac ccacgtaaag 1320
   acgttaggtc aaggtgtagc ccatgaggtg gcaagaaatg ggctacattt tctaccccag 1380
   aaaactacga tagcccttat gaaacttaag ggtcgaaggt ggatttagca gtaaactaag 1440
   agtagagtgc ttagttgaac agggccctga agcgcgtaca caccgcccgt caccctcctc 1500
   aagtatactt caaaggacat ttaactaaaa cccctacgca tttatataga ggagacaagt 1560
   cgtaacatgg taagtgtact ggaaagtgca cttggacgaa ccagagtgta gcttaacaca 1620
   aagcacccaa cttacactta ggagatttca acttaacttg accgctctga gctaaaccta 1680
   gccccaaacc cactccacct tactaccaga caaccttagc caaaccattt acccaaataa 1740
   agtataggcg atagaaattg aaacctggcg caatagatat agtaccgcaa gggaaagatg 1800
   aaaaattata accaagcata atatagcaag gactaacccc tataccttct gcataatgaa 1860
   ttaactagaa ataactttgc aaggagagcc aaagctaaga cccccgaaac cagacgagct 1920
   acctaagaac agctaaaaga gcacacccgt ctatgtagca aaatagtggg aagatttata 1980
   ggtagaggcg acaaacctac cgagcctggt gatagctggt tgtccaagat agaatcttag 2040
   ttcaacttta aatttgccca cagaaccctc taaatcccct tgtaaattta actgttagtc 2100
   caaagaggaa cagctctttg gacactagga aaaaaccttg tagagagagt aaaaaattta 2160
   acacccatag taggcctaaa agcagccacc aattaagaaa gcgttcaagc tcaacaccca 2220
   ctacctaaaa aatcccaaac atataactga actcctcaca cccaattgga ccaatctatc 2280
   accctataga agaactaatg ttagtataag taacatgaaa acattctcct ccgcataagc 2340
   ctgcgtcaga ttaaaacact gaactgacaa ttaacagccc aatatctaca atcaaccaac 2400
   aagtcattat taccctcact gtcaacccaa cacaggcatg ctcataagga aaggttaaaa 2460
   aaagtaaaag gaactcggca aatcttaccc cgcctgttta ccaaaaacat cacctctagc 2520
   atcaccagta ttagaggcac cgcctgccca gtgacacatg tttaacggcc gcggtaccct 2580
   aaccgtgcaa aggtagcata atcacttgtt ccttaaatag ggacctgtat gaatggctcc 2640
   acgagggttc agctgtctct tacttttaac cagtgaaatt gacctgcccg tgaagaggcg 2700
   ggcataacac agcaagacga gaagacccta tggagcttta atttattaat gcaaacagta 2760
   cctaacaaac ccacaggtcc taaactacca aacctgcatt aaaaatttcg gttggggcga 2820
   cctcggagca gaacccaacc tccgagcagt acatgctaag acttcaccag tcaaagcgaa 2880
   ctactatact caattgatcc aataacttga ccaacggaac aagttaccct agggataaca 2940
   gcgcaatcct attctagagt ccatatcaac aatagggttt acgacctcga tgttggatca 3000
   ggacatcccg atggtgcagc cgctattaaa ggttcgtttg ttcaacgatt aaagtcctac 3060
   gtgatctgag ttcagaccgg agtaatccag gtcggtttct atctacbttc aaattcctcc 3120
   ctgtacgaaa ggacaagaga aataaggcct acttcacaaa gcgccttccc ccgtaaatga 3180
   tatcatctca acttagtatt atacccacac ccacccaaga acagggtttg ttaagatggc 3240
   agagcccggt aatcgcataa aacttaaaac tttacagtca gaggttcaat tcctcttctt 3300
   aacaacatac ccatggccaa cctcctactc ctcattgtac ccattctaat cgcaatggca 3360
   ttcctaatgc ttaccgaacg aaaaattcta ggctatatac aactacgcaa aggccccaac 3420
   gttgtaggcc cctacgggct actacaaccc ttcgctgacg ccataaaact cttcaccaaa 3480
   gagcccctaa aacccgccac atctaccatc accctctaca tcaccgcccc gaccttagct 3540
   ctcaccatcg ctcttctact atgaaccccc ctccccatac ccaaccccct ggtcaacctc 3600
   aacctaggcc tcctatttat tctagccacc tctagcctag ccgtttactc aatcctctga 3660
   tcagggtgag catcaaactc aaactacgcc ctgatcggcg cactgcgagc agtagcccaa 3720
   acaatctcat atgaagtcac cctagccatc attctactat caacattact aataagtggc 3780
   tcctttaacc tctccaccct tatcacaaca caagaacacc tctgattact cctgccatca 3840
   tgacccttgg ccataatatg atttatctcc acactagcag agaccaaccg aacccccttc 3900
   gaccttgccg aaggggagtc cgaactagtc tcaggcttca acatcgaata cgccgcaggc 3960
   cccttcgccc tattcttcat agccgaatac acaaacatta ttataataaa caccctcacc 4020
   actacaatct tcctaggaac aacatatgac gcactctccc ctgaactcta cacaacatat 4080
   tttgtcacca agaccctact tctaacctcc ctgttcttat gaattcgaac agcatacccc 4140
   cgattccgct acgaccaact catacacctc ctatgaaaaa acttcctacc actcacccta 4200
   gcattactta tatgatatgt ctccataccc attacaatct ccagcattcc ccctcaaacc 4260
   taagaaatat gtctgataaa agagttactt tgatagagta aataatagga gcttaaaccc 4320
   ccttatttct aggactatga gaatcgaacc catccctgag aatccaaaat tctccgtgcc 4380
   acctatcaca ccccatccta aagtaaggtc agctaaataa gctatcgggc ccataccccg 4440
   aaaatgttgg ttataccctt cccgtactaa ttaatcccct ggcccaaccc gtcatctact 4500
   ctaccatctt tgcaggcaca ctcatcacag cgctaagctc gcactgattt tttacctgag 4560
   taggcctaga aataaacatg ctagctttta ttccagttct aaccaaaaaa ataaaccctc 4620
   gttccacaga agctgccatc aagtatttcc tcacgcaagc aaccgcatcc ataatccttc 4680
   taatagctat cctcttcaac aatatactct ccggacaatg aaccataacc aatactacca 4740
   atcaatactc atcattaata atcataatag ctatagcaat aaaactagga atagccccct 4800
   ttcacttctg agtcccagag gttacccaag gcacccctct gacatccggc ctgcttcttc 4860
   tcacatgaca aaaactagcc cccatctcaa tcatatacca aatctctccc tcactaaacg 4920
   taagccttct cctcactctc tcaatcttat ccatcatagc aggcagttga ggtggattaa 4980
   accaaaccca gctacgcaaa atcttagcat actcctcaat tacccacata ggatgaataa 5040
   tagcagttct accgtacaac cctaacataa ccattcttaa tttaactatt tatattatcc 5100
   taactactac cgcattccta ctactcaact taaactccag caccacgacc ctactactat 5160
   ctcgcacctg aaacaagcta acatgactaa cacccttaat tccatccacc ctcctctccc 5220
   taggaggcct gcccccgcta accggctttt tgcccaaatg ggccattatc gaagaattca 5280
   caaaaaacaa tagcctcatc atccccacca tcatagccac catcaccctc cttaacctct 5340
   acttctacct acgcctaatc tactccacct caatcacact actccccata tctaacaacg 5400
   taaaaataaa atgacagttt gaacatacaa aacccacccc attcctcccc acactcatcg 5460
   cccttaccac gctactccta cctatctccc cttttatact aataatctta tagaaattta 5520
   ggttaaatac agaccaagag ccttcaaagc cctcagtaag ttgcaatact taatttctgt 5580
   aacagctaag gactgcaaaa ccccactctg catcaactga acgcaaatca gccactttaa 5640
   ttaagctaag cccttactag accaatggga cttaaaccca caaacactta gttaacagct 5700
   aagcacccta atcaactggc ttcaatctac ttctcccgcc gccgggaaaa aaggcgggag 5760
   aagccccggc aggtttgaag ctgcttcttc gaatttgcaa ttcaatatga aaatcacctc 5820
   ggagctggta aaaagaggcc taacccctgt ctttagattt acagtccaat gcttcactca 5880
   gccattttac ctcaccccca ctgatgttcg ccgaccgttg actattctct acaaaccaca 5940
   aagacattgg aacactatac ctattattcg gcgcatgagc tggagtccta ggcacagctc 6000
   taagcctcct tattcgagcc gagctgggcc agccaggcaa ccttctaggt aacgaccaca 6060
   tctacaacgt tatcgtcaca gcccatgcat ttgtaataat cttcttcata gtaataccca 6120
   tcataatcgg aggctttggc aactgactag ttcccctaat aatcggtgcc cccgatatgg 6180
   cgtttccccg cataaacaac ataagcttct gactcttacc tccctctctc ctactcctgc 6240
   tcgcatctgc tatagtggag gccggagcag gaacaggttg aacagtctac cctcccttag 6300
   cagggaacta ctcccaccct ggagcctccg tagacctaac catcttctcc ttacacctag 6360
   caggtgtctc ctctatctta ggggccatca atttcatcac aacaattatc aatataaaac 6420
   cccctgccat aacccaatac caaacgcccc tcttcgtctg atccgtccta atcacagcag 6480
   tcctacttct cctatctctc ccagtcctag ctgctggcat cactatacta ctaacagacc 6540
   gcaacctcaa caccaccttc ttcgaccccg ccggaggagg agaccccatt ctataccaac 6600
   acctattctg atttttcggt caccctgaag tttatattct tatcctacca ggcttcggaa 6660
   taatctccca tattgtaact tactactccg gaaaaaaaga accatttgga tacataggta 6720
   tggtctgagc tatgatatca attggcttcc tagggtttat cgtgtgagca caccatatat 6780
   ttacagtagg aatagacgta gacacacgag catatttcac ctccgctacc ataatcatcg 6840
   ctatccccac cggcgtcaaa gtatttagct gactcgccac actccacgga agcaatatga 6900
   aatgatctgc tgcagtgctc tgagccctag gattcatctt tcttttcacc gtaggtggcc 6960
   tgactggcat tgtattagca aactcatcac tagacatcgt actacacgac acgtactacg 7020
   ttgtagccca cttccactat gtcctatcaa taggagctgt atttgccatc ataggaggct 7080
   tcattcactg atttccccta ttctcaggct acaccctaga ccaaacctac gccaaaatcc 7140
   atttcactat catattcatc ggcgtaaatc taactttctt cccacaacac tttctcggcc 7200
   tatccggaat gccccgacgt tactcggact accccgatgc atacaccaca tgaaacatcc 7260
   tatcatctgt aggctcattc atttctctaa cagcagtaat attaataatt ttcatgattt 7320
   gagaagcctt cgcttcgaag cgaaaagtcc taatagtaga agaaccctcc ataaacctgg 7380
   agtgactata tggatgcccc ccaccctacc acacattcga agaacccgta tacataaaat 7440
   ctagacaaaa aaggaaggaa tcgaaccccc caaagctggt ttcaagccaa ccccatggcc 7500
   tccatgactt tttcaaaaag gtattagaaa aaccatttca taactttgtc aaagttaaat 7560
   tataggctaa atcctatata tcttaatggc acatgcagcg caagtaggtc tacaagacgc 7620
   tacttcccct atcatagaag agcttatcac ctttcatgat cacgccctca taatcatttt 7680
   ccttatctgc ttcctagtcc tgtatgccct tttcctaaca ctcacaacaa aactaactaa 7740
   tactaacatc tcagacgctc aggaaataga aaccgtctga actatcctgc ccgccatcat 7800
   cctagtcctc atcgccctcc catccctacg catcctttac ataacagacg aggtcaacga 7860
   tccctccctt accatcaaat caattggcca ccaatggtac tgaacctacg agtacaccga 7920
   ctacggcgga ctaatcttca actcctacat acttccccca ttattcctag aaccaggcga 7980
   cctgcgactc cttgacgttg acaatcgagt agtactcccg attgaagccc ccattcgtat 8040
   aataattaca tcacaagacg tcttgcactc atgagctgtc cccacattag gcttaaaaac 8100
   agatgcaatt cccggacgtc taaaccaaac cactttcacc gctacacgac cgggggtata 8160
   ctacggtcaa tgctctgaaa tctgtggagc aaaccacagt ttcatgccca tcgtcctaga 8220
   attaattccc ctaaaaatct ttgaaatagg gcccgtattt accctatagc accccctcta 8280
   ccccctctag agcccactgt aaagctaact tagcattaac cttttaagtt aaagattaag 8340
   agaaccaaca cctctttaca gtgaaatgcc ccaactaaat actaccgtat ggcccaccat 8400
   aattaccccc atactcctta cactattcct catcacccaa ctaaaaatat taaacacaaa 8460
   ctaccaccta cctccctcac caaagcccat aaaaataaaa aattataaca aaccctgaga 8520
   accaaaatga acgaaaatct gttcgcttca ttcattgccc ccacaatcct aggcctaccc 8580
   gccgcagtac tgatcattct atttccccct ctattgatcc ccacctccaa atatctcatc 8640
   aacaaccgac taatcaccac ccaacaatga ctaatcaaac taacctcaaa acaaatgata 8700
   accatacaca acactaaagg acgaacctga tctcttatac tagtatcctt aatcattttt 8760
   attgccacaa ctaacctcct cggactcctg cctcactcat ttacaccaac cacccaacta 8820
   tctataaacc tagccatggc catcccctta tgagcgggca cagtgattat aggctttcgc 8880
   tctaagatta aaaatgccct agcccacttc ttaccacaag gcacacctac accccttatc 8940
   cccatactag ttattatcga aaccatcagc ctactcattc aaccaatagc cctggccgta 9000
   cgcctaaccg ctaacattac tgcaggccac ctactcatgc acctaattgg aagcgccacc 9060
   ctagcaatat caaccattaa ccttccctct acacttatca tcttcacaat tctaattcta 9120
   ctgactatcc tagaaatcgc tgtcgcctta atccaagcct acgttttcac acttctagta 9180
   agcctctacc tgcacgacaa cacataatga cccaccaatc acatgcctat catatagtaa 9240
   aacccagccc atgaccccta acaggggccc tctcagccct cctaatgacc tccggcctag 9300
   ccatgtgatt tcacttccac tccataacgc tcctcatact aggcctacta accaacacac 9360
   taaccatata ccaatgatgg cgcgatgtaa cacgagaaag cacataccaa ggccaccaca 9420
   caccacctgt ccaaaaaggc cttcgatacg ggataatcct atttattacc tcagaagttt 9480
   ttttcttcgc aggatttttc tgagcctttt accactccag cctagcccct accccccaat 9540
   taggagggca ctggccccca acaggcatca ccccgctaaa tcccctagaa gtcccactcc 9600
   taaacacatc cgtattactc gcatcaggag tatcaatcac ctgagctcac catagtctaa 9660
   tagaaaacaa ccgaaaccaa ataattcaag cactgcttat tacaatttta ctgggtctct 9720
   attttaccct cctacaagcc tcagagtact tcgagtctcc cttcaccatt tccgacggca 9780
   tctacggctc aacatttttt gtagccacag gcttccacgg acttcacgtc attattggct 9840
   caactttcct cactatctgc ttcatccgcc aactaatatt tcactttaca tccaaacatc 9900
   actttggctt cgaagccgcc gcctgatact ggcattttgt agatgtggtt tgactatttc 9960
   tgtatgtctc catctattga tgagggtctt actcttttag tataaatagt accgttaact 10020
   tccaattaac tagttttgac aacattcaaa aaagagtaat aaacttcgcc ttaattttaa 10080
   taatcaacac cctcctagcc ttactactaa taattattac attttgacta ccacaactca 10140
   acggctacat agaaaaatcc accccttacg agtgcggctt cgaccctata tcccccgccc 10200
   gcgtcccttt ctccataaaa ttcttcttag tagctattac cttcttatta tttgatctag 10260
   aaattgccct ccttttaccc ctaccatgag ccctacaaac aactaacctg ccactaatag 10320
   ttatgtcatc cctcttatta atcatcatcc tagccctaag tctggcctat gagtgactac 10380
   aaaaaggatt agactgaacc gaattggtat atagtttaaa caaaacgaat gatttcgact 10440
   cattaaatta tgataatcat atttaccaaa tgcccctcat ttacataaat attatactag 10500
   catttaccat ctcacttcta ggaatactag tatatcgctc acacctcata tcctccctac 10560
   tatgcctaga aggaataata ctatcgctgt tcattatagc tactctcata accctcaaca 10620
   cccactccct cttagccaat attgtgccta ttgccatact agtctttgcc gcctgcgaag 10680
   cagcggtggg cctagcccta ctagtctcaa tctccaacac atatggccta gactacgtac 10740
   ataacctaaa cctactccaa tgctaaaact aatcgtccca acaattatat tactaccact 10800
   gacatgactt tccaaaaaac acataatttg aatcaacaca accacccaca gcctaattat 10860
   tagcatcatc cctctactat tttttaacca aatcaacaac aacctattta gctgttcccc 10920
   aaccttttcc tccgaccccc taacaacccc cctcctaata ctaactacct gactcctacc 10980
   cctcacaatc atggcaagcc aacgccactt atccagtgaa ccactatcac gaaaaaaact 11040
   ctacctctct atactaatct ccctacaaat ctccttaatt ataacattca cagccacaga 11100
   actaatcata ttttatatct tcttcgaaac cacacttatc cccaccttgg ctatcatcac 11160
   ccgatgaggc aaccagccag aacgcctgaa cgcaggcaca tacttcctat tctacaccct 11220
   agtaggctcc cttcccctac tcatcgcact aatttacact cacaacaccc taggctcact 11280
   aaacattcta ctactcactc tcactgccca agaactatca aactcctgag ccaacaactt 11340
   aatatgacta gcttacacaa tagcttttat agtaaagata cctctttacg gactccactt 11400
   atgactccct aaagcccatg tcgaagcccc catcgctggg tcaatagtac ttgccgcagt 11460
   actcttaaaa ctaggcggct atggtataat acgcctcaca ctcattctca accccctgac 11520
   aaaacacata gcctacccct tccttgtact atccctatga ggcataatta taacaagctc 11580
   catctgccta cgacaaacag acctaaaatc gctcattgca tactcttcaa tcagccacat 11640
   agccctcgta gtaacagcca ttctcatcca aaccccctga agcttcaccg gcgcagtcat 11700
   tctcataatc gcccacgggc ttacatcctc attactattc tgcctagcaa actcaaacta 11760
   cgaacgcact cacagtcgca tcataatcct ctctcaagga cttcaaactc tactcccact 11820
   aatagctttt tgatgacttc tagcaagcct cgctaacctc gccttacccc ccactattaa 11880
   cctactggga gaactctctg tgctagtaac cacgttctcc tgatcaaata tcactctcct 11940
   acttacagga ctcaacatac tagtcacagc cctatactcc ctctacatat ttaccacaac 12000
   acaatggggc tcactcaccc accacattaa caacataaaa ccctcattca cacgagaaaa 12060
   caccctcatg ttcatacacc tatcccccat tctcctccta tccctcaacc ccgacatcat 12120
   taccgggttt tcctcttgta aatatagttt aaccaaaaca tcagattgtg aatctgacaa 12180
   cagaggctta cgacccctta tttaccgaga aagctcacaa gaactgctaa ctcatgcccc 12240
   catgtctaac aacatggctt tctcaacttt taaaggataa cagctatcca ttggtcttag 12300
   gccccaaaaa ttttggtgca actccaaata aaagtaataa ccatgcacac tactataacc 12360
   accctaaccc tgacttccct aattcccccc atccttacca ccctcgttaa ccctaacaaa 12420
   aaaaactcat acccccatta tgtaaaatcc attgtcgcat ccacctttat tatcagtctc 12480
   ttccccacaa caatattcat gtgcctagac caagaagtta ttatctcgaa ctgacactga 12540
   gccacaaccc aaacaaccca gctctcccta agcttcaaac tagactactt ctccataata 12600
   ttcatccctg tagcattgtt cgttacatgg tccatcatag aattctcact gtgatatata 12660
   aactcagacc caaacattaa tcagttcttc aaatatctac tcatcttcct aattaccata 12720
   ctaatcttag ttaccgctaa caacctattc caactgttca tcggctgaga gggcgtagga 12780
   attatatcct tcttgctcat cagttgatga tacgcccgag cagatgccaa cacagcagcc 12840
   attcaagcaa tcctatacaa ccgtatcggc gatatcggtt tcatcctcgc cttagcatga 12900
   tttatcctac actccaactc atgagaccca caacaaatag cccttctaaa cgctaatcca 12960
   agcctcaccc cactactagg cctcctccta gcagcagcag gcaaatcagc ccaattaggt 13020
   ctccacccct gactcccctc agccatagaa ggccccaccc cagtctcagc cctactccac 13080
   tcaagcacta tagttgtagc aggaatcttc ttactcatcc gcttccaccc cctagcagaa 13140
   aatagcccac taatccaaac tctaacacta tgcttaggcg ctatcaccac tctgttcgca 13200
   gcagtctgcg cccttacaca aaatgacatc aaaaaaatcg tagccttctc cacttcaagt 13260
   caactaggac tcataatagt tacaatcggc atcaaccaac cacacctagc attcctgcac 13320
   atctgtaccc acgccttctt caaagccata ctatttatgt gctccgggtc catcatccac 13380
   aaccttaaca atgaacaaga tattcgaaaa ataggaggac tactcaaaac catacctctc 13440
   acttcaacct ccctcaccat tggcagccta gcattagcag gaataccttt cctcacaggt 13500
   ttctactcca aagaccacat catcgaaacc gcaaacatat catacacaaa cgcctgagcc 13560
   ctatctatta ctctcatcgc tacctccctg acaagcgcct atagcactcg aataattctt 13620
   ctcaccctaa caggtcaacc tcgcttcccc acccttacta acattaacga aaataacccc 13680
   accctactaa accccattaa acgcctggca gccggaagcc tattcgcagg atttctcatt 13740
   actaacaaca tttcccccgc atcccccttc caaacaacaa tccccctcta cctaaaactc 13800
   acagccctcg ctgtcacttt cctaggactt ctaacagccc tagacctcaa ctacctaacc 13860
   aacaaactta aaataaaatc cccactatgc acattttatt tctccaacat actcggattc 13920
   taccctagca tcacacaccg cacaatcccc tatctaggcc ttcttacgag ccaaaacctg 13980
   cccctactcc tcctagacct aacctgacta gaaaagctat tacctaaaac aatttcacag 14040
   caccaaatct ccacctccat catcacctca acccaaaaag gcataattaa actttacttc 14100
   ctctctttct tcttcccact catcctaacc ctactcctaa tcacataacc tattcccccg 14160
   agcaatctca attacaatat atacaccaac aaacaatgtt caaccagtaa ctactactaa 14220
   tcaacgccca taatcataca aagcccccgc accaatagga tcctcccgaa tcaaccctga 14280
   cccctctcct tcataaatta ttcagcttcc tacactatta aagtttacca caaccaccac 14340
   cccatcatac tctttcaccc acagcaccaa tcctacctcc atcgctaacc ccactaaaac 14400
   actcaccaag acctcaaccc ctgaccccca tgcctcagga tactcctcaa tagccatcgc 14460
   tgtagtatat ccaaagacaa ccatcattcc ccctaaataa attaaaaaaa ctattaaacc 14520
   catataacct cccccaaaat tcagaataat aacacacccg accacaccgc taacaatcaa 14580
   tactaaaccc ccataaatag gagaaggctt agaagaaaac cccacaaacc ccattactaa 14640
   acccacactc aacagaaaca aagcatacat cattattctc gcacggacta caaccacgac 14700
   caatgatatg aaaaaccatc gttgtatttc aactacaaga acaccaatga ccccaatacg 14760
   caaaactaac cccctaataa aattaattaa ccactcattc atcgacctcc ccaccccatc 14820
   caacatctcc gcatgatgaa acttcggctc actccttggc gcctgcctga tcctccaaat 14880
   caccacagga ctattcctag ccatgcacta ctcaccagac gcctcaaccg ccttttcatc 14940
   aatcgcccac atcactcgag acgtaaatta tggctgaatc atccgctacc ttcacgccaa 15000
   tggcgcctca atattcttta tctgcctctt cctacacatc gggcgaggcc tatattacgg 15060
   atcatttctc tactcagaaa cctgaaacat cggcattatc ctcctgcttg caactatagc 15120
   aacagccttc ataggctatg tcctcccgtg aggccaaata tcattctgag gggccacagt 15180
   aattacaaac ttactatccg ccatcccata cattgggaca gacctagttc aatgaatctg 15240
   aggaggctac tcagtagaca gtcccaccct cacacgattc tttacctttc acttcatctt 15300
   gcccttcatt attgcagccc tagcaacact ccacctccta ttcttgcacg aaacgggatc 15360
   aaacaacccc ctaggaatca cctcccattc cgataaaatc accttccacc cttactacac 15420
   aatcaaagac gccctcggct tacttctctt ccttctctcc ttaatgacat taacactatt 15480
   ctcaccagac ctcctaggcg acccagacaa ttatacccta gccaacccct taaacacccc 15540
   tccccacatc aagcccgaat gatatttcct attcgcctac acaattctcc gatccgtccc 15600
   taacaaacta ggaggcgtcc ttgccctatt actatccatc ctcatcctag caataatccc 15660
   catcctccat atatccaaac aacaaagcat aatatttcgc ccactaagcc aatcacttta 15720
   ttgactccta gccgcagacc tcctcattct aacctgaatc ggaggacaac cagtaagcta 15780
   cccttttacc atcattggac aagtagcatc cgtactatac ttcacaacaa tcctaatcct 15840
   aataccaact atctccctaa ttgaaaacaa aatactcaaa tgggcctgtc cttgtagtat 15900
   aaactaatac accagtcttg taaaccggag atgaaaacct ttttccaagg acaaatcaga 15960
   gaaaaagtct ttaactccac cattagcacc caaagctaag attctaattt aaactattct 16020
   ctgttctttc atggggaagc agatttgggt accacccaag tattgactca cccatcaaca 16080
   accgctatgt atttcgtaca ttactgccag ccaccatgaa tattgtacgg taccataaat 16140
   acttgaccac ctgtagtaca taaaaaccca atccacatca aaaccccctc cccatgctta 16200
   caagcaagta cagcaatcaa ccctcaacta tcacacatca actgcaactc caaagccacc 16260
   cctcacccac taggatacca acaaacctac ccacccttaa cagtacatag tacataaagc 16320
   catttaccgt acatagcaca ttacagtcaa atcccttctc gtccccatgg atgacccccc 16380
   tcagataggg gtcccttgac caccatcctc cgtgaaatca atatcccgca caagagtgct 16440
   actctcctcg ctccgggccc ataacacttg ggggtagcta aagtgaactg tatccgacat 16500
   ctggttccta cttcagggtc ataaagccta aatagcccac acgttcccct taaataagac 16560
   atcacgatg 16569
<210> 2
   <211> 318
   <212> PRT
   <213> homo sapiens
<400> 2
   Met Pro Met Ala Asn Leu Leu Leu Leu Ile Val Pro Ile Leu Ile Ala
   1               5                   10                  15
   Met Ala Phe Leu Met Leu Thr Glu Arg Lys Ile Leu Gly Tyr Met Gln
               20                  25                  30
   Leu Arg Lys Gly Pro Asn Val Val Gly Pro Tyr Gly Leu Leu Gln Pro
           35                  40                  45
   Phe Ala Asp Ala Met Lys Leu Phe Thr Lys Glu Pro Leu Lys Pro Ala
       50                  55                  60
   Thr Ser Thr Ile Thr Leu Tyr Ile Thr Ala Pro Thr Leu Ala Leu Thr
   65                  70                  75                  80
   Ile Ala Leu Leu Leu Trp Thr Pro Leu Pro Met Pro Asn Pro Leu Val
                   85                  90                  95
   Asn Leu Asn Leu Gly Leu Leu Phe Ile Leu Ala Thr Ser Ser Leu Ala
               100                 105                 110
   Val Tyr Ser Ile Leu Trp Ser Gly Trp Ala Ser Asn Ser Asn Tyr Ala
           115                 120                 125
   Leu Ile Gly Ala Leu Arg Ala Val Ala Gln Thr Ile Ser Tyr Glu Val
       130                 135                 140
   Thr Leu Ala Ile Ile Leu Leu Ser Thr Leu Leu Met Ser Gly Ser Phe
   145                 150                 155                 160
   Asn Leu Ser Thr Leu Ile Thr Thr Gln Glu His Leu Trp Leu Leu Leu
                   165                 170                 175
   Pro Ser Trp Pro Leu Ala Met Met Trp Phe Ile Ser Thr Leu Ala Glu
               180                 185                 190
   Thr Asn Arg Thr Pro Phe Asp Leu Ala Glu Gly Glu Ser Glu Leu Val
           195                 200                 205
   Ser Gly Phe Asn Ile Glu Tyr Ala Ala Gly Pro Phe Ala Leu Phe Phe
       210                 215                 220
   Met Ala Glu Tyr Thr Asn Ile Ile Met Met Asn Thr Leu Thr Thr Thr
   225                 230                 235                 240
   Ile Phe Leu Gly Thr Thr Tyr Asp Ala Leu Ser Pro Glu Leu Tyr Thr
                   245                 250                 255
   Thr Tyr Phe Val Thr Lys Thr Leu Leu Leu Thr Ser Leu Phe Leu Trp
               260                 265                 270
   Ile Arg Thr Ala Tyr Pro Arg Phe Arg Tyr Asp Gln Leu Met His Leu
           275                 280                 285
   Leu Trp Lys Asn Phe Leu Pro Leu Thr Leu Ala Leu Leu Met Trp Tyr
       290                 295                 300
   Val Ser Met Pro Ile Thr Ile Ser Ser Ile Pro Pro Gln Thr
   305                 310                 315
<210> 3
   <211> 347
   <212> PRT
   <213> homo sapiens
<400> 3
   Met Asn Pro Leu Ala Gln Pro Val Ile Tyr Ser Thr Ile Phe Ala Gly
   1               5                   10                  15
   Thr Leu Ile Thr Ala Leu Ser Ser His Trp Phe Phe Thr Trp Val Gly
               20                  25                  30
   Leu Glu Met Asn Met Leu Ala Phe Ile Pro Val Leu Thr Lys Lys Met
           35                  40                  45
   Asn Pro Arg Ser Thr Glu Ala Ala Ile Lys Tyr Phe Leu Thr Gln Ala
       50                  55                  60
   Thr Ala Ser Met Ile Leu Leu Met Ala Ile Leu Phe Asn Asn Met Leu
   65                  70                  75                  80
   Ser Gly Gln Trp Thr Met Thr Asn Thr Thr Asn Gln Tyr Ser Ser Leu
                   85                  90                  95
   Met Ile Met Met Ala Met Ala Met Lys Leu Gly Met Ala Pro Phe His
               100                 105                 110
   Phe Trp Val Pro Glu Val Thr Gln Gly Thr Pro Leu Thr Ser Gly Leu
           115                 120                 125
   Leu Leu Leu Thr Trp Gln Lys Leu Ala Pro Ile Ser Ile Met Tyr Gln
       130                 135                 140
   Ile Ser Pro Ser Leu Asn Val Ser Leu Leu Leu Thr Leu Ser Ile Leu
   145                 150                 155                 160
   Ser Ile Met Ala Gly Ser Trp Gly Gly Leu Asn Gln Thr Gln Leu Arg
                   165                 170                 175
   Lys Ile Leu Ala Tyr Ser Ser Ile Thr His Met Gly Trp Met Met Ala
               180                 185                 190
   Val Leu Pro Tyr Asn Pro Asn Met Thr Ile Leu Asn Leu Thr Ile Tyr
           195                 200                 205
   Ile Ile Leu Thr Thr Thr Ala Phe Leu Leu Leu Asn Leu Asn Ser Ser
       210                 215                 220
   Thr Thr Thr Leu Leu Leu Ser Arg Thr Trp Asn Lys Leu Thr Trp Leu
   225                 230                 235                 240
   Thr Pro Leu Ile Pro Ser Thr Leu Leu Ser Leu Gly Gly Leu Pro Pro
                   245                 250                 255
   Leu Thr Gly Phe Leu Pro Lys Trp Ala Ile Ile Glu Glu Phe Thr Lys
               260                 265                 270
   Asn Asn Ser Leu Ile Ile Pro Thr Ile Met Ala Thr Ile Thr Leu Leu
           275                 280                 285
   Asn Leu Tyr Phe Tyr Leu Arg Leu Ile Tyr Ser Thr Ser Ile Thr Leu
       290                 295                 300
   Leu Pro Met Ser Asn Asn Val Lys Met Lys Trp Gln Phe Glu His Thr
   305                 310                 315                 320
   Lys Pro Thr Pro Phe Leu Pro Thr Leu Ile Ala Leu Thr Thr Leu Leu
   325 330 335
   Leu Pro Ile Ser Pro Phe Met Leu Met Ile Leu
   340 345
<210> 4
   <211> 513
   <212> PRT
   <213> homo sapiens
<400> 4
   Met Phe Ala Asp Arg Trp Leu Phe Ser Thr Asn His Lys Asp Ile Gly
   1 5 10 15
   Thr Leu Tyr Leu Leu Phe Gly Ala Trp Ala Gly Val Leu Gly Thr Ala
   20 25 30
   Leu Ser Leu Leu Ile Arg Ala Glu Leu Gly Gln Pro Gly Asn Leu Leu
   35 40 45
   Gly Asn Asp His Ile Tyr Asn Val Ile Val Thr Ala His Ala Phe Val
   50 55 60
   Met Ile Phe Phe Met Val Met Pro Ile Met Ile Gly Gly Phe Gly Asn
   65 70 75 80
   Trp Leu Val Pro Leu Met Ile Gly Ala Pro Asp Met Ala Phe Pro Arg
   85 90 95
   Met Asn Asn Met Ser Phe Trp Leu Leu Pro Pro Ser Leu Leu Leu Leu
   100 105 110
   Leu Ala Ser Ala Met Val Glu Ala Gly Ala Gly Thr Gly Trp Thr Val
   115 120 125
   Tyr Pro Pro Leu Ala Gly Asn Tyr Ser His Pro Gly Ala Ser Val Asp
   130 135 140
   Leu Thr Ile Phe Ser Leu His Leu Ala Gly Val Ser Ser Ile Leu Gly
   145 150 155 160
   Ala Ile Asn Phe Ile Thr Thr Ile Ile Asn Met Lys Pro Pro Ala Met
   165 170 175
   Thr Gln Tyr Gln Thr Pro Leu Phe Val Trp Ser Val Leu Ile Thr Ala
   180 185 190
   Val Leu Leu Leu Leu Ser Leu Pro Val Leu Ala Ala Gly Ile Thr Met
           195                 200                 205
   Leu Leu Thr Asp Arg Asn Leu Asn Thr Thr Phe Phe Asp Pro Ala Gly
       210                 215                 220
   Gly Gly Asp Pro Ile Leu Tyr Gln His Leu Phe Trp Phe Phe Gly His
   225                 230                 235                 240
   Pro Glu Val Tyr Ile Leu Ile Leu Pro Gly Phe Gly Met Ile Ser His
                   245                 250                 255
   Ile Val Thr Tyr Tyr Ser Gly Lys Lys Glu Pro Phe Gly Tyr Met Gly
               260                 265                 270
   Met Val Trp Ala Met Met Ser Ile Gly Phe Leu Gly Phe Ile Val Trp
           275                 280                 285
   Ala His His Met Phe Thr Val Gly Met Asp Val Asp Thr Arg Ala Tyr
       290                 295                 300
   Phe Thr Ser Ala Thr Met Ile Ile Ala Ile Pro Thr Gly Val Lys Val
   305                 310                 315                 320
   Phe Ser Trp Leu Ala Thr Leu His Gly Ser Asn Met Lys Trp Ser Ala
                   325                 330                 335
   Ala Val Leu Trp Ala Leu Gly Phe Ile Phe Leu Phe Thr Val Gly Gly
               340                 345                 350
   Leu Thr Gly Ile Val Leu Ala Asn Ser Ser Leu Asp Ile Val Leu His
           355                 360                 365
   Asp Thr Tyr Tyr Val Val Ala His Phe His Tyr Val Leu Ser Met Gly
       370                 375                 380
   Ala Val Phe Ala Ile Met Gly Gly Phe Ile His Trp Phe Pro Leu Phe
   385                 390                 395                 400
   Ser Gly Tyr Thr Leu Asp Gln Thr Tyr Ala Lys Ile His Phe Thr Ile
                   405                 410                 415
   Met Phe Ile Gly Val Asn Leu Thr Phe Phe Pro Gln His Phe Leu Gly
               420                 425                 430
   Leu Ser Gly Met Pro Arg Arg Tyr Ser Asp Tyr Pro Asp Ala Tyr Thr
           435                 440                 445
   Thr Trp Asn Ile Leu Ser Ser Val Gly Ser Phe Ile Ser Leu Thr Ala
   450 455 460
   Val Met Leu Met Ile Phe Met Ile Trp Glu Ala Phe Ala Ser Lys Arg
   465 470 475 480
   Lys Val Leu Met Val Glu Glu Pro Ser Met Asn Leu Glu Trp Leu Tyr
   485 490 495
   Gly Cys Pro Pro Pro Tyr His Thr Phe Glu Glu Pro Val Tyr Met Lys
   500 505 510
   Ser
<210> 5
   <211> 227
   <212> PRT
   <213> homo sapiens
<400> 5
   Met Ala His Ala Ala Gln Val Gly Leu Gln Asp Ala Thr Ser Pro Ile
   1 5 10 15
   Met Glu Glu Leu Ile Thr Phe His Asp His Ala Leu Met Ile Ile Phe
   20 25 30
   Leu Ile Cys Phe Leu Val Leu Tyr Ala Leu Phe Leu Thr Leu Thr Thr
   35 40 45
   Lys Leu Thr Asn Thr Asn Ile Ser Asp Ala Gln Glu Met Glu Thr Val
   50 55 60
   Trp Thr Ile Leu Pro Ala Ile Ile Leu Val Leu Ile Ala Leu Pro Ser
   65 70 75 80
   Leu Arg Ile Leu Tyr Met Thr Asp Glu Val Asn Asp Pro Ser Leu Thr
   85 90 95
   Ile Lys Ser Ile Gly His Gln Trp Tyr Trp Thr Tyr Glu Tyr Thr Asp
   100 105 110
   Tyr Gly Gly Leu Ile Phe Asn Ser Tyr Met Leu Pro Pro Leu Phe Leu
   115 120 125
   Glu Pro Gly Asp Leu Arg Leu Leu Asp Val Asp Asn Arg Val Val Leu
   130 135 140
   Pro Ile Glu Ala Pro Ile Arg Met Met Ile Thr Ser Gln Asp Val Leu
   145                 150                 155                 160
   His Ser Trp Ala Val Pro Thr Leu Gly Leu Lys Thr Asp Ala Ile Pro
                   165                 170                 175
   Gly Arg Leu Asn Gln Thr Thr Phe Thr Ala Thr Arg Pro Gly Val Tyr
               180                 185                 190
   Tyr Gly Gln Cys Ser Glu Ile Cys Gly Ala Asn His Ser Phe Met Pro
           195                 200                 205
   Ile Val Leu Glu Leu Ile Pro Leu Lys Ile Phe Glu Met Gly Pro Val
       210                 215                 220
   Phe Thr Leu
   225
<210> 6
   <211> 68
   <212> PRT
   <213> homo sapiens
<400> 6
   Met Pro Gln Leu Asn Thr Thr Val Trp Pro Thr Met Ile Thr Pro Met
   1               5                   10                  15
   Leu Leu Thr Leu Phe Leu Ile Thr Gln Leu Lys Met Leu Asn Thr Asn
               20                  25                  30
   Tyr His Leu Pro Pro Ser Pro Lys Pro Met Lys Met Lys Asn Tyr Asn
           35                  40                  45
   Lys Pro Trp Glu Pro Lys Trp Thr Lys Ile Cys Ser Leu His Ser Leu
       50                  55                  60
   Pro Pro Gln Ser
   65
<210> 7
   <211> 226
   <212> PRT
   <213> homo sapiens
<400> 7
   Met Asn Glu Asn Leu Phe Ala Ser Phe Ile Ala Pro Thr Ile Leu Gly
   1               5                   10                  15
   Leu Pro Ala Ala Val Leu Ile Ile Leu Phe Pro Pro Leu Leu Ile Pro
               20                  25                  30
   Thr Ser Lys Tyr Leu Ile Asn Asn Arg Leu Ile Thr Thr Gln Gln Trp
           35                  40                  45
   Leu Ile Lys Leu Thr Ser Lys Gln Met Met Thr Met His Asn Thr Lys
       50                  55                  60
   Gly Arg Thr Trp Ser Leu Met Leu Val Ser Leu Ile Ile Phe Ile Ala
   65                  70                  75                  80
   Thr Thr Asn Leu Leu Gly Leu Leu Pro His Ser Phe Thr Pro Thr Thr
                   85                  90                  95
   Gln Leu Ser Met Asn Leu Ala Met Ala Ile Pro Leu Trp Ala Gly Thr
               100                 105                 110
   Val Ile Met Gly Phe Arg Ser Lys Ile Lys Asn Ala Leu Ala His Phe
           115                 120                 125
   Leu Pro Gln Gly Thr Pro Thr Pro Leu Ile Pro Met Leu Val Ile Ile
       130                 135                 140
   Glu Thr Ile Ser Leu Leu Ile Gln Pro Met Ala Leu Ala Val Arg Leu
   145                 150                 155                 160
   Thr Ala Asn Ile Thr Ala Gly His Leu Leu Met His Leu Ile Gly Ser
                   165                 170                 175
   Ala Thr Leu Ala Met Ser Thr Ile Asn Leu Pro Ser Thr Leu Ile Ile
               180                 185                 190
   Phe Thr Ile Leu Ile Leu Leu Thr Ile Leu Glu Ile Ala Val Ala Leu
           195                 200                 205
   Ile Gln Ala Tyr Val Phe Thr Leu Leu Val Ser Leu Tyr Leu His Asp
       210                 215                 220
   Asn Thr
   225
<210> 8
   <211> 261
   <212> PRT
   <213> homo sapiens
<400> 8
   Met Thr His Gln Ser His Ala Tyr His Met Val Lys Pro Ser Pro Trp
   1               5                   10                  15
   Pro Leu Thr Gly Ala Leu Ser Ala Leu Leu Met Thr Ser Gly Leu Ala
               20                  25                  30
   Met Trp Phe His Phe His Ser Met Thr Leu Leu Met Leu Gly Leu Leu
           35                  40                  45
   Thr Asn Thr Leu Thr Met Tyr Gln Trp Trp Arg Asp Val Thr Arg Glu
       50                  55                  60
   Ser Thr Tyr Gln Gly His His Thr Pro Pro Val Gln Lys Gly Leu Arg
   65                  70                  75                  80
   Tyr Gly Met Ile Leu Phe Ile Thr Ser Glu Val Phe Phe Phe Ala Gly
                   85                  90                  95
   Phe Phe Trp Ala Phe Tyr His Ser Ser Leu Ala Pro Thr Pro Gln Leu
               100                 105                 110
   Gly Gly His Trp Pro Pro Thr Gly Ile Thr Pro Leu Asn Pro Leu Glu
           115                 120                 125
   Val Pro Leu Leu Asn Thr Ser Val Leu Leu Ala Ser Gly Val Ser Ile
       130                 135                 140
   Thr Trp Ala His His Ser Leu Met Glu Asn Asn Arg Asn Gln Met Ile
   145                 150                 155                 160
   Gln Ala Leu Leu Ile Thr Ile Leu Leu Gly Leu Tyr Phe Thr Leu Leu
                   165                 170                 175
   Gln Ala Ser Glu Tyr Phe Glu Ser Pro Phe Thr Ile Ser Asp Gly Ile
               180                 185                 190
   Tyr Gly Ser Thr Phe Phe Val Ala Thr Gly Phe His Gly Leu His Val
           195                 200                 205
   Ile Ile Gly Ser Thr Phe Leu Thr Ile Cys Phe Ile Arg Gln Leu Met
       210                 215                 220
   Phe His Phe Thr Ser Lys His His Phe Gly Phe Glu Ala Ala Ala Trp
   225                 230                 235                 240
   Tyr Trp His Phe Val Asp Val Val Trp Leu Phe Leu Tyr Val Ser Ile
                   245                 250                 255
   Tyr Trp Trp Gly Ser
   260
<210> 9
   <211> 115
   <212> PRT
   <213> homo sapiens
<400> 9
   Met Asn Phe Ala Leu Ile Leu Met Ile Asn Thr Leu Leu Ala Leu Leu
   1               5                   10                  15
   Leu Met Ile Ile Thr Phe Trp Leu Pro Gln Leu Asn Gly Tyr Met Glu
               20                  25                  30
   Lys Ser Thr Pro Tyr Glu Cys Gly Phe Asp Pro Met Ser Pro Ala Arg
           35                  40                  45
   Val Pro Phe Ser Met Lys Phe Phe Leu Val Ala Ile Thr Phe Leu Leu
       50                  55                  60
   Phe Asp Leu Glu Ile Ala Leu Leu Leu Pro Leu Pro Trp Ala Leu Gln
   65                  70                  75                  80
   Thr Thr Asn Leu Pro Leu Met Val Met Ser Ser Leu Leu Leu Ile Ile
                   85                  90                  95
   Ile Leu Ala Leu Ser Leu Ala Tyr Glu Trp Leu Gln Lys Gly Leu Asp
               100                 105                 110
   Trp Thr Glu
           115
<210> 10
   <211> 98
   <212> PRT
   <213> homo sapiens
<400> 10
   Met Pro Leu Ile Tyr Met Asn Ile Met Leu Ala Phe Thr Ile Ser Leu
   1               5                   10                  15
   Leu Gly Met Leu Val Tyr Arg Ser His Leu Met Ser Ser Leu Leu Cys
               20                  25                  30
   Leu Glu Gly Met Met Leu Ser Leu Phe Ile Met Ala Thr Leu Met Thr
           35                  40                  45
   Leu Asn Thr His Ser Leu Leu Ala Asn Ile Val Pro Ile Ala Met Leu
       50                  55                  60
   Val Phe Ala Ala Cys Glu Ala Ala Val Gly Leu Ala Leu Leu Val Ser
   65                  70                  75                  80
   Ile Ser Asn Thr Tyr Gly Leu Asp Tyr Val His Asn Leu Asn Leu Leu
                   85                  90                  95
   Gln Cys
<210> 11
   <211> 459
   <212> PRT
   <213> homo sapiens
<400> 11
   Met Leu Lys Leu Ile Val Pro Thr Ile Met Leu Leu Pro Leu Thr Trp
   1               5                   10                  15
   Leu Ser Lys Lys His Met Ile Trp Ile Asn Thr Thr Thr His Ser Leu
               20                  25                  30
   Ile Ile Ser Ile Ile Pro Leu Leu Phe Phe Asn Gln Ile Asn Asn Asn
           35                  40                  45
   Leu Phe Ser Cys Ser Pro Thr Phe Ser Ser Asp Pro Leu Thr Thr Pro
       50                  55                  60
   Leu Leu Met Leu Thr Thr Trp Leu Leu Pro Leu Thr Ile Met Ala Ser
   65                  70                  75                  80
   Gln Arg His Leu Ser Ser Glu Pro Leu Ser Arg Lys Lys Leu Tyr Leu
                   85                  90                  95
   Ser Met Leu Ile Ser Leu Gln Ile Ser Leu Ile Met Thr Phe Thr Ala
               100                 105                 110
   Thr Glu Leu Ile Met Phe Tyr Ile Phe Phe Glu Thr Thr Leu Ile Pro
           115                 120                 125
   Thr Leu Ala Ile Ile Thr Arg Trp Gly Asn Gln Pro Glu Arg Leu Asn
       130                 135                 140
   Ala Gly Thr Tyr Phe Leu Phe Tyr Thr Leu Val Gly Ser Leu Pro Leu
   145                 150                 155                 160
   Leu Ile Ala Leu Ile Tyr Thr His Asn Thr Leu Gly Ser Leu Asn Ile
                   165                 170                 175
   Leu Leu Leu Thr Leu Thr Ala Gln Glu Leu Ser Asn Ser Trp Ala Asn
               180                 185                 190
   Asn Leu Met Trp Leu Ala Tyr Thr Met Ala Phe Met Val Lys Met Pro
           195                 200                 205
   Leu Tyr Gly Leu His Leu Trp Leu Pro Lys Ala His Val Glu Ala Pro
       210                 215                 220
   Ile Ala Gly Ser Met Val Leu Ala Ala Val Leu Leu Lys Leu Gly Gly
   225                 230                 235                 240
   Tyr Gly Met Met Arg Leu Thr Leu Ile Leu Asn Pro Leu Thr Lys His
                   245                 250                 255
   Met Ala Tyr Pro Phe Leu Val Leu Ser Leu Trp Gly Met Ile Met Thr
               260                 265                 270
   Ser Ser Ile Cys Leu Arg Gln Thr Asp Leu Lys Ser Leu Ile Ala Tyr
           275                 280                 285
   Ser Ser Ile Ser His Met Ala Leu Val Val Thr Ala Ile Leu Ile Gln
       290                 295                 300
   Thr Pro Trp Ser Phe Thr Gly Ala Val Ile Leu Met Ile Ala His Gly
   305                 310                 315                 320
   Leu Thr Ser Ser Leu Leu Phe Cys Leu Ala Asn Ser Asn Tyr Glu Arg
                   325                 330                 335
   Thr His Ser Arg Ile Met Ile Leu Ser Gln Gly Leu Gln Thr Leu Leu
               340                 345                 350
   Pro Leu Met Ala Phe Trp Trp Leu Leu Ala Ser Leu Ala Asn Leu Ala
           355                 360                 365
   Leu Pro Pro Thr Ile Asn Leu Leu Gly Glu Leu Ser Val Leu Val Thr
       370                 375                 380
   Thr Phe Ser Trp Ser Asn Ile Thr Leu Leu Leu Thr Gly Leu Asn Met
   385                 390                 395                 400
   Leu Val Thr Ala Leu Tyr Ser Leu Tyr Met Phe Thr Thr Thr Gln Trp
                   405                 410                 415
   Gly Ser Leu Thr His His Ile Asn Asn Met Lys Pro Ser Phe Thr Arg
   420 425 430
   Glu Asn Thr Leu Met Phe Met His Leu Ser Pro Ile Leu Leu Leu Ser
   435 440 445
   Leu Asn Pro Asp Ile Ile Thr Gly Phe Ser Ser
   450 455
<210> 12
   <211> 603
   <212> PRT
   <213> homo sapiens
<400> 12
   Met Thr Met His Thr Thr Met Thr Thr Leu Thr Leu Thr Ser Leu Ile
   1 5 10 15
   Pro Pro Ile Leu Thr Thr Leu Val Asn Pro Asn Lys Lys Asn Ser Tyr
   20 25 30
   Pro His Tyr Val Lys Ser Ile Val Ala Ser Thr Phe Ile Ile Ser Leu
   35 40 45
   Phe Pro Thr Thr Met Phe Met Cys Leu Asp Gln Glu Val Ile Ile Ser
   50 55 60
   Asn Trp His Trp Ala Thr Thr Gln Thr Thr Gln Leu Ser Leu Ser Phe
   65 70 75 80
   Lys Leu Asp Tyr Phe Ser Met Met Phe Ile Pro Val Ala Leu Phe Val
   85 90 95
   Thr Trp Ser Ile Met Glu Phe Ser Leu Trp Tyr Met Asn Ser Asp Pro
   100 105 110
   Asn Ile Asn Gln Phe Phe Lys Tyr Leu Leu Ile Phe Leu Ile Thr Met
   115 120 125
   Leu Ile Leu Val Thr Ala Asn Asn Leu Phe Gln Leu Phe Ile Gly Trp
   130 135 140
   Glu Gly Val Gly Ile Met Ser Phe Leu Leu Ile Ser Trp Trp Tyr Ala
   145 150 155 160
   Arg Ala Asp Ala Asn Thr Ala Ala Ile Gln Ala Ile Leu Tyr Asn Arg
   165 170 175
   Ile Gly Asp Ile Gly Phe Ile Leu Ala Leu Ala Trp Phe Ile Leu His
               180                 185                 190
   Ser Asn Ser Trp Asp Pro Gln Gln Met Ala Leu Leu Asn Ala Asn Pro
           195                 200                 205
   Ser Leu Thr Pro Leu Leu Gly Leu Leu Leu Ala Ala Ala Gly Lys Ser
       210                 215                 220
   Ala Gln Leu Gly Leu His Pro Trp Leu Pro Ser Ala Met Glu Gly Pro
   225                 230                 235                 240
   Thr Pro Val Ser Ala Leu Leu His Ser Ser Thr Met Val Val Ala Gly
                   245                 250                 255
   Ile Phe Leu Leu Ile Arg Phe His Pro Leu Ala Glu Asn Ser Pro Leu
               260                 265                 270
   Ile Gln Thr Leu Thr Leu Cys Leu Gly Ala Ile Thr Thr Leu Phe Ala
           275                 280                 285
   Ala Val Cys Ala Leu Thr Gln Asn Asp Ile Lys Lys Ile Val Ala Phe
       290                 295                 300
   Ser Thr Ser Ser Gln Leu Gly Leu Met Met Val Thr Ile Gly Ile Asn
   305                 310                 315                 320
   Gln Pro His Leu Ala Phe Leu His Ile Cys Thr His Ala Phe Phe Lys
                   325                 330                 335
   Ala Met Leu Phe Met Cys Ser Gly Ser Ile Ile His Asn Leu Asn Asn
               340                 345                 350
   Glu Gln Asp Ile Arg Lys Met Gly Gly Leu Leu Lys Thr Met Pro Leu
           355                 360                 365
   Thr Ser Thr Ser Leu Thr Ile Gly Ser Leu Ala Leu Ala Gly Met Pro
       370                 375                 380
   Phe Leu Thr Gly Phe Tyr Ser Lys Asp His Ile Ile Glu Thr Ala Asn
   385                 390                 395                 400
   Met Ser Tyr Thr Asn Ala Trp Ala Leu Ser Ile Thr Leu Ile Ala Thr
                   405                 410                 415
   Ser Leu Thr Ser Ala Tyr Ser Thr Arg Met Ile Leu Leu Thr Leu Thr
               420                 425                 430
   Gly Gln Pro Arg Phe Pro Thr Leu Thr Asn Ile Asn Glu Asn Asn Pro
   435 440 445
   Thr Leu Leu Asn Pro Ile Lys Arg Leu Ala Ala Gly Ser Leu Phe Ala
   450 455 460
   Gly Phe Leu Ile Thr Asn Asn Ile Ser Pro Ala Ser Pro Phe Gln Thr
   465 470 475 480
   Thr Ile Pro Leu Tyr Leu Lys Leu Thr Ala Leu Ala Val Thr Phe Leu
   485 490 495
   Gly Leu Leu Thr Ala Leu Asp Leu Asn Tyr Leu Thr Asn Lys Leu Lys
   500 505 510
   Met Lys Ser Pro Leu Cys Thr Phe Tyr Phe Ser Asn Met Leu Gly Phe
   515 520 525
   Tyr Pro Ser Ile Thr His Arg Thr Ile Pro Tyr Leu Gly Leu Leu Thr
   530 535 540
   Ser Gln Asn Leu Pro Leu Leu Leu Leu Asp Leu Thr Trp Leu Glu Lys
   545 550 555 560
   Leu Leu Pro Lys Thr Ile Ser Gln His Gln Ile Ser Thr Ser Ile Ile
   565 570 575
   Thr Ser Thr Gln Lys Gly Met Ile Lys Leu Tyr Phe Leu Ser Phe Phe
   580 585 590
   Phe Pro Leu Ile Leu Thr Leu Leu Leu Ile Thr
   595 600
<210> 13
   <211> 174
   <212> PRT
   <213> homo sapiens
<400> 13
   Met Met Tyr Ala Leu Phe Leu Leu Ser Val Gly Leu Val Met Gly Phe
   1 5 10 15
   Val Gly Phe Ser Ser Lys Pro Ser Pro Ile Tyr Gly Gly Leu Val Leu
   20 25 30
   Ile Val Ser Gly Val Val Gly Cys Val Ile Ile Leu Asn Phe Gly Gly
   35 40 45
   Gly Tyr Met Gly Leu Met Val Phe Leu Ile Tyr Leu Gly Gly Met Met
       50                  55                  60
   Val Val Phe Gly Tyr Thr Thr Ala Met Ala Ile Glu Glu Tyr Pro Glu
   65                  70                  75                  80
   Ala Trp Gly Ser Gly Val Glu Val Leu Val Ser Val Leu Val Gly Leu
                   85                  90                  95
   Ala Met Glu Val Gly Leu Val Leu Trp Val Lys Glu Tyr Asp Gly Val
               100                 105                 110
   Val Val Val Val Asn Phe Asn Ser Val Gly Ser Trp Met Ile Tyr Glu
           115                 120                 125
   Gly Glu Gly Ser Gly Leu Ile Arg Glu Asp Pro Ile Gly Ala Gly Ala
       130                 135                 140
   Leu Tyr Asp Tyr Gly Arg Trp Leu Val Val Val Thr Gly Trp Thr Leu
   145                 150                 155                 160
   Phe Val Gly Val Tyr Ile Val Ile Glu Ile Ala Arg Gly Asn
                   165                 170
<210> 14
   <211> 380
   <212> PRT
   <213> Homo sapiens
<400> 14
   Met Thr Pro Met Arg Lys Thr Asn Pro Leu Met Lys Leu Ile Asn His
   1               5                   10                  15
   Ser Phe Ile Asp Leu Pro Thr Pro Ser Asn Ile Ser Ala Trp Trp Asn
               20                  25                  30
   Phe Gly Ser Leu Leu Gly Ala Cys Leu Ile Leu Gln Ile Thr Thr Gly
           35                  40                  45
   Leu Phe Leu Ala Met His Tyr Ser Pro Asp Ala Ser Thr Ala Phe Ser
       50                  55                  60
   Ser Ile Ala His Ile Thr Arg Asp Val Asn Tyr Gly Trp Ile Ile Arg
   65                  70                  75                  80
   Tyr Leu His Ala Asn Gly Ala Ser Met Phe Phe Ile Cys Leu Phe Leu
                   85                  90                  95
   His Ile Gly Arg Gly Leu Tyr Tyr Gly Ser Phe Leu Tyr Ser Glu Thr
               100                 105                 110
   Trp Asn Ile Gly Ile Ile Leu Leu Leu Ala Thr Met Ala Thr Ala Phe
           115                 120                 125
   Met Gly Tyr Val Leu Pro Trp Gly Gln Met Ser Phe Trp Gly Ala Thr
       130                 135                 140
   Val Ile Thr Asn Leu Leu Ser Ala Ile Pro Tyr Ile Gly Thr Asp Leu
   145                 150                 155                 160
   Val Gln Trp Ile Trp Gly Gly Tyr Ser Val Asp Ser Pro Thr Leu Thr
                   165                 170                 175
   Arg Phe Phe Thr Phe His Phe Ile Leu Pro Phe Ile Ile Ala Ala Leu
               180                 185                 190
   Ala Thr Leu His Leu Leu Phe Leu His Glu Thr Gly Ser Asn Asn Pro
           195                 200                 205
   Leu Gly Ile Thr Ser His Ser Asp Lys Ile Thr Phe His Pro Tyr Tyr
       210                 215                 220
   Thr Ile Lys Asp Ala Leu Gly Leu Leu Leu Phe Leu Leu Ser Leu Met
   225                 230                 235                 240
   Thr Leu Thr Leu Phe Ser Pro Asp Leu Leu Gly Asp Pro Asp Asn Tyr
                   245                 250                 255
   Thr Leu Ala Asn Pro Leu Asn Thr Pro Pro His Ile Lys Pro Glu Trp
               260                 265                 270
   Tyr Phe Leu Phe Ala Tyr Thr Ile Leu Arg Ser Val Pro Asn Lys Leu
           275                 280                 285
   Gly Gly Val Leu Ala Leu Leu Leu Ser Ile Leu Ile Leu Ala Met Ile
       290                 295                 300
   Pro Ile Leu His Met Ser Lys Gln Gln Ser Met Met Phe Arg Pro Leu
   305                 310                 315                 320
   Ser Gln Ser Leu Tyr Trp Leu Leu Ala Ala Asp Leu Leu Ile Leu Thr
                   325                 330                 335
   Trp Ile Gly Gly Gln Pro Val Ser Tyr Pro Phe Thr Ile Ile Gly Gln
                   340                 345                 350
   Val Ala Ser Val Leu Tyr Phe Thr Thr Ile Leu Ile Leu Met Pro Thr
           355                 360                 365
   Ile Ser Leu Ile Glu Asn Lys Met Leu Lys Trp Ala
       370                 375                 380